(19) Europäisches Patentamt
European Patent Office
Office européen des brevets

(11) Publication number: **0 399 645 B1**

(12) **EUROPEAN PATENT SPECIFICATION**

(45) Date of publication of patent specification: **09.11.94**

(51) Int. Cl.[5]: **C07D 413/14**, A61K 31/42, //(C07D413/14,309:00,303:00, 263:00,263:00),(C07D413/14, 309:00,307:00,303:00,263:00)

(21) Application number: **90303850.3**

(22) Date of filing: **10.04.90**

(54) **Tetrahydropyranyl derivatives, process for their preparation and pharmaceutical or veterinary compositions containing them.**

(30) Priority: **12.04.89 GB 8908284**
**12.04.89 GB 8908285**

(43) Date of publication of application:
**28.11.90 Bulletin 90/48**

(45) Publication of the grant of the patent:
**09.11.94 Bulletin 94/45**

(84) Designated Contracting States:
**AT BE CH DE DK ES FR GB GR IT LI LU NL SE**

(56) References cited:
**EP-A- 0 087 953**
**EP-A- 0 123 378**

**Journal of The Chemical Society,Perkin Transactions I no. 3, 1985, London pages 549-555; Michael J.Crimmin et al.: "The Chemistry of Pseudomonic Acid. Part 8. Electrophilic Substitutions at C-2 and C-15 of the Pseudomonic Acid Nucleus by Means of Lithium Dienolates"**

(73) Proprietor: **BEECHAM GROUP plc**
**Four New Horizons Court**
**Harlequin Avenue**
**Brentford Middlesex TW8 9EP (GB)**

(72) Inventor: **O'Hanlon, Peter John, Beecham Pharmaceuticals**
**Brockham Park**
**Betchworth, Surrey RH3 7AJ (GB)**
Inventor: **Walker, Graham, Beecham Pharmaceuticals**
**Brockham Park**
**Betchworth, Surrey RH3 7AJ (GB)**

(74) Representative: **Connell, Anthony Christopher et al**
**SmithKline Beecham plc**
**Corporate Intellectual Property**
**SB House**
**Great West Road**
**Brentford, Middlesex TW8 9BD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMICAL ABSTRACTS**, vol. 110, no. 5, 30 January 1989 Columbus, Ohio, USA. Klein Larry L. et al.: "Synthesis and activity of nonhydrolysable pseudomonic acid analogs." & J. Med.Chem. 1989, 32 (1), 151-60 page 522; column 2, ref. no. 38793.

## Description

The present invention relates to a class of compounds having antimycoplasmal and antibacterial activity, to processes for their preparation and to their use in human and veterinary medicine and also to intermediates for use in the preparation of such compounds.

Compounds of the general formula (A):

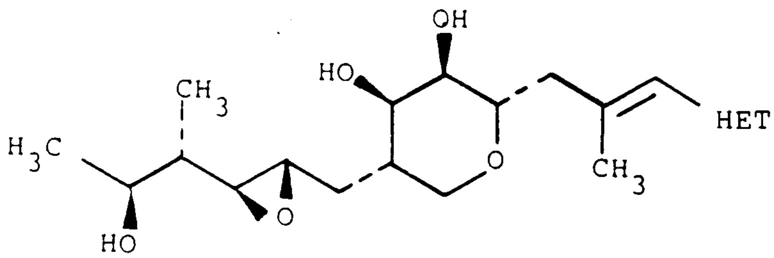

(A)

in which the group HET represents an optionally substituted 5-membered heteroaryl ring containing from 1 to 4 heteroatoms selected from oxygen, nitrogen and sulphur,
have been previously disclosed in EP-A-0 087 953 (Beecham Group p.l.c.) and EP-A-0 123 378 (Beecham Group p.l.c.) and will be recognised as derivatives of monic acid in which the carboxylic acid group thereof has been replaced by the group HET. Such compounds were shown to have antibacterial and antimycoplasmal activity.

Amongst the compounds of formula (A) described in EP-A-0 087 953 are examples in which the group HET is an oxazolyl ring, substituted with a range of groups including substituted phenyl, substituted alkyl and thienyl.

Surprisingly, it has now been found that biological activity may be enhanced in other types of substituted oxazolyl derivatives compared with those described in EP-A-0 087 953.

Accordingly the present invention provides a compound of formula (I):

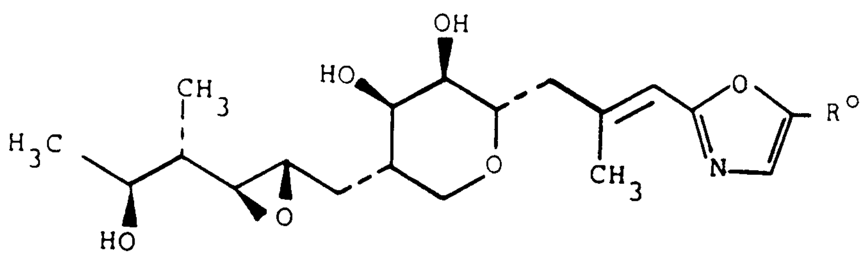

(I)

in which $R^o$ denotes a group of the formula (a), (b) or (c):

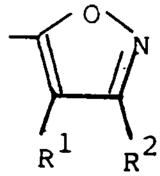

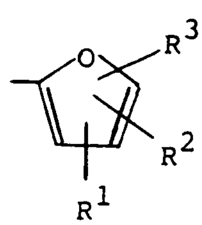

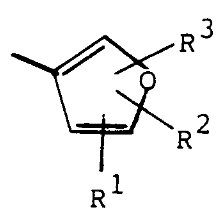

(a) (b) (c)

in which formulae $R^1$, $R^2$, and $R^3$ may be the same or different and each is selected from hydrogen, halogen, optionally substituted $(C_{1-6})$alkyl, aryl, aralkyl, heterocyclyl, $(C_{1-6})$alkoxy, hydroxy, carboxy and salts thereof, $(C_{1-6})$alkoxycarbonyl, carbamoyl, mono- or di-$(C_{1-6})$alkylcarbamoyl, sulphamoyl, mono- and di-$(C_{1-6})$alkylsulphamoyl, cyano, nitro, amino, mono-and di-$(C_{1-6})$alkylamino, acylamino, ureido, $(C_{1-6})$-alkoxycarbonylamino, $(C_{1-6})$alkoxyimino, 2,2,2-trichloroethoxycarbonylamino, acyl, $(C_{1-6})$alkylthio, arylthio, $(C_{1-6})$alkanesulphinyl, arylsulphinyl, $(C_{1-6})$alkanesulphonyl and arylsulphonyl.

Suitably, $R^o$ is a group of formula (a) and $R^1$ is preferably hydrogen and $R^2$ preferably $(C_{1-6})$alkyl or $R^o$ is a group of formula (b) and $R^1$ and $R^3$ are preferably hydrogen and $R^2$ preferably cyano.

Preferably $R^1$ and $R^3$ is each hydrogen.

When used herein, the term 'aryl' includes for example phenyl and naphthyl optionally substituted with up to five, preferably up to three, groups selected from halogen, optionally substituted $(C_{1-6})$alkyl, phenyl, $(C_{1-6})$alkoxy, halo$(C_{1-6})$alkyl, hydroxy, amino, mono- or di-$(C_{1-6})$alkylamino, nitro, carboxy, $(C_{1-6})$-alkoxycarbonyl, optionally substituted $(C_{1-6})$alkoxycarbonyl$(C_{1-6})$alkyl, $(C_{1-6})$alkylcarbonyloxy, $(C_{1-6})$-alkylcarbonyl, $(C_{1-6})$alkylthio, $(C_{1-6})$alkanesulphinyl, and $(C_{1-6})$alkanesulphonyl.

When used herein, the term 'aralkyl' includes groups in which the aryl moiety is a phenyl group which may be optionally substituted as herein before defined for aryl and in which the alkylene radical has from 1 to 4 carbon atoms.

When used herein, the term 'heterocyclyl' includes for example single or fused rings comprising up to four hetero atoms in the ring selected from oxygen, nitrogen and sulphur and optionally substituted with up to three groups selected from halogen, optionally substituted $(C_{1-6})$alkyl, $(C_{1-6})$alkoxy, halo$(C_{1-6})$alkyl, hydroxy, amino, mono- or di$(C_{1-6})$alkylamino, carboxy, $(C_{1-6})$alkoxycarbonyl, $(C_{1-6})$alkoxycarbonyl$(C_{1-6})$-alkyl, aryl or oxo.

Suitably the heterocyclic ring comprises from 4 to 7 ring atoms, preferably 5 or 6 atoms.

When used herein, the term 'halogen' refers to fluorine, chlorine, bromine or iodine.

Suitable substituents for alkyl include for example halogen, hydroxy, ( $C_{1-6}$)alkoxy, carboxy and salt thereof, $(C_{1-6})$alkoxycarbonyl, carbamoyl, mono- or di$(C_{1-6})$alkylcarbamoyl, sulphamoyl, mono- and di-$(C_{1-6})$alkylsulphamoyl, amino, mono- and di$(C_{1-6})$alkylamino, $(C_{1-6})$acylamino, ureido, $(C_{1-6})$-alkoxycarbonylamino, 2,2,2-trichloroethoxycarbonylamino, aryl, heterocyclyl, oxo, acyl, 2-thenoyl, $(C_{1-6})$-alkylthio, arylthio, $(C_{1-6})$alkanesulphinyl, arylsulphinyl, $(C_{1-6})$alkanesulphonyl, arylsulphonyl, $(C_{1-6})$-alkoxyimino, hydroxyimino, hydrazono, benzohydroxyimoyl, and 2-thiophene-carbohydroxyimoyl.

Compounds of formula (I) may conveniently be named as '(1-normon-2-yl)heterocycles'. Normonyl is the trivial name for the 3-[(2S,3R,4R,5S)-5-[(2S,4S,5S)-2,3-epoxy-5-hydroxy-4-methylhexyl]-3,4-dihydroxytetrahydropyran-2-yl]-2-methylprop-1(E)-enyl radical, as shown in formula (II):

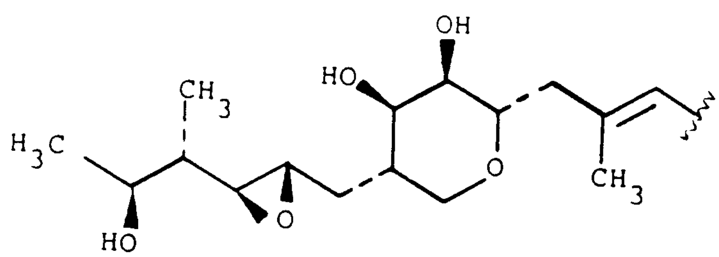

(II)

It will be appreciated that in compounds of formula (I), $R^1$, $R^2$, or $R^3$ may contain one or more chiral centres. The present invention encompasses all such resultant isomeric possibilities.

Specific compounds within this invention include the following:

2-(1-normon-2-yl)-5-(3-phenylisoxazol-5-yl)oxazole;
2(1-normon-2-yl)-5-(3-p-methylthiophenylisoxazol-5-yl)oxazole;
2-(1-normon-2-yl)-5-(3-p-methylsulphonylphenylisoxazol-5-yl)oxazole;
2-(1-normon-2-yl)-5-(3-methylisoxazol-5-yl)oxazole;
5-(3-bromo-5-isoxazolyl)-2-(1-normon-2-yl)oxazole;
2-(1-normon-2-yl)-5-(3-methoxy-5-isoxazolyl)oxazole;
2-(1-normon-2-yl)-5-(3-isopropyl-5-isoxazolyl)oxazole;
5-(3-t-butyl-5-isoxazolyl)-2-(1-normon-2-yl)oxazole;
2-(1-normon-2-yl)-5-(3-ethoxycarbonyl-5-isoxazolyl)oxazole;
2-(1-normon-2-yl)-5-(3-formyl-5-isoxazolyl)oxazole;
2-(1-normon-2-yl)-5-(3-hydroxymethyl-5-isoxazolyl)oxazole;

2-(1-normon-2-yl)-5-(3-pyrrolidin-1-yl-5-isoxazolyl)oxazole;
5-(2-furyl)-2-(1-normon-2-yl)oxazole;
5-(5-bromo-2-furyl)-2-(normon-2-yl)oxazole;
5-(3-furyl)-2-(1-normon-2-yl)oxazole;
5-(5-methyl-2-furyl)-2-(1-normon-2-yl)oxazole;
5-(5-methoxycarbonyl-2-furyl)-2-(1-normon-2-yl)oxazole;
5-(5-hydroxymethyl-2-furyl)-2-(1-normon-2-yl)oxazole;
sodium 5-(5-carboxylato-2-furyl)-2-(1-normon-2-yl)oxazole;
5-(5-carboxyamido-2-furyl)-2-(1-normon-2-yl)oxazole;
5-(5-methoxyformimidoyl-2-furyl)-2-(1-normon-2-yl)oxazole;
5-(2,5-dimethyl-3-furyl)-2-(1-normon-2-yl)oxazole;
5-(4-cyano-2-furyl)-2-(1-normon-2-yl)oxazole;
5-(5-cyano-2-furyl)-2-(1-normon-2-yl)oxazole;
5-(4-formyl-2-furyl)-2-(1-normon-2-yl)oxazole;
5-(2-cyanofuran-4-yl)-2-(1-normon-2-yl)oxazole;
5-(2-methoxyformimidoylfuran-4-yl)-2-(1-normon-2-yl)oxazole;
5-(4-methylfuran-2-yl)-2-(1-normon-2-yl)oxazole; and
5-(4-hydroxymethylfur-2-yl)-2-(1-normon-2-yl)oxazole.

Compounds of formula (I) can be prepared by analogy with the methods described in EP-A-0 087 953 and EP-A-0 123 378.

In particular, the present invention provides a process for the preparation of a compound of formula (I) which process comprises reacting a compound of formula (III):

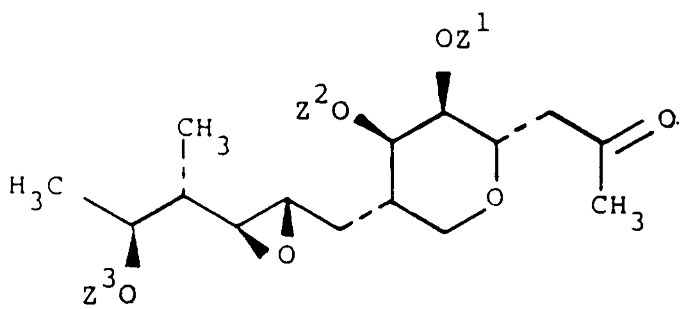

(III)

in which $Z^1$, $Z^2$ and $Z^3$ are the same or different and each is hydrogen or a hydroxyl-protecting group, with a compound of formula (IV):

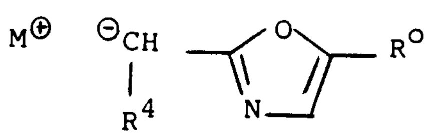

(IV)

in which:

$R^o$ is as defined in relation to formula (I);

$M^+$ is a metal cation, preferably an alkali metal cation, most preferably a lithium or sodium cation, and;

$R^4$ is an anion-stabilising group which will spontaneously eliminate with a $\beta$-hydroxyl group to produce an olefin, preferably a trialkylsilyl or a dialkylphosphonate group, most preferably trimethylsilyl or diethylphosphonate; and, where necessary, removing any hydroxyl-protecting groups, and, if desired, converting one compound of formula (I) into a further compound of formula (I).

The reaction of a compound of formula (III) with a compound of formula (IV) may conveniently be effected in an organic solvent, such as tetrahydrofuran, diethyl ether or dimethyl sulphoxide, at from reduced to elevated temperature, such as from -80° to 100°C.

The compound of formula (III) in which each of $Z^1$, $Z^2$ and $Z^3$ is hydrogen, and processes for its production, are described in GB 1 587 060. Derivatives thereof wherein $Z^1$, $Z^2$ and $Z^3$ are hydroxyl-protecting groups may be produced by conventional methods such as those mentioned below. When this compound is produced with hydroxyl-protecting groups already in place it may be used directly or even in

situ in the above reaction or it may be optionally deprotected and/or isolated.

The compounds of formula (IV) may be produced by conventional processes such as described in EP-A-0 123 378 and shown in Scheme I.

Scheme I

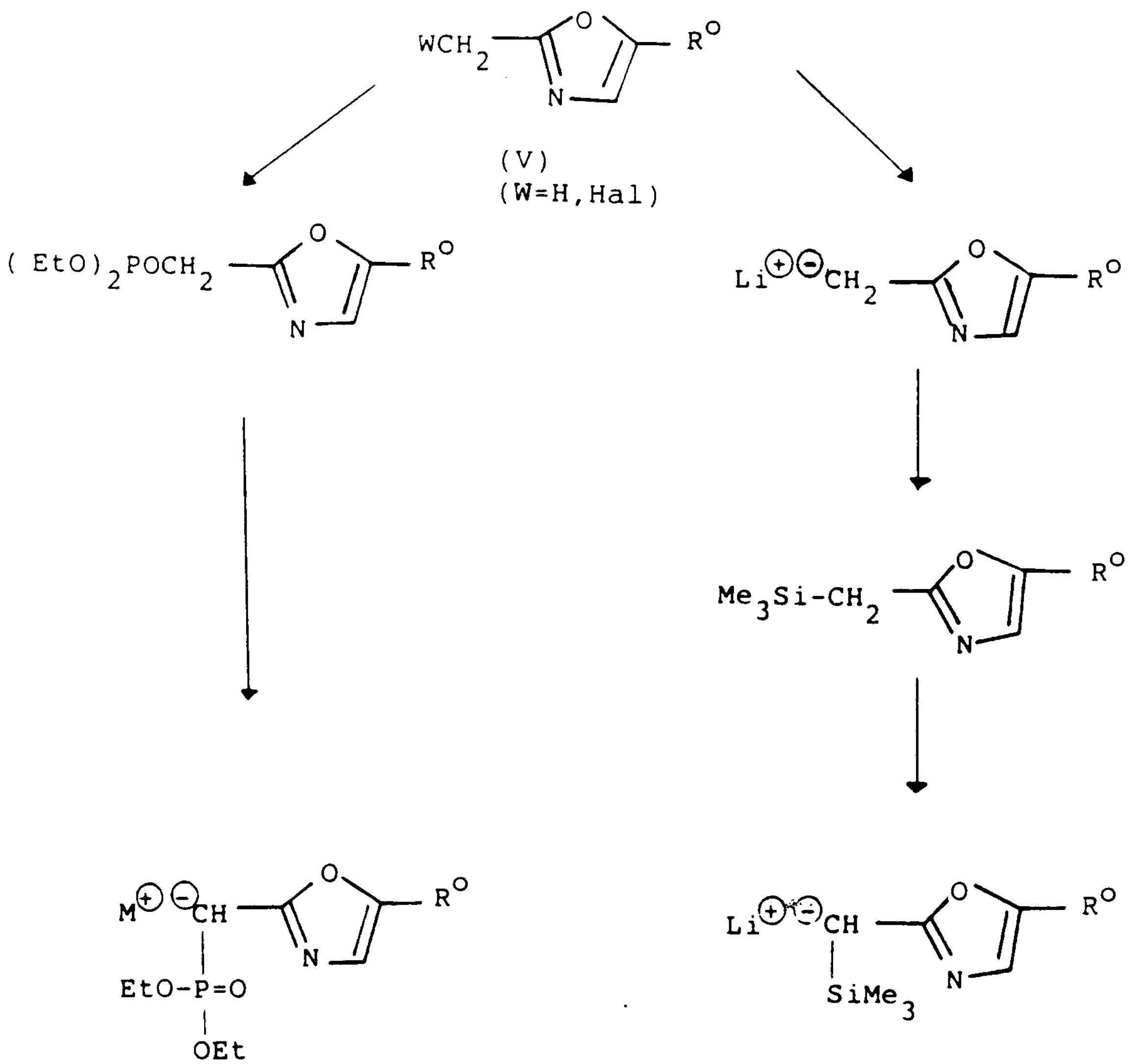


(IVa); $R^4 = PO(OEt)_2$. (IVb); M = Li, $R^4 = SiMe_3$.

In which Et and Me denote ethyl and methyl respectively.

The starting material utilised in Scheme I, the compound of formula (V):

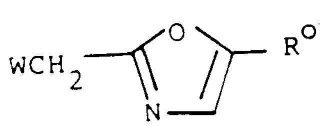

(V)

in which $R^o$ is as hereinbefore defined and W is hydrogen or halogen,
is a 2-substituted-5-(isoxazol-5-yl)oxazole, a 2-substituted-5-(fur-2-yl)oxazole or a 2-substituted-5-(fur-3-yl)-oxazole and may be prepared by adaption of conventional methodology used for the preparation of 2,5-disubstituted oxazoles and 5-substituted isoxazoles or 2- or 3- substituted furans, respectively. Suitable methodology is described in Comprehensive Heterocyclic Chemistry, ed. Katritzky and Rees 6, chapters 4.18 (oxazoles) and 4.16 (isoxazoles) and 4, chapters 3.03 and 3.12 (furans).

By way of illustration, for the instance when $R^o$ is a 5-isoxazolyl group, a convenient synthetic strategy takes as its starting point a 5-acetyl-isoxazole which is either commercially available or well known or prepared from readily available starting materials by adaptation of conventional methodology. The 5-acetyl substituent can be manipulated using procedures similar to those described in Organic Synthesis; Coll. Vol. V, 909, to give an α-aminoketone of formula (VI):

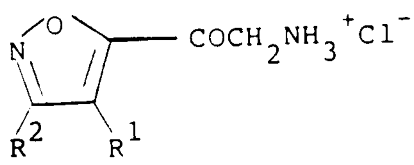

(VI)

in which $R^1$ and $R^2$ are as hereinbefore defined.

The α-amino ketone functionality can then be used to form an oxazole ring, according to the methodology described by J.L. La Mattina, J. Org. Chem., 1980, 45, 2261.

A similar procedure may be used for the instance when $R^o$ is a furyl group, using an acetylfuran as the starting material.

Suitable reaction conditions for the preparation of compounds of general formulae (IVa) and (IVb) are described by W.S. Wadsworth Jr, Organic Reactions, 1977, 25, 73 and by E.J. Corey and D.L. Boger, Tet. Letters, 1978, 5; T.H. Chan,, Acc. Chem. Res. 1977, 10, 442 and B.H. Lipshutz and K.W. Hungate, J. Org. Chem., 1981, 46, 1410; respectively.

The present invention also provides a process for the preparation of a compound of formula (I) which process comprises cyclising a compound of formula (VII):

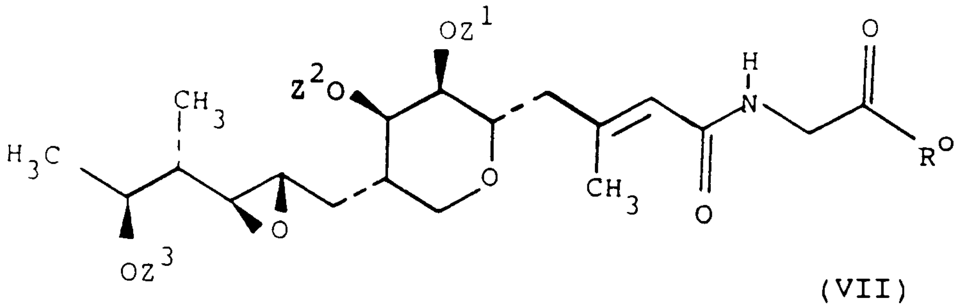

(VII)

in which $R^o$ is as defined in relation to formula (I), and $Z^1$, $Z^2$ and $Z^3$ are the same or different and each is hydrogen or a hydroxyl-protecting group;
to form a compound of formula (I) and, where necessary, removing any hydroxyl-protecting groups, and, if desired, converting one compound of formula (I) into a further compound of formula (I).

The cyclisation of a compound of formula (VII) is suitably effected using a chlorinating agent such as phosphorus oxychloride, phosgene, thionyl chloride or phosphorus pentachloride in the presence of a tertiary amine, such as triethylamine or pyridine. Such reactions are conveniently effected in an organic solvent, for instance dichloromethane or tetrahydrofuran, at from reduced to elevated temperature, for instance -80° to 100°C, over a period of several hours to a few days. Preferably phosgene or phosphorus oxychloride is used, at a temperature of from 0° to 20°C.

Alternatively, cyclisation may be effected using triphenylphosphine and carbon tetrachloride as the chlorinating reagent, in the presence of a tertiary amine, for instance triethylamine, in an inert solvent such as acetonitrile or acetonitrile-pyridine. This type of process is described by H. Vorbruggen and K.

Krolikiewicz in Tet. Letters., 1981, 4471: it is particularly advantageous in that the formation of compounds of formula (VII) and cyclisation of these to compounds of formula (I) may be conducted in situ.

Compounds of formula (VII) may also be cyclised using a carboxylic anhydride or mixed anhydride or acid chloride, such as trifluoroacetic anhydride or trichloroacetic anhydride or trichloroacetyl chloride which latter is used in the presence of pyridine and 4-dimethylaminopyridine. In this reaction the hydroxy groups of the 1-normon-2-yl radical become acylated and must subsequently be deprotected. Trihaloacetyl groups formed during cyclisation may be removed using potassium carbonate in solvents such as water, alkanols or admixtures thereof. Appropriate deprotecting conditions for removing other acyl residues will be readily apparent to the skilled person. Alternatively the hydroxy groups of the 1-normon-2-yl radical may be protected, prior to cyclising with a carboxylic anhydride, and deprotected by conventional methods such as described below.

Compounds of formula (VII) as defined above are novel and useful as chemical intermediates in the aforementioned process.

Accordingly, the present invention also provides a compound of formula (VII), as hereinbefore defined.

Compounds of formula (VII) may be produced according to the reaction sequence previously described in EP 0 087 953 and shown in Scheme II below. The compounds of formula (VI) utilised in this Scheme may be prepared as hereinbefore described.

Scheme II

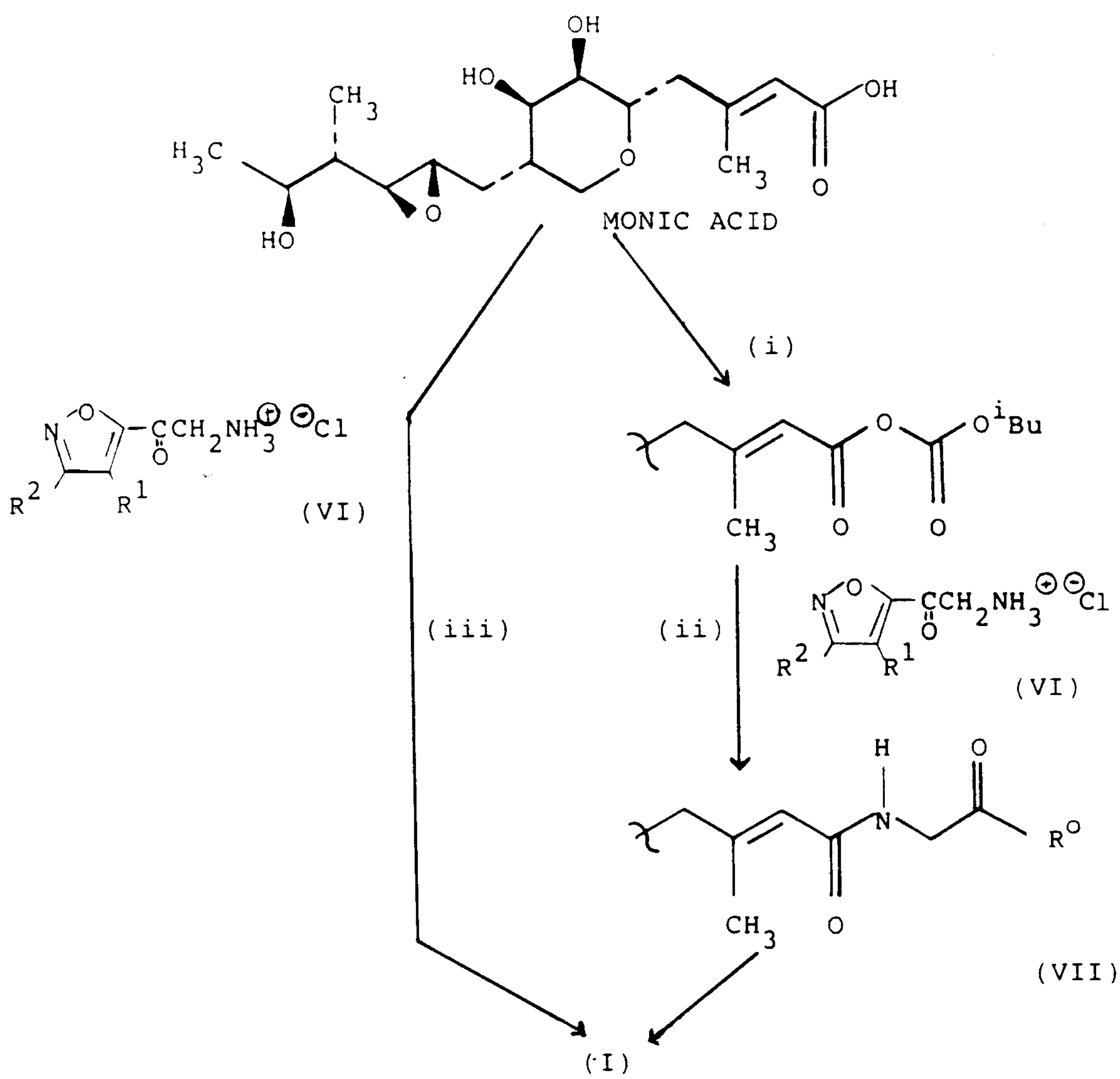


(i) iso-butyl chloroformate/tetrahydrofuran/triethylamine/ -5 to 20 °C, ca. 30min.

(ii) triethylamine/ -20 to 20 °C/1-16 hours.

(iii) triphenylphosphine/carbon tetrachloride/triethylamine.

The present invention further provides a process for the preparation of a compound of formula (I) which process comprises treating a compound of formula (VIII):

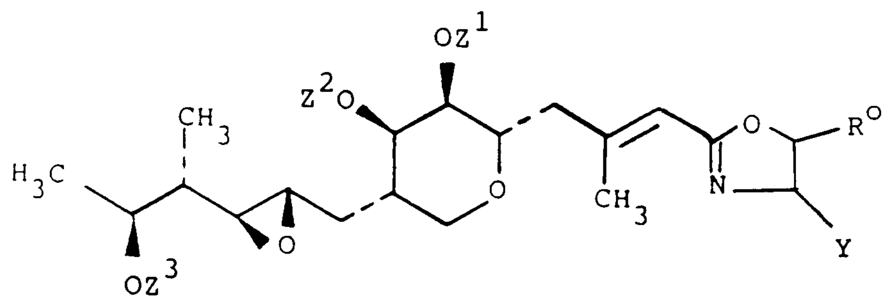

(VIII)

in which:

$Z^1$, $Z^2$, and $Z^3$ are the same or different and each is hydrogen or a hydroxyl-protecting group,

$R^o$ is as hereinbefore defined, and

Y is a leaving group;

with a strong base, and thereafter removing, if necessary, any hydroxyl-protecting groups.

Suitable values for Y include, for instance, aryl sulphonyl, for example p-toluenesulphonyl, alkylsulphonyl, alkyl or aryl sulphinyl, quaternary ammonium, for example trialkylammonium, and dialkoxy phosphine oxide.

Suitable strong bases include for example 1,8-diazobicyclo[5.4.0]undec-7-ene (DBU) and 1,5-diazobicyclo[4.3.0]non-5-ene (DBN).

Suitably the reaction is effected in a solvent such as acetonitrile, and at a temperature in the range from -20 to +80 °C.

Compounds of formula (VIII) as hereinbefore defined are novel and useful as intermediates in the aforementioned processs.

Accordingly, the present invention also provides a compound of formula (VIII), as hereinbefore defined.

Compounds of formula (VIII) may be prepared by the treatment of a compound of formula (IX):

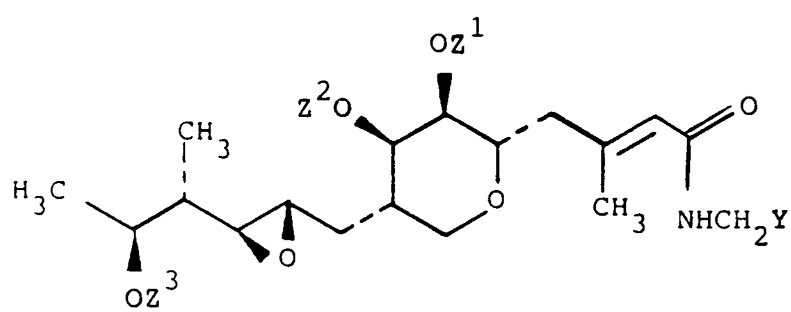

(IX)

in which $Z^1$, $Z^2$, and $Z^3$ are the same or different and each is hydrogen or a hydroxyl-protecting group, and Y is as hereinbefore defined,

with a compound formula (X):

$R^oCHO$ (X)

in which $R^o$ is as hereinbefore defined,

or a corresponding analogue thereof in which the aldehyde functionality of the compound formula (X) is masked,

under dehydrating conditions.

Suitable dehydrating conditions are similar to those hereinbefore described in respect of the cyclisation of a compound of formula (VIII) to give a compound of formula (I).

Particularly suitable conditions include the use of triphenylphosphine in combination with carbon tetrachloride or hexachloroethane, in the presence of triethylamine.

Compounds of formula (IX) as hereinbefore defined are novel and useful as chemical intermediates in the aforementioned process.

Accordingly, the present invention also provides a compound of formula (IX) as hereinbefore defined.

Compounds of formula (X) are recognisable as aldehyde derivatives of furans and isoxazoles and are either available commercially or well known or prepared from readily available starting materials by the adaptation of standard methodology.

A compound of formula (IX) may be prepared from an amide of formula (XI):

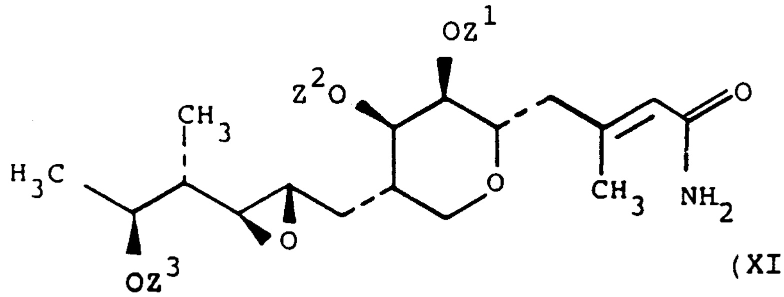

(XI)

in which $Z^1$, $Z^2$, and $Z^3$ are the same or different and each is hydrogen or a hydroxyl protecting group, by application of conventional methodology.

For instance, an amide of formula (XI) may be treated with formaldehyde in the presence of an aryl or an alkylsulphonic acid (in the instance when is aryl- or alkylsulphonyl).

Alternatively, an amide of formula (XI) may be treated with either N,N,N,N-tetramethylmethylene diamine or N,N-dimethylmethylene-ammonium iodide (Eschenmoser's salt), followed by quaternisation of the intermediate amine and then treatment with an aryl- or alkylsulphonic acid (in the instance when Y is aryl- or alkylsulphonyl).

A compound of formula (I) may also be prepared from compound of formula (XII):

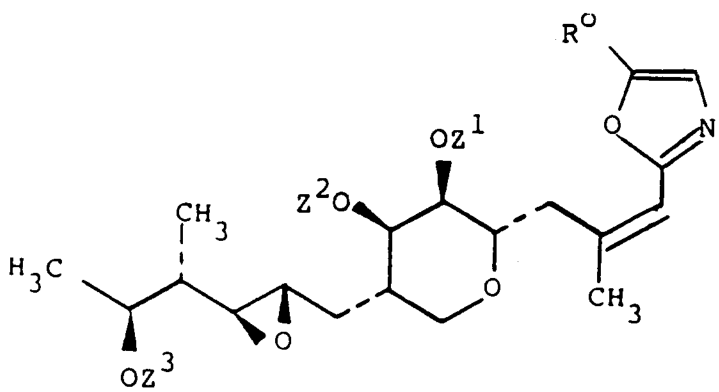

(XII)

in which $R^o$, $Z^1$, $Z^2$ and $Z^3$ are as hereinbefore defined; by methods known for the isomerisation of a carbon-carbon double bond.

Suitable isomerisation methods are described by Sonnet in Tetrahedron, 1980, 36, 557 and include photochemical and addition-elimination methods.

A compound of formula (XII) may be prepared by treatment of a compound of formula (III) with a compound of formula (IV), as hereinbefore described. This reaction may lack stereoselectivity and may lead to the formation of compounds of formulae (I) and (XII), which may then be separated by conventional procedures such as chromatography.

Compounds of formula (XII) as hereinbefore defined are novel and useful as chemical intermediates in the aforementioned process.

Accordingly, the present invention also provides a compound of formula (XII) as hereinbefore defined.

Conversion of one compound of formula (I) to another compound of formula (I) may be effected by conventional methods. Thus, for instance, all or any of $R^1$, $R^2$, and $R^3$ may be modified or converted. Included within modification of $R^1$, $R^2$, and $R^3$ are salification and esterification of a carboxy substituent, trans- and de-esterification of an ester-containing substituent, reduction of an alkoxycarbonyl substituent and formation of the free carboxy group from a carboxylate salt. Another example of such conversion is the formation of alkanesulphinyl and alkanesulphonyl compounds from the corresponding alkylthio compound of formula (I). This latter conversion may be achieved using conventional oxidising agents such as percarboxylic acids, for instance m-chloroperbenzoic acid, in a suitable solvent.

When used herein, the term 'hydroxyl-protecting group' refers to any such group known in the art which may be removed without disruption of the remainder of the molecule. Suitable hydroxyl-protecting groups include those described in Protective Groups in Organic Synthesis, T.W. Greene, Wiley-Interscience, New York 1981.

The hydroxyl groups of monic acid and the compounds of formulae (III), (IX), (XI) and (XII) may be protected at any stage of the above processes, using conventional methods. The hydroxyl-protecting group may be removed by methods known in the art, including enzymatic methods.

Particularly suitable protecting groups are silyl groups since these are readily removed under mild conditions. Such groups are introduced using conventional silylating agents, including halosilanes and silazanes, of the formulae below:

$L_3SiX$

$L_2SiX_2$

$L_3SiNL_2$

$L_3SiNHSiL_3$

$L_3SiNHCOL$

$L_3SiNHCONHSiL_3$

$LNHCONHSiL_3$

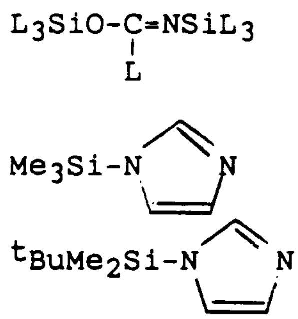

$^tBuMe_2Si-O-SO_2-CF_3$

in which Me is methyl, $^tBu$ is t-butyl, X is halogen and each group L is independently selected from hydrogen, $(C_{1-6})$alkyl, $(C_{1-6})$alkoxy, aryl or ar$(C_{1-4})$alkyl.

A preferred silyating agent is trimethylsilyl chloride. Particularly suitable protecting groups are trimethylsilyl, t-butyldimethylsilyl and t-butyldiphenylsilyl groups. Preferred protecting groups are trimethylsilyl groups because of their ease of removal.

The glycol function of monic acid and the compounds of formulae (III), (IX), (XI) and (XII) may be protected by forming a cyclic derivative using a compound of formula (XIII):

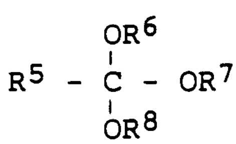

(XIII)

wherein $R^6$ is hydrogen or $(C_{1-6})$alkyl and each of $R^6$, $R^7$ and $R^8$ is $(C_{1-6})$alkyl. In the cyclic derivative $Z^1$ and $Z^2$ together are a moiety:

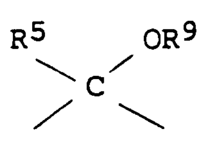

wherein $R^9$ is $(C_{1-6})$alkyl.

Suitably $R^5$ is hydrogen, methyl, ethyl, n- or iso-propyl; most suitably it is hydrogen. The groups $R^6$, $R^7$ and $R^8$ are suitably methyl, ethyl, n- or iso-propyl, n-, iso-, sec- or t-butyl; most suitably methyl.

Similarly the hydroxyl groups of a compound of formula (I) may be protected prior to conversion to a further compound of formula (I) as described above.

In each case the protecting groups described above may be removed by mild acid hydrolysis followed by alkaline hydrolysis, for instance, as described by J.P. Clayton, K. Luk and N.H. Rogers, in 'Chemistry of Pseudomonic Acid, Part II', J.C.S. Perkin Trans. I, 1979, 308.

The compounds of this invention are useful for the treatment of bacterial infections in animals, including man, such as respiratory tract infections, otitis, meningitis, and skin and soft tissue infections in man, and mastitis in cattle and respiratory infections in animals such as pigs and cattle.

The compounds of this invention are active against both Gram negative and Gram positive organisms, including Haemophilus for instance H.influenzae Q1; Branhamella, for instance B.Catarrhalis 1502; Streptococci, for instance S.pyogenes CN10 and S.pneumonia PU7; and Staphylococci for instance S.aureus Oxford.

This invention also provides a pharmaceutical or veterinary composition which comprises a compound of formula (I) (hereinafter referred to as the 'drug') together with a pharmaceutically or veterinarily acceptable carrier or excipient.

The compositions may be formulated for administration by any route, and would depend on the disease being treated. The compositions may be in the form of tablets, capsules, powders, granules, lozenges, liquid or gel preparations, such as oral, topical or sterile parenteral suspensions.

Tablets and capsules for oral administration may be in unit dose presentation form, and may contain conventional excipients such as binding agents, for example syrup, acacia, gelatin, sorbitol, tragacanth, or polyvinyl-pyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin, hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin the drug may be made up into a cream, lotion or ointment. Cream or ointment formulations that may be used for the drug are conventional formulations well known in the art, for example, as described in standard text books of pharmaceutics and cosmetics, such as Harry's Cosmeticology published by Leonard Hill Books, and the British Pharmacopoeia.

Suppositories will contain conventional suppository bases, e.g. cocoa-butters or other glyceride.

For parenteral administration, fluid unit dosage forms are prepared utilizing the drug and a sterile vehicle. The drug, depending on the vehicle and concentration used, can be suspended in the vehicle. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. The drug can be sterilised by exposure to ethylene oxide before suspending in the sterile vehicle. Advantageously, a surfactant or wetting agent is included in the composition to facilitate uniform distribution of the drug.

For topical application to the ear, the drug may be made up into a suspension in a suitable liquid carrier, such as water, glycerol, diluted ethanol, propylene glycol, polyethylene glycol or fixed oils.

For topical application to the eye, the drug is formulated as a suspension in a suitable, sterile aqueous or non-aqueous vehicle. Additives, for instance buffers such as sodium metabisulphite or disodium edetate; preservatives including bactericidal and fungicidal agents, such as phenylmercuric acetate or nitrate, benzalkonium chloride or chlorhexidine, and thickening agents such as hypromellose may also be included.

The dosage employed for compositions administered topically will, of course, depend on the size of the area being treated. For the ears and eyes each dose will typically be in the range from 10 to 100 mg of the drug.

Veterinary compositions for intramammary treatment of mammary disorders in animals, especially bovine mastitis, will generally contain a suspension of the drug in an oily vehicle.

The compositions may contain from 0.1% to 99% by weight, preferably from 10-60% by weight, of the drug, depending on the method of administration. Where the compositions are in unit dose form, each dosage unit will preferably contain from 50-500 mg, of the drug. The dosage as employed for adult human treatment will preferably range from 100 mg to 3 g, per day, for instance 250 mg to 2 g of the drug per day, depending on the route and frequency of administration.

Alternatively, the drug may be administered as part of the total dietary intake. In this case the amount of drug employed may be less than 1% by weight of the diet and in preferably no more than 0.5% by weight. The diet for animals may consist of normal foodstuffs to which the drug may be added or the drug may be included in a premix for admixture with the foodstuff. A suitable method of administration of the drug to animals is to add it to the animals' drinking water. In this case a concentration of the drug in the drinking water of about 5-500 $\mu$g/ml, for example 5-200 $\mu$g/ml, is suitable.

The present invention further provides a method for treating the human or non-human animal which method comprises administering an effective amount of a compound of formula (I) to a human or non-human animal in need of such therapy.

Alternatively, a pharmaceutical composition as hereinbefore described may be employed in the treatment.

In particular aspects of the treatment there are provided methods for treating bacterial infections of human or non-human animals, especially respiratory infections in human and non-human animals.

The following Examples illustrate the invention.

The following abbreviations have been used in the Examples:

| | |
|---|---|
| DMF | N,N-Dimethylformamide |
| DMSO | Dimethyl sulphoxide |
| THF | Tetrahydrofuran |
| $MgSO_4$ | Anhydrous magnesium sulphate |

Chromatography and flash chromatography were executed using silica gel as the adsorbent.

HP2055 is a grade of DIAION HP20® (Trade Mark) styrene-divinylbenzene copolymer resin.

Monic acid A is obtained from pseudomonic acid according to the process described in GB 1 587 058

<u>2-Acetonyl-3,4-bis(t-butyldimethylsilyloxy)-5-(5-t-butyldimethylsilyloxy-2,3-epoxy-4-methylhexyl)-tetrahydropyran (TBDMS-protected ketone)</u>

t-Butyldimethylsilyl trifluoromethane sulphonate (1.30ml, 5.7mmol) was added to a solution containing 3,4-dihydroxy-5-(2,3-epoxy-5-hydroxy-4-methylhexyl)tetrahydropyran-2-yl acetone (0.36g, 1.2mmol), 2,6-lutidine (1.1ml, 10mmol), and THF (10ml) at -20 °C. After 30min at -20 °C aqueous ammonium chloride and ethyl acetate were added. The organic layer was dried and evaporated and the residue chromatographed (silica, ether/hexane) to give the crude silyl enol ether of the title compound as a colourless oil (0.4g, 45%); $\nu_{max}$ (film) 1630cm$^{-1}$.

This crude product (0.1g, 0.13mmol) was dissolved in THF (10ml)/5M hydrochloric acid (1 drop) at 20 °C. After 10min at 20 °C aqueous sodium hydrogen carbonate and ethyl acetate were added. The organic layer was dried and evaporated and the residue chromatographed (silica, ether/hexane) to give the title compound as a colourless oil (0.04g, 37%, 12% overall); $\nu_{max}$ (film) 1715cm$^{-1}$; $\delta_H$ ($CDCl_3$) 1.41 (30H, s, $^t$Bu, 17-$H_3$), 1.20 (3H, d, J 7.0Hz, 14-$H_3$), 1.38 (1H, d-quintet, J 3.3, 7.3Hz, 12-H), 1.52 (1H, dd, J 6.2, 16.4Hz, $9_a$-H), 1.72 (2H, m, 8-H, $9_b$-H), 2.18 (3H, s, 15-$H_3$), 2.40 (1H, dd, J 10.4, 15.2Hz, $4_{ax}$-H), 2.67 (3H, m, 4eq-H, 10-H, 11-H), 3.40 (1H, dd, J 2.1, 9.3Hz, 6-H), 3.54 (1H, d, J 22.1Hz, 16eq-H), 3.88 (3H, m, $16_{ax}$-H, 7-H, 13-H), and 4.11 (1H, dt, J 2.4, 9.7Hz, 5-H).

<u>2-Acetonyl-3,4-bis(trimethylsilyloxy)-5-(5-trimethylsilyloxy-2,3-epoxy-4-methylhexyl)tetrahydropyran (TMS-protected ketone)</u>

To a solution of 3,4-dihydroxy-5-(2,3-epoxy-5-hydroxy-4-methylhexyl)tetrahydropyran-2-yl acetone (151mg, 0.5mmol) in dry THF (20ml) was added triethylamine (0.6ml, 4.3mmol), trimethylsilyl chloride (0.5ml, 4.0mmol) and a catalytic amount of 4-(N,N-dimethylamino)pyridine. After stirring at room temperature for 2h the triethylamine hydrochloride was filtered off and the solution concentrated under reduced pressure. The resultant oil was taken up in anhydrous ether, filtered and the solvent removed under reduced pressure to give a colourless oil which was used without purification; $\nu_{max}$ (film) 1715cm$^{-1}$.

General method of preparation of a monamide

To a solution of monic acid A in dry THF (5ml/mmol) at 0 °C were added triethylamine (1.1 equiv.) and isobutyl chloroformate (1 equiv.). After 0.5h at 0 °C the appropriate ammonium salt (1 equiv.) and triethylamine (1 equiv.) were added and the reaction mixture stirred at 0 °C for 3h. Ethyl acetate was added and the solution washed with aqueous sodium hydrogen carbonate and brine,, then dried ($MgSO_4$) and evaporated under reduced pressure. The resulting residue was purified by chromatography (silica gel, eluting with 0 to 6% methanol in dichloromethane) to yield the pure monamide.

General method of preparation of an oxazole from a monamide

Trichloroacetyl chloride (9 equiv.) was added to a solution of a monamide, 4-dimethylaminopyridine (few crystals/mmol) and pyridine (20 equiv.) in dichloromethane (10ml/mmol), cooled in an ice bath. After 0.5h the solution was washed with aqueous sodium hydrogen carbonate solution and then evaporated under reduced pressure. The resulting residue was dissolved in methanol (5ml/mmol) and the solution cooled to 0 °C before addition of potassium carbonate (3 equiv.). After 15min at 0 °C brine and ethyl acetate were added and the organic layer separated. The aqueous layer was further extracted with ethyl acetate and the combined extracts washed with brine, dried ($MgSO_4$) then evaporated under reduced pressure. The resulting residue was chromatographed on silica (0 to 10% methanol in dichloromethane) to give the pure oxazole.

Formulae

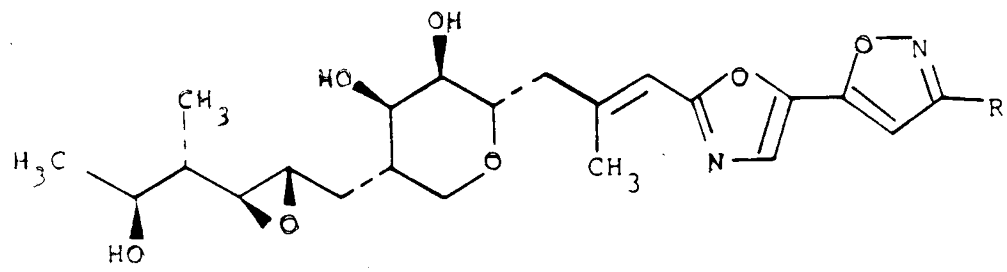

| Example | R |
|---|---|
| 1 | Ph |
| 2 | 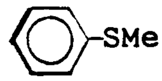 |
| 3 | 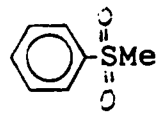 |
| 4 | -Me |
| 5 | -Br |
| 6 | -OMe |
| 7 | $-Pr^i$ |
| 8 | $-Bu^t$ |
| 9 | $-CO_2Et$ |
| 10 | -CHO |
| 11 | $-CH_2OH$ |
| 12 | -Me |
| 13 | 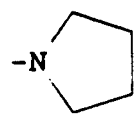 |

Formulae

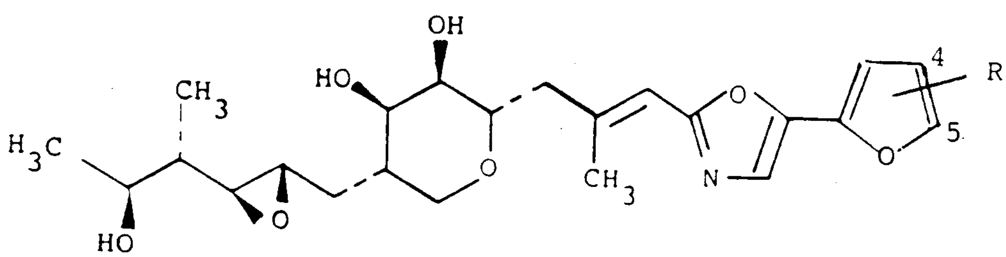

| Example | R |
|---|---|
| 14 | H |
| 15 | 5-Br |
| 17 | 5-Me |
| 18 | 5-$CO_2$Me |
| 19 | 5-$CH_2$OH |
| 20 | 5-$CO_2$Na |
| 21 | 5-$CONH_2$ |
| 22 | 5-C(=NH)OMe |
| 24 | 4-CN |
| 25 | 5-CN |
| 26 | 4-CHO |
| 29 | 4-Me |
| 30 | 4-$CH_2$OH |

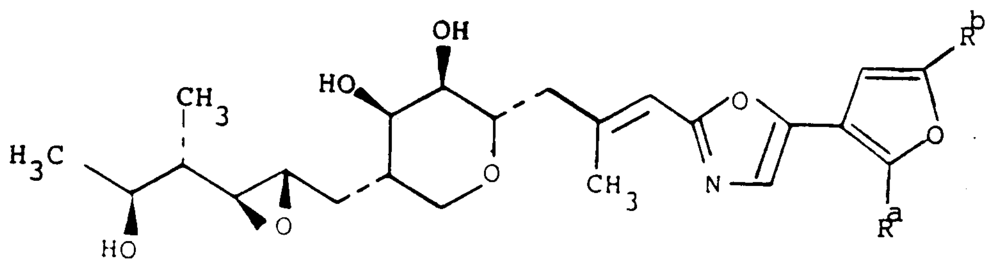

| Example | $R^a$ | $R^b$ |
|---|---|---|
| 16 | H | H |
| 23 | Me | Me |
| 27 | H | CN |
| 28 | H | C(=NH)OMe |

Example 1

2-(1-Normon-2-yl)-5-(3-phenylisoxazol-5-yl)oxazole

a) 5-Acetyl-3-phenylisoxazole

A vigorously stirred mixture of 3-butyn-2-one (1.6ml, 1.4g, 20mmol), dichloromethane (20ml), and aqueous sodium hypochlorite (1.3M; 26ml, 34mmol) was treated at 0°C with a solution of benzaldehyde oxime (3.2g, 26mmol) in dichloromethane (20ml) at 0°C. After 5 minutes the phases were separated, and the organic phase washed with half-saturated brine, dried ($MgSO_4$) and evaporated, affording a yellow solid (3.6g, 96%). This was chromatographed, eluting with 50% hexane in dichloromethane, then neat dichloromethane, to give 5-acetyl-3-phenylisoxazole as a white solid (2.01g, 54%), m.p. 103-105°C (ethanol); $\delta_H$ ($CDCl_3$) 2.75 (3H, s, $CH_3$), 7.15 (1H, s, Het-H), 7.45 (3H, m, ArH), and 7.80 (2H, m, ArH); $\nu_{max}$ ($CH_2Cl_2$) 1705, 1600, 1580 and 1435$cm^{-1}$.

b) 5-Bromoacetyl-3-phenylisoxazole

A solution of the methyl ketone (1.37g, 7.3mmol) [prepared in (a)] in chloroform (30ml) was treated with bromine (0.4ml, 1.2g, 7.7mmol) then refluxed for 0.25h. Evaporation afforded an off-white solid (1.37g), which was recrystallised from ethanol affording 5-bromoacetyl-3phenylisoxazole as white crystals (1.2g, 62%), m.p. 113-115°C, $\delta_H$ ($CDCl_3$) 4.45 (2H, s, $CH_2$Br), 7.30 (1H, s, Het-H), 7.45 (3H, m, ArH), and 7.80 (2H, m, ArH); $\nu_{max}$ (KBr) 1720, 1705, 1600, 1580 and 1440$cm^{-1}$.

c) 5-Aminoacetyl-3-phenylisoxazole hydrochloride

A solution of the bromide (3.38g, 12.7mmol) [prepared in (b)] in dichloromethane (100ml) was treated

with hexamethylene tetramine (1.96g, 14mmol). The reaction was stirred for 1h at room temperature, during which time a precipitate formed. The reaction mixture was stored in the fridge for 1h, then filtered, affording a white solid (4.43g). A suspension of this material in concentrated hydrochloric acid - ethanol (10ml-20ml) was stirred at 15°C (room temperature) for 1.5h. Water (10ml) was added, and stirring was continued for a further 1h. The reaction mixture was filtered, and the filtrate stored in the fridge overnight. Filtration then gave a second crop which was combined with the first, affording 5-aminoacetyl-3-phenylisoxazole hydrochloride as a white solid (1.76g, 58%), m.p. 180-193°C, $\delta_H$ [$(CD_3)_2SO$] 4.50 (2H, bs, $CH_2CO$), 7.60 (3H, m, ArH), 8.00 (2H, m, 2 x ArH), 8.30 (1H, s, Het-H), and 8.80 (3H, m, $NH_3$); $\nu_{max}$ (KBr) 3300-3400, 2800-3100, 1710, 1600, 1580 and 1545cm$^{-1}$.

d) N-[2-Oxo-2-(3-phenylisoxazol-5-yl)ethyl]monamide

A solution of monic acid (1.53g, 4.45mmol) in tetrahydrofuran (120ml) was treated with triethylamine (0.7ml, 0.5g, 4.9mmol), then at 0°C with isobutyl chloroformate (0.58ml, 0.6g, 4.45mmol). After 0.75h at 0°C, the reaction mixture was treated with triethylamine (0.7ml, 0.5g, 4.9mmol) and then the above amine hydrochloride (1.062g, 4.45mmol). The reaction mixture was stirred at 0°C for 1.5h, then partitioned between ethyl acetate - half saturated brine. The organic phase was dried ($MgSO_4$) and evaporated affording a yellow foam (2.4g). Chromatography, eluting with 0-6% methanol in dichloromethane afforded N-[2-oxo-2-(3phenylisoxaz-5-yl)ethyl] monamide as a yellow foam (0.67g, 29%), $\nu_{max}$ ($CH_2Cl_2$) 3300-3600, 2900, 1710, 1665 and 1640cm$^{-1}$; $\delta_H$ ($CDCl_3$) 0.89 (3H, d, J 6.7Hz, 17-$H_3$), 1.18 (3H, d, J 6.3Hz, 14-$H_3$), 1.31 (1H, m, 12-H), 1.70 (2H, m, 9-$H_2$), 1.99 (1H, m, 8-H), 2.17 (3H, s, 15-$H_3$), 2.25 (1H, m, 4-H), 2.55-2.78 (3H, m, 4-H, 10- and 11-H), 3.45-3.89 (9H, m, 16-$H_2$, 5-, 6-, 7- and 13-H and 3 x OH), 4.75 (2H, s, $CH_2N$), 5.85 (1H, s, 2-H), 7.02 (1H, s, Het-H), 7.44 (3H, m, 3 x ArH), and 7.79 (2H, m, 2 x ArH); $\delta_C$ ($CDCl_3$) 184.8 (C-2′), 167.6 (C-1), 165.1 and 163.2 (C-5″ and -3″), 153.1 (C-3), 130.8 (1 x Ph), 129.2 (2 x Ph), 127.6 (1 x Ph), 126.9 (2 x Ph), 119.2 (C-2), 106.5 (C-4″), 75.0 (C-5), 71.3 (C-13), 70.5 (C-7), 68.9 (C-6), 65.3 (C-16), 61.2 (C-11), 55.7 (C-10), 47.0 (C-1′), 42.8 (C-12), 42.6 (C-4), 39.6 (C-8), 31.7 (C-9), 20.9 (C-14), 19.0 (C-15), and 12.8 (C-17); $\lambda_{max}$ (EtOH) 236nm ($\epsilon_m$ 29,150); and m/z 529 ($MH^+$), (F.A.B., thioglycerol matrix).

e) 2-(1-Normon-2-yl)-5-(3-phenylisoxazol-5-yl)oxazole

A solution of the monamide (330mg, 0.65mmol) [prepared in (d)] in dichloromethane (6ml) was treated at 0°C with pyridine (1.05ml, 1.01g, 12.8mmol), catalytic N,N-dimethylamino pyridine, and trichloroacetyl chloride (0.65ml, 1.05g, 5.8mmol). After 0.5h the reaction mixture was partitioned between ethyl acetate and aqueous sodium hydrogen carbonate. The organic phase was dried ($MgSO_4$) and evaporated affording a brown oil. This was dissolved in methanol (12ml) and treated at 0°C with potassium carbonate (0.3g). After 0.25h the reaction mixture was partitioned between ethyl acetate and halfsaturated brine. The aqueous phase was further extracted with ethyl acetate and the combined organic extracts were dried ($MgSO_4$) and evaporated affording a dark brown foam (0.4g). This was subjected to chromatography (0 to 4% methanol in dichloromethane), and the partially purified product re-chromatographed (0 to 4% ethanol in ethyl acetate) affording the title oxazole as a pale orange foam (128mg, 40%), $\nu_{max}$ ($CH_2Cl_2$) 3600, 3560, 2940, 1730, 1650, 1540, and 1460cm$^{-1}$; $\delta_H$ ($CDCl_3$), 0.92 (3H, d, J 6.7Hz, 17-$H_3$), 1.29 (3H, d, J 7.1Hz, 14-$H_3$), 1.34 (1H, m, 12-H), 1.75 (2H, m, 9-$H_2$), 2.05 (1H, m, 8-H), 2.34 (3H, s, 15-$H_3$), 2.41 (1H, m, 4-H), 2.71-2.84 (3H, m, 4-, 10- and 11-H), 3.50-4.00 (6H, m, 16-$H_2$, 5-, 6-, 7-and 13-H), 5.30 (1H, s, 2-H), 6.78 (1H, s, 4″-H), 7.48 (3H, m, 3 x PhH), 7.60 (1H, s, 4′-H) and 7.85 (2H, m, 2 x PhH); $\delta_C$ ($CDCl_3$) 162.8, 162.5 and 159.7 (C-1, -5″ and -3″), 149.8 (C-3), 139.0 (C-5″), 130.4 (1 x Ph), 129.1 (2 x Ph), 128.4 (C-4′), 127.7 (1 x Ph), 127.0 (2 x Ph), 112.5 (C-2), 98.5 (C-4″), 75.1 (C-5), 71.5 (C-13), 70.5 (C-7), 69.0 (C-6), 65.5 (C-16), 61.4 (C-11), 55.6 (C-10), 43.0 (C-4), 42.9 (C-12), 39.6 (C-8), 31.7 (C-9), 20.9 (C-14), 19.8 (C-15), and 12.8 (C-17); $\lambda_{max}$ (EtOH) 306nm ($\epsilon_m$ 30,500). (Found: m/z $M^+$, 510.23040; $C_{28}H_{34}O_7N_2$ requires 510.2360).

Example 2

2(1-Normon-2-yl)-5-(3-p-methylthiophenylisoxazol-5-yl)oxazole

a) 3-p-Methylthiophenyl-5-acetylisoxazole

A solution of p-methylthiobenzaldoxime (3.3g; 20mmol) in dichloromethane (20ml) was slowly added to a mixture of but-3-yn-2-one (1.6ml, 20mmol), aqueous sodium hypochlorite (30ml x 1.3M, 40mmol) and dichloromethane (20ml) at 0°C. After a further 10min at 0°C the organic phase was separated, washed with water, dried ($MgSO_4$), and evaporated. The residue was purified by chromatography (silica, dichloromethane) to give 3-p-methylthiophenyl-5-acetylisoxazole a white rhombs (0.9g, 19%); m.p. 135-138°C (ethanol); $\nu_{max}$ (KBr) 1690, 1590cm$^{-1}$; $\delta_H$ ($CDCl_3$) 2.5 (3H, s, SMe), 2.6 (3H, s, CMe), 7.2 (1H, s,

het), 7.4 (2H, d, aryl), 7.8 (2H, d, aryl); (Found: C, 61.7; H, 4.6; N, 6.1%, $C_{12}H_{11}NO_2S$ requires C, 61.8; H, 4.8; N, 6.0%).

b) 3-p-Methylthiophenyl-5-bromoacetylisoxazole

A solution containing the above ketone (0.5g, 3mmol), bromine (0.15ml, 3mmol), dichloromethane (5ml), and a few drops of methanolic hydrogen chloride was boiled for a few minutes then stood at 20°C for 1.5h. Evaporation gave 3-p-methylthiophenyl-5-bromoacetylisoxazole as white rhombs (0.63g, 67%; m.p. 156-159°C (ethanol), $\nu_{max}$ (KBr) 1710, 1600cm$^{-1}$, $\delta_H$ ($CDCl_3$) 2.5 (3H, s, Me), 4.5 (2H, s, $CH_2$), 7.4 (1H, s, het), 7.5 (2H, d, aryl), 7.9 (2H, d, aryl).

c) 3-p-Methylthiophenyl-5-aminoacetylisoxazole hydrochloride

A solution containing the above bromide (0.6g, 2.0mmol) hexamethylenetetramine (0.3g, 2.2mmol) and dichloromethane (10ml) was stirred at 20°C for 1h then filtered. The residue was stirred with ethanol (2ml) and concentrated hydrochloric acid (1ml) at 20°C for 2h then cooled to 0°C and filtered to give 3-p-methylthiophenyl-5-aminoacetylisoxazole hydrochloride as a white powder (0.35g, 62%); $\delta_H$ ($d_6$-DMSO) 2.5 (3H, s, Me), 4.5 (2H, d, $CH_2$), 7.4 (2H, d, aryl), 7.9 (2H, d, aryl), 8.2 (1H, s, het), 8.5 (3H, t, $NH_3^+$).

d) 3-p-Methylthiophenylisoxazol-5-ylcarbonylmethyl monamide

Using the general method, but adding the amine hydrochloride in methanol solution, the above salt (0.5g, 1.8mmol) was converted to 3-p-methylthiophenylisoxazol-5-ylcarbonylmethyl monamide, obtained as an extremely insoluble yellow gum; (0.61g, 58%). A satisfactory $^1$H n.m.r. spectrum of this compound could not be obtained.

e) 2-(1-Normon-2-yl)-5-(3-p-methylthiophenylisoxazol-5-yl)oxazole

Using the general method this crude amide (0.3g, 0.54mmol) was converted to the title compound, obtained as a yellow foam (0.11g, 37%); $\nu_{max}$ (KBr) 1650, 1630, and 1600cm$^{-1}$; $\lambda_{max}$ (EtOH) 303nm ($\epsilon_m$ 36,200); $\delta_H$ ($CDCl_3$-$CD_3OD$) 0.94 (3H, d, 17-$H_3$), 1.22 (3H, d, 14-$H_3$), 2.35 (3H, s, 15-$H_3$), 2.56 (3H, s, SMe), 6.31 (1H, s, 2-H), 6.72 (1H, s, isoxazole), 7.32 (2H, d, aryl), 7.59 (1H, s, oxazole), 7.77 (2H, d, aryl); $\delta_C$ ($CDCl_3$-$CD_3OD$) 12.5 (C-17), 15.2 (SMe), 19.7 (C-15), 20.6 (C-14), 31.7 (C-9), 39.6 (C-8), 42.6, 43.0 (C-4, -12), 55.6 (C-10), 61.1 (C-11), 65.6 (C-16), 68.6 (C-6), 70.1 (C-7), 70.9 (C-13), 75.0 (C-5), 98.4 (isoxazole C-4), 112.3 (C-2), 124.6, 126.3, 127.2 (aryl C-2, 3, 4, 5, 6), 127.5 (oxazole C-5), 139.0 (oxazole C-4), 141.9 (aryl C-1), 150.2 (C-3), 159.7, 162.3, 162.7 (C-1, isoxazole C-3, 5); m/z (thioglycerol C.I.) 557 ($MH^+$).

## Example 3

### 2-(1-Normon-2-yl)-5-(3-p-methylsulphonylphenylisoxazol-5-yl)oxazole

A solution containing the sulphide (Example 2) (65mg, 0.12mmol), sodium hydrogen carbonate (25mg, 0.30mmol), 3-chloroperbenzoic acid (51mg, 0.25mmol) and methanol (5ml) was stirred for 1h at 20°C. Saturated aqueous sodium hydrogen carbonate and ethyl acetate were then added and the organic layer washed with brine, dried ($MgSO_4$), and evaporated. The residue was purified by chromatography (silica, methanol-dichloromethane mixtures) to give the title compound as pale yellow plates (32mg, 45%); m.p. 141-144°C (ethanol); $\nu_{max}$ (KBr) 1620, 1640cm$^{-1}$; $\lambda_{max}$ (EtOH) 306nm ($\epsilon_m$ 25,200); $\delta_H$ ($CDCl_3$-$CD_3OD$) 0.96 (3H, d, 17-$H_3$), 1.22 (3H, d, 14-$H_3$), 2.36 (3H, s, 15-$H_3$), 3.17 (3H, s, $SO_2Me$), 6.33 (1H, s, 2-H), 7.04 (1H, s, isoxazole) 7.69 (1H, s, oxazole), 8.1 (4H, m, aryl); $\delta_C$ ($CDCl_3$-$CD_3OD$) 12.5 (C-17), 19.7 (C-15), 20.6 (C-14), 31.7 (C-9), 39.6 (C-8), 42.6 (C-12), 43.0 (C-4), 44.4 ($SO_2Me$), 55.6 (C-10), 61.1 (C-11), 65.6 (C-16), 68.7 (C-6), 70.1 (C-7), 71.0 (C-13), 75.0 (C-5), 98.4 (isoxazole C-4), 112.2 (C-2), 127.9, 128.1 128.2 (oxazole C-5, aryl C-2, 3, 5, 6), 133.7 (aryl C-4), 139.5 (oxazole C-4), 14.9 (aryl C-1), 150.7 (C-13), 160.6, 161.2, 162.9 (C-1 isoxazole C-3, 5); m/z (thioglycerol C.I.) 589 ($MH^+$).

## Example 4

### 2-(1-Normon-2-yl)-5-(3-methylisoxazol-5-yl)oxazole

a) 3-Methyl-5-isoxazolylmethanol

Chlorine (12g, 0.17mmol) was added to acetaldoxime (10g, 0.17mmol) in hydrochloric acid (40ml, 1.5M) at -5°C. Saturated aqueous ammonium sulphate was then added and the solution extracted with ether. The combined extracts were added during 30min to a well stirred mixture of potassium hydrogen carbonate (44g, 0.45mmol), but-2-yn-1-ol (18ml, 0.30mmol), ethyl acetate (600ml), and water (6ml). After 18h the solution was washed with water, dried, and evaporated to give 3-methyl-5-isoxazolylmethanol as a yellow oil; $\delta_H$ ($CDCl_3$) 2.3 (3H, s, Me), 4.1 (1H, bs, OH), 4.8 (2H, s, $CH_2$), and 6.2 (1H, s, CH).

b) 3-Methylisoxazole-5-carboxylic acid

The above alcohol (6.8g, 50mmol) in aqueous sodium hydroxide (200ml, 0.5M) was treated with potassium manganate (VII) in water (300ml). After 1h dilute sulphuric acid and aqueous sodium sulphite were added until the solution was clear and acidic. Extraction with ether then gave 3-methylisoxazole-5-carboxylic acid as a white powder (3.8g, 59%); $\delta_H$ ($CDCl_3$-$C_3D_6O$) 2.4 (3H, s, Me), 6.9 (1H, s, het).

c) 3-Methylisoxaxole-5-carbonyl chloride

The above acid (0.45g, 3.5mmol) was dissolved in thionyl chloride (5ml)/DMF (5 drops) and the solution refluxed for 1h then evaporated to give crude 3-methylisoxazole-5-carbonyl chloride as a brown oil; $\nu_{max}$ - (film) 1770cm$^{-1}$.

d) 3-Methyl-5-diazoacetylisoxazole

The whole of the above crude acid chloride in ether (5ml) was added dropwise to diazomethane (ca. 10mmol) in ether (100ml) at 0°C. After another 1h at 0°C, excess diazomethane was blown off with argon. Evaporation and trituration with ether/hexane gave 3-methyl-5-diazoacetylisoxazole as cream needles; (0.4g, 76%); $\nu_{max}$ (film) 2130, 1610cm$^{-1}$; $\delta_H$ ($CDCl_3$), 2.4 (3H, s, Me), 6.1 (1H, s, $CH=N_2$), 6.8 (1H, s, het).

e) 2-Chloromethyl-5-(3-methyl-5-isoxazolyl)oxazole

The above diazoketone (0.15g, 1mmol) in choroacetonitrile (2ml) was added dropwise to a solution of boron trifluoride (0.24g, 3.6mmol) in more chloroacetonitrile (2ml) at 10°C. After a further 1h at 20°C water and ether were added. The organic layer was washed with brine, dried ($MgSO_4$) and evaporated. The residue was extracted with boiling hexane to give, after evaporation, 2-chloromethyl-5-(3-methyl-5-isoxazolyl)oxazole as white rhombs (0.16g, 80%); $\delta_H$ ($CDCl_3$) 2.4 (3H, s, Me), 4.7 (2H, s, $CH_2$), 6.5 (1H, s, isoxazole-H), 7.6 (1H, s, oxazole-H).

f) 2-Diethylphosphonomethyl-5-(3-methyl-5-isoxazolyl) oxazole

The above chloromethyloxazole (0.10g, 0.5mmol) and triethyl phosphite (0.16ml, 1.0mmol) were heated together at 150°C for 30min then the mixture purified by chromatography (silica, dichloromethane-methanol) to give 2-diethylphosphonomethyl-5-(3-methyl-5-isoxazolyl) oxazole as a yellow oil (0.12g, 80%); $\delta_H$ ($CDCl_3$) 1.4 (6H, t, ethyl-$CH_3$), 3.5 (2H, d, $J$ 22Hz, $PCH_2$), 4.3 (4H, m, ethyl-$CH_2$), 6.5 (1H, s, isoxazole-H), 7.5 (1H, s, oxazole-H).

g) Sodium hydride (24mg x 50% in oil, 0.5mmol) was added to a solution of the above phosphonate (120mg, 0.4mmol) in THF (2ml) at 0°C. Hydrogen was vigorously evolved. After 20min at 0°C the very dark solution was treated with a solution of the TMS-protected ketone (0.5mmol) in THF (5ml). After 1h without cooling saturated aqueous sodium hydrogen carbonate was added. The solution was extracted with ethyl acetate, and the extracts dried ($MgSO_4$) and evaporated and the resulting residue dissolved in a mixture of THF (20ml), water (5ml) and concentrated hydrochloric acid (5 drops). After 5min at 20°C saturated aqueous sodium hydrogen carbonate and ethyl acetate were added. The organic layer was separated, washed with brine, dried ($MgSO_4$), evaporated, and the residue purified by chromatography (silica gel, methanol/dichloromethane) to give a yellow foam (0.03g, 14%); $\nu_{max}$ (film) 1630cm$^{-1}$; $\lambda_{max}$ - (EtOH) 299nm ($\epsilon_m$ 25,000); $\delta_H$ ($CDCl_3$) 0.93 (3H, d, 17-$H_3$), 1.22 (3H, d, 14-$H_3$), 2.31, 2.33 (6H, 2s, het-Me, 15-$H_3$), 6.27, 6.29 (2H, s, 2-H isoxazole-H), 7.52 (1H, s, oxazole-H); $\delta_H$ ($CDCl_3$) 11.3 (het-Me), 12.7 (C-17), 19.6 (C-15), 20.8 (C-14), 31.6 (C-9), 39.6 (C-8), 42.8 (C-12), 42.9 (C-4), 55.5 (C-10), 61.3 (C-11), 65.4 (C-16), 68.9 (C-6), 70.4 (C-7), 71.4 (C-13), 75.0 (C-5), 101.1 (isoxazole C-4), 112.5 (C-2), 127.3 (oxazole C-4), 139.1 (oxazole C-5), 149.4 (C-3), 159.0 160.1, 162.3 (C-1, isoxazole C-3, -5); $m/z$ 448 ($M^+$, 12%), 204 (100).

## Example 5

### 5-(3-Bromo-5-isoxazolyl)-2-(1-normon-2-yl)oxazole

a) 3-Bromo-5-diazoacetylisoxazole

3-Bromoisoxazole-5-carboxylic acid (3.5g, 18mmol), thionyl chloride (10ml), and DMF (10 drops) were refluxed for 1h then evaporated and the residue dissolved in dichloromethane (5ml) and added slowly to diazomethane (ca. 50mmol) in ether (150ml) at 0°C. After 1h at 0°C excess diazomethane was blown off with argon and the solution evaporated to give 3-bromo-5-diazoacetylisoxazole as a pink powder from ether/hexane (2.3g, 58%); $\delta_H$ ($CDCl_3$) 6.1 (1H, s, -$CH=N_2$) and 7.0 (1H, s, het-H).

b) 5-(3-Bromo-5-isoxazolyl)-2-chloromethyloxazole

The above diazoketone (1.1g, 5mmol) in chloroacetonitrile (10ml) was added slowly to a solution of aluminium chloride (1.4g, 11mmol) in chloroacetonitrile (10ml) at 20°C. After 10min at 20°C water and ether were added. The organic layer was dried and evaporated to give 5-(3-bromo-5-isoxazolyl)-2-chloromethyl oxazole as white rhombs from hexane (1.0g, 76%); $\delta_H$ ($CDCl_3$) 4.7 (2H, s, $CH_2$), 6.7 (1H, s, isoxazole), and 7.7 (1H, s, oxazole); m/z 262 ($M^+$, 37%) and 227 (100).

c) 5-(3-Bromo-5-isoxazolyl)-2-diethylphosphonomethyloxazole

The above chloride (1.0g, 3.8mmol) and triethyl phosphite (0.6g, 4.0mmol) were heated together for 30min at 160°C then chromatographed (silica, methanol/dichloromethane) to give 5-(3-bromo-5-isoxazolyl)-2-diethylphosphonomethyloxazole as a yellow oil (0.6g, 43%); $\delta_H$ ($CDCl_3$) 1.3 (6H, m, Me), 3.5 (2H, d, J 22Hz, $CH_2P$), 4.2 (4H, m, $CH_2O$), 6.7 (1H, s, isoxazole), and 7.6 (1H, s, oxazole).

d) 5-(3-Bromo-5-isoxazolyl)-2-(1-normon-2-yl)oxazole

To a solution of the above phosphonate (0.12g, 0.33mmol) in DME (2ml) were added sodium hydride (0.02g, 50% in oil, 0.40mmol) and after 30min TMSprotected ketone (0.52g, 1.00mmol) in DME (2ml). After 1h at 35°C aqueous ammonium chloride and ethyl acetate were added. The organic layer was dried and evaporated and the residue dissolved in THF/0.1M hydrochloric acid (4:1, 50ml). After 10min ethyl acetate and aqueous sodium hydrogen carbonate were added and the organic layer washed with brine, dried, and evaporated and the residue chromatographed (silica, methanol/dichloromethane) to give the title compound as a white foam (0.035g, 21%); $\nu_{max}$ (film) 1640cm$^{-1}$; $\lambda_{max}$ (EtOH) 303nm ($\epsilon_m$ 24,800); $\delta_H$ ($CDCl_3$) 0.95 (3H, d, J 7.0Hz, 17-$H_3$), 1.22 (3H, d, J 7.0Hz, 14-$H_3$), 2.31 (3H, s, 15-$H_3$), 6.31 (1H, s, 2-H), 6.52 (1H, s, isoxazole), and 7.63 (1H,, s, oxazole); $\delta_C$ ($CDCl_3$) 12.7 (C-17), 19.7 (C-15), 20.8 (C-14), 31.6 (C-9), 39.6 (C-8), 42.8 (C-12), 43.0 (C-4), 55.5 (C-10), 61.3 (C-11), 65.4 (C-16), 68.9 (C-6), 70.4 (C-7), 71.4 (C-13), 75.0 (C-5), 103.5 (isoxazole-4), 112.3 (C-2), 128.7 (oxazole-4), 136.7 (isoxazole-3), 140.6 (oxazole-5), 150.5 (C-3), 160.9 (isoxazole-5), and 162.9 (C-1) m/z 512 ($M^+$, 8%) and 227 (79).

## Example 6

2-(1-Normon-2-yl)-5-(3-methoxy-5-isoxazolyl)oxazole and 2-(1-Norisomon-2-yl)-5-(3-methoxv-5-isoxazolyl)-oxazole

a) 5-Diazoacetyl-3-methoxyisoxazole

3-Methoxyisoxazole-5-carbxylic acid (1.5g, 10mmol), thionyl chloride (10ml), and DMF (1 drop) were refluxed for 1h then evaporated and the residue dissolved in dichloromethane (5ml) and added slowly to diazomethane (ca. 35mmol) in ether (100ml) at 0°C. After 1h at 0°C excess diazomethane was blown off with argon and the solution evaporated to give 5-diazoacetyl-3-methoxyisoxazole a light brown rhombs from ether/hexane (1.7g, 60%); $\delta_H$ ($CDCl_3$-$CD_3OD$) 3.8 (3H, s, $CH_3$), 6.0 (1H, s, -$CH=N_2$), and 6.3 (1H, s, isoxazole).

b) 2-Chloromethyl-5-(3-methoxv-5-isoxazolyl)oxazole

The above diazoketone (1.5g, 9mmol) in chloroacetonitrile (20ml) was added slowly to aluminium chloride (2.7g, 20mmol) in chloroacetonitrile (20ml) at 20°C. After 30min at 20°C water and ether were added. The organic layer was dried and evaporated and the residue chromatographed (silica, ether/hexane) to give 2-chloromethyl-5-(3-methoxy-5-isoxazolyl)oxazole as a white powder (0.7g, 37%); $\delta_H$ ($CDCl_3$) 4.0 (3H, s, $CH_3$), 4.7 (2H, s, $CH_2$), 6.2 (1H, s, isoxazole), and 7.6 (1H, s, oxazole); m/z 214 ($M^+$, 48%) and 179 (100).

c) 2-Diethylphosphonomethyl-5-(3-methoxy-5-isoxazolyl)oxazole

The above chloride (0.43g, 2mmol) and triethyl phosphite (0.32ml, 2mmol) were heated together for 1h at 160°C then chromatographed (silica, menthol/dichloromethane) to give 2-diethylphosphonomethyl-5-(3-methoxy-5-isoxazolyl)oxazole as a yellow oil (0.56g, 89%); $\delta_H$ ($CDCl_3$) 1.3 (6H, m, $CCH_3$), 3.5 (2H, d, J 22Hz, $PCH_2$), 4.2 (7H, m, $OCH_2$, $OCH_3$), 6.2 (1H, isoxazole), and 7.5 (1H, s, oxazole).

d) 2-(1-Normon-2-yl)-5-(3-methoxy-5-isoxazolyl)oxazole

To a solution of the above phosphonate (0.57g, 1.8mmol) in DME (3ml) were added at 0°C, sodium hydride (0.10g, 50% in oil, 2.0mmol), and after 20min at 0°C, TMS-protected ketone (1.04g, 2.0mmol) in DME (2ml). After 1.5h at 20°C aqueous ammonium chloride and ethyl acetate were added. The organic layer was dried and evaporated and the residue dissolved in THF/0.1M hydrochloric acid (4:1, 100ml). After 10min ethyl acetate and aqueous sodium hydrogen carbonate were added and the organic layer washed with brine, dried, and evaporated and the residue chromatographed (silica, methanol/dichloromethane) to give: Z-isomer as a white foam (0.11g, 13%); $\nu_{max}$ (KBr) 1660cm$^{-1}$; $\lambda_{max}$ (EtOH) 298nm ($\epsilon_m$ 19,600); $\delta_H$ ($CDCl_3$) 0.93 (3H, d, J 7.0Hz, 17-$H_3$), 1.21 (3H, d, J 7.0Hz, 14-$H_3$), 2.14 (3H, d, J 0.9Hz, 15-$H_3$), 4.03 (3H, s, $OCH_3$), 6.12 (1H, s, isoxazole), 6.29 (1H, s, 2-H), and 7.47 (1H, s, oxazole); $\delta_C$ ($CDCl_3$) 12.7 (C-17), 20.6 (C-14), 27.6 (C-15), 31.7 (C-9), 36.2 (C-4), 38.8 (C-8), 42.9 (C-12), 56.0 (C-10), 57.4 (OMe), 61.4 (C-11), 65.5 (C-16), 67.1 (C-6), 70.1 (C-7), 71.3 (C-13), 76.9 (C-5), 92.2 (isoxazole-4), 111.7 (C-2), 126.3 (oxazole-4), 139.2 (oxazole-5), 153.4 (C-3), 159.2 (isoxazole-5), 162.1 (C-1), and 172.4

(isoxazole-3); m/z 464 ($M^+$, 11%) and 220 (100); and the E-isomer as a white foam (0.18g, 22%); $\nu_{max}$ - (KBr) 1660cm$^{-1}$; $\lambda_{max}$ (EtOH) 298nm ($\epsilon_m$ 23,500); $\delta_H$ ($CDCl_3$) 0.95 (3H, d, J 7.0Hz, 17-$H_3$), 1.22 (3H, d, J 7.0Hz, 14-$H_3$), 2.31 (3H, s, 15-$H_3$), 4.05 (3H, s, OMe), 6.07 (1H, s, isoxazole), 6.26 (1H, s, 2-H) and 7.51 (1H, s, oxazole); $\delta_C$ ($CDCl_3$) 12.7 (C-17), 19.7 (C-15), 20.8 (C-14), 31.6 (C-9), 39.6 (C-8), 42.8 (C-12), 43.0 (C-4), 55.5 (C-10), 57.3 (OMe), 61.3 (C-11), 65.4 (C-16), 68.9 (C-6), 70.4 (C-7), 71.4 (C-13), 75.0 (C-5), 91.6 (isoxazole-4), 112.3 (C-2), 127.4 (oxazole-4), 139.9 (oxazole-5), 149.9 (C-3), 159.8 (isoxazole-5), 162.4 (C-1), and 172.4 (isoxazole-3); m/z 464 ($M^+$, 9%) and 220 (100).

## Example 7

### 2-(1-Normon-2-yl)-5-(3-isopropyl-5-isoxazolyl)oxazole

a) Methyl 3-isopropylisoxazole-5-carboxylate

A solution of isobutyraldoxime (8.5g, 0.10mmol) in dichloromethane (100ml) was added slowly to a mixture of 1.3M aqueous sodium hypochlorite (100ml, 0.13mmol), methyl propiolate (8.9ml, 0.10mmol), and dichloromethane (100ml) at 0°C to 10°C (internal). After 1h at this temperature the organic layer was dried and evaporated to give methyl 3-isopropyl-isoxazole-5-carboxylate as a blue oil (11.5g, 70%); $\delta_H$ ($CDCl_3$) 1.3 (6H, d, J 7.0Hz, CMe), 3.2 (1H, sept, J 7.0Hz, CHMe$_2$), 4.0 (3H, s, OMe), and 6.9 (1H, s, isoxazole).

b) 3-Isopropylisoxazole-5-carboxylic acid

The above ester (11.5g, 170mmol) and 1M aqueous sodium hydroxide (75ml, 75mmol) were heated to 100°C then cooled, acidified and extracted with ether which was dried and evaporated to give 3-isopropylisoxazole-5-carboxylic acid as a pale green oil (10.8g, 89%); $\delta_H$ ($CDCl_3$) 1.3 (6H, d, J 7.0Hz, Me), 3.2 (1H, sept, J 7.0Hz, CHMe$_2$), and 7.0 (1H, s, isoxazole).

c) 5-Diazoacetyl-3-isopropylisoxazole

The above acid (4.8g, 31mmol), and thionyl chloride (15ml) were refluxed together for 1h then evaporated and the residue added slowly to diazomethane (ca. 70mmol) in ether (200ml) at 0°C. After 1h at 0°C excess diazomethane was blown off with argon and the solution evaporated to give 5-diazoacetyl-3-isopropylisoxazole as a yellow solid from ether/hexane (1.7g, 32%); $\delta_H$ ($CDCl_3$) 1.3 (6H, d, J 7.0Hz, Me), 3.2 (1H, sept, J 7.0Hz, CHMe$_2$), 6.1 (1H, s, $-CH=N_2$), and 6.9 (1H, s, isoxazole).

d) 2-Chloromethyl-5-(3-isopropyl-5-isoxazolyl)oxazole

The above diazoketone (0.9g, 5mmol) in chloroacetonitrile (5ml) was added slowly to a solution of aluminium chloride (1.6g, 12mmol) in chloroacetonitrile (5ml) at 20°C. After 5 min at 20°C, ether and water were added. The organic layer was dried and evaporated to give 2-chloromethyl-5-(3-isopropyl-5-isoxazolyl)oxazole as a low m.p. yellow solid from hexane (0.16g, 18%); $\delta_H$ ($CDCl_3$) 1.3 (6H, d, J 7.0Hz, Me), 3.2 (1H, sept, J 8.0Hz, CHMe$_2$), 4.7 (2H, s, $CH_2$), 6.5 (1H, s, isoxazole), and 7.6 (1H, s, oxazole).

e) 2-Diethylphosphonomethyl-5-(3-isopropyl-5-isoxazolyl)oxazole

The above chloride (0.16g, 0.9mmol) and triethyl phosphite (0.16ml, 1.0mmol) were heated together for 1h at 160°C then cooled and chromatographed (silica, methanol/dichloromethane) to give 2-diethylphosphonomethyl-5-(3-isopropyl-5-isoxazolyl)oxazole as a yellow oil (0.16g, 54%); $\delta_H$ ($CDCl_3$) 1.4 (12H, m, Me), 3.2 (1H, sept, J 7.0Hz, CHMe$_2$), 3.5 (2H, d, J 22Hz, $CH_2P$), 4.2 (4H, m, $CH_2O$), 6.5 (1H, s, isoxazole), and 7.6 (1H, s, oxazole).

f) 2-(1-Normon-2-yl)-5-(3-isopropyl-5-isoxazolyl)oxazole

To a solution of the above phosphonate (0.16g, 0.50mmol) in DME (3ml) was added, at 0°C, sodium hydride (24mg, 55% in oil, 0.55mmol), and after 5min at 0°C, TMS-protected ketone (0.26g, 0.50mmol). After 2h at 20°C, aqueous ammonium chloride and ethyl acetate were added. The organic layer was dried and evaporated and the residue dissolved in THF/0.1M hydrochloric acid (4:1, 25ml). After 10min ethyl acetate and aqueous sodium hydrogen carbonate were added and the organic layer washed with brine, dried, and evaporated and the residue chromatographed (silica, methanol/dichloromethane) to give the title compound as a white foam (30mg, 13%); $\nu_{max}$ (KBr) 1650cm$^{-1}$; $\lambda_{max}$ (EtOH) 300nm ($\epsilon_m$ 26,000); $\delta_H$ ($CDCl_3$) 0.95 (3H, d, J 7.0Hz, 17-$H_3$), 1.22 (3H, d, J 7.0Hz, 14-$H_3$), 1.32 (6H, d, J 7.0Hz, CHMe$_2$), 2.31 (3H, s, 15-$H_3$), 3.10 (1H, sept, J 7.0Hz, CHMe$_2$), 6.26 (1H, s, 2-H), 6.35 (1H, s, isoxazole), and 7.51 (1H, s, oxazole); $\delta_C$ ($CDCl_3$) 12.8 (C-17), 19.7 (C-15), 20.8 (C-14), 21.7 (CHMe$_2$), 26.4 (CHMe$_2$), 31.6 (C-9), 39.6 (C-8), 42.8 (C-12), 43.0 (C-4), 55.5 (C-10), 61.3 (C-11), 65.4 (C-16), 68.9 (C-6), 709.4 (C-7), 71.4 (C-13), 75.5 (C-5), 98.6 (isoxazole-4), 112.6 (C-2), 127.2 (oxazole-4), 139.2 (oxazole-5), 149.2 (C-3), 158.9 (isoxazole-5), 162.2 (C-1), and 169.6 (isoxazole-3); m/z 476 ($M^+$, 11%) and 232 (84) (Found: $M^+$ 476.2517. $C_{25}H_{36}N_2O_7$ requires M 476.2523).

Example 8

5-(3-t-Butyl-5-isoxazolyl)-2-(1-normon-2-yl)oxazole

a) Methyl 3-t-butylisoxazole-5-carboxylate

A solution of pivaldoxime (7.5g, 0.074mmol) in dichloromethane (75ml) was added slowly to a well stirred mixture of methyl propiolate (6.7ml, 75mmol), aqueous sodium hypochlorite (75ml) at 0°C to 10°C (internal). After 1h at 20°C the organic layer was dried and evaporated to give methyl 3-t-butylisoxazole-5-carboxylate as a green oil (9.1g, 65%); $\delta_H$ ($CDCl_3$) 1.3 (9H, s, $^tBu$), 3.8 (3H, s, OMe), and 6.8 (1H, s, het).

b) 3-t-Butylisoxazole-5-carboxylic acid

The above ester (9.1g, 50mmol) and aqueous sodium hydroxide (75ml, 1M, 75mmol) were heated to 100°C, cooled, acidified, and extracted with ether, which was dried and evaporated to give 3-t-butylisoxazole-5-carboxylic acid as a white powder (7.9g, 96%); $\delta_H$ ($CDCl_3$) 1.4 (9H, s, $^tBu$), 7.1 (1H, s, het), and 11.4 (1H, s, OH).

c) 3-t-Butyl-5-diazoacetylisoxazole

The above acid (5.1g, 30mmol), thionyl chloride (10ml), and DMF (0.1ml) were refluxed together for 1h then evaporated and the residue added to diazomethane (ca. 70mmol) in ether (200ml) at 0°C. After 1h at 0°C excess diazomethane was blown off with argon and the solution evaporated to give 3-t-butyl-5-diazoacetylisoxazole as a yellow solid from ether/hexane (3.3g, 57%); $\delta_H$ ($CDCl_3$) 1.4 (9H, s, $^tBu$), 6.1 (1H, s, $-CH=N_2$), and 6.9 (1H, s, het).

d) 5-(3-t-Butyl-5-isoxazolyl)-2-chloromethyloxazole

The above diazoketone (1.0g, 5mmol) in chloroacetonitrile (5ml) was added slowly to a solution of boron trifluoride (1.2g, 18mmol) in chloroacetonitrile (10ml) at 20°C. After 5min hydrochloric acid and ether were added. The organic layer was washed with brine, dried, and evaporated to give 5-(3-t-butyl-5-isoxazolyl)-2-chloromethyloxazole as a yellow oil (0.9g, 75%); $\delta_H$ ($CDCl_3$) 1.4 (9H, s, $^tBu$), 4.8 (2H, s, $CH_2$), 6.6 (1H, s, isoxazole), and 7.6 (1H, s, oxazole).

e) 5-(3-t-Butyl-5-isoxazolyl)-2-diethylphosphonomethyloxazole

The above chloride (0.9g, 3.8mmol) and triethyl phosphite (0.8ml, 5.0mmol) were heated together for 1h at 160°C then cooled and chromatographed (silica, methanol/dichloromethane) to give 5-(3-t-butyl-5-isoxazolyl)-2-diethylphosphonomethyloxazole as a yellow oil (0.9g, 70%); $\delta_H$ ($CDCl_3$) 1.4 (15H, m, Me), 3.5 (2H, d, J 22Hz, $CH_2P$), 4.2 (4H, m, $CH_2O$), 6.5 (1H, s, isoxazole), and 7.6 (1H, s, oxazole).

f) 5-(3-t-Butyl-5-isoxazolyl)-2-(1-normon-2-yl)oxazole

To a solution of the above phosphonate (0.34g, 1mmol) in DME (2ml) at 20°C were added sodium hydride (50mg, 50%, 1mmol) and after 10min at 20°C, TBDMSprotected ketone (0.65g, 1mmol) in DME (2ml). After 4h, aqueous ammonium chloride and ethyl acetate were added. The organic layer was dried and evaporated and the resulting residue chromatographed (silica, ether/hexane) to give crude protected title compound as an oil which was dissolved in THF (20ml) containing tetra-n-butylammonium fluoride dihydrate (1.6g, 5mmol). After 16h at 20°C, ethyl acetate and water were added. The organic layer was dried and evaporated and the residue chromatographed twice (silica; menthanol/dichloromethane then methanol/methyl acetate) to give the title compound as a white foam (80mg, 16%); $\nu_{max}$ (KBr), 1650cm$^{-1}$; $\lambda_{max}$ (EtOH) 299nm ($\epsilon_m$ 23,700); $\delta_H$ 0.95 (3H, d, J 7.0Hz, 17-$H_3$), 1.23 (3H, d, J 7.0Hz, 14-$H_3$), 1.47 (9H, s, $^tBu$), 6.28 (1H, s, H-2), 6.39 (1H, s, isoxazole), and 7.52 (1H, s, oxazole); $\delta_C$ ($CDCl_3$) 12.7 (C-17), 19.7 (C-15), 20.6 (C-14), 29.5 ($CMe_3$), 31.6 ($CMe_3$), 42.1 (C-9), 39.6 (C-8), 42.8 (C-12), 43.0 (C-4), 55.4 (C-10), 61.4 (C-11), 65.4 (C-16), 68.9 (C-6), 70.4 (C-7), 71.4 (C-13), 75.0 (C-5), 98.6 (isoxazole-4), 112.4 (C-2), 127.1 (oxazole-4), 139.3 (oxazole-5), 149.4 (C-3), 158.7 (isoxazole-5), 162.2 (C-1), and 172.4 (isoxazole-3); m/z 490 ($M^+$, 8%) and 246 (100) (Found; $M^+$ 490.2654. $C_{26}H_{38}N_2O_7$ requires M 490.2679).

Example 9

2-(1-Normon-2-yl)-5-(3-ethoxycarbonyl-5-isoxazolyl)oxazole

a) Ethyl 5-hydroxymethylisoxazole-3-carboxylate

A solution of ethyl chlorooximinoacetate (30g, 0.2mmol) in ethyl acetate (200ml) was added dropwise to a mixture of propargyl alcohol (24ml, 0.4mmol), potassium hydrogen carbonate (60g, 0.6mmol), ethyl acetate (800ml), and water (10ml) at 20°C. After 24h at 20°C the solution was washed with water, dried ($MgSO_4$), and evaporated to give the title compound as a yellow oil (23g, 61%); $\delta_H$ ($CDCl_3$) 1.4 (3H, t, J 7.0Hz, Me), 4.5 (2H, q, J 7.0Hz, $CH_2$-Me), 5.0 (2H, s, $CH_2$-OH), and 6.8 (1H, s, het-H).

b) 3-Ethoxycarbonylisoxazole-5-carboxylic acid

The above alcohol (20.4g, 0.12mmol) in acetone (120ml) was treated dropwise with Jones reagent (100ml, 2M in chromic acid, 0.20mmol) at 15 °C to 20 °C (internal). After 30min brine and ether were added. The organic layer was washed with aqueous sodium hydrogen sulphite and brine then extracted with aqueous sodium hydrogen carbonate. This extract was taken to pH 2 then extracted twice with ether. The combined extracts were washed with brine, dried ($MgSO_4$), and evaporated to give the title compound as a white powder (16g, 70%); $\delta_H$ (acetone-$d_6$) 1.4 (3H, t, J 7.0Hz, $CH_3$), 4.5 (2H, 2, J 7.0Hz, $CH_2$), and 7.5 (1H, s, isoxazole).

c) Ethyl 5-diazoacetylisoxazole-3-carboxylate

The above acid (6.0g, 30mmol) was refluxed with thionyl chloride (10ml) and DMF (0.1ml) for 2h then evaporated to give the acid chloride as a yellow oil, which was added to diazomethane (ca. 70mmol) in ether (200ml) at 0 °C. After 30min at 0 °C evaporation gave the title compound as needles from ether/hexane, containing some chloroketone (4.8g, 48%); $\delta_H$ ($CDCl_3$) 1.45 (3H, t, J 7.0Hz, $CH_3$), 4.52 (2H, q, J 7.0Hz, $CH_2$), 6.24 (1H, s, het), and 7.37 (2H, s, $CHN_2$).

d) 2-Chloromethyl-5-(3-ethoxycarbonyl-5-isoxazolyl)oxazole

The above diazoketone (4.8g, 15mmol) in chloroacetonitrile (15ml) was added to boron trifluoride (3.3g, 50mmol) in more chloroacetonitrile (30ml) at 20 °C. After 15min water and ether were added, and the organic layer dried ($MgSO_4$) and evaporated to give an oil which was purified by chromatography (silica, ether/hexane) to give the title compound again contaminated with chloroketone (5.4g, 98%); $\delta_H$ ($CDCl_3$) 1.45 (3H, t, J 7.0Hz, $CH_3$), 4.52 (2H, q, J 7.0Hz, $MeCH_2$), 4.84 (2H, s, $CH_2Cl$), 7.05 (1H, s, isoxazole), and 7.71 (1H, s, oxazole).

e) 2-Diethylphosphonomethyl-5-(3-ethoxycarbonyl-5-isoxazolyl)oxazole

The above chloromethyloxazole (0.8g, 2mmol) and triethyl phosphite (0.64ml, 4mmol) were heated together for 1h at 160 °C then cooled and chromatographed (silica, methanol/dichloromethane), to give the title compound as a yellow oil (0.5g, 70%); $\delta_H$ ($CDCl_3$) 1.4 (6H, m, Me), 3.52 (2H, d, J 22.0Hz, $CH_2P$), 4.3 (6H, m, $CH_2O$), 7.01 (1H, s, isoxazole), 7.69 (1H, s, oxazole).

f) 2-(1-Normon-2-yl)-5-(3-ethoxycarbonyl-5-isoxazolyl)oxazole

To a solution of the above phosphonate (0.57g, 1.8mmol) in DME (3ml) were added at 0 °C, sodium hydride (0.10g x 50% in oil, 2.0mmol), and after 20min at 0 °C, TMS-protected ketone (1.04g, 2.0mmol) in DME (2ml). After 1.5h at 20 °C aqueous ammonium chloride and ethyl acetate were added. The organic layer was dried and evaporated and the residue dissolved in THF/0.1M hydrochloric acid (4:1, 100ml). After 10min ethyl acetate and aqueous sodium hydrogen carbonate were added and the organic layer washed with brine, dried, and evaporated and the residue chromatographed (silica, methanol/dichloromethane) to give the title compound as a white foam (0.20g, 20%); $\nu_{max}$ (film) 1740 and 1640cm$^{-1}$; $\lambda_{max}$ (EtOH) 306nm ($\epsilon_m$ 20,700); $\delta_H$ ($CDCl_3$) 0.95 (3H, d, J 7.0Hz, 17-$H_3$), 1.22 (3H, d, J 7.0Hz, 14-$H_3$), 1.46 (3H, t, J 7.0Hz, ester $CH_3$), 2.31 (3H, s, 15-$H_3$), 4.50 (2H, q, J 7.0Hz, ester $CH_2$), 6.31 (1H, s, 2-H), 6.88 (1H, s, isoxazole), and 7.63 (1H, s, oxazole); $\delta_C$ ($CDCl_3$) 12.7 (C-17), 14.1 (ester-$CH_3$), 19.7 (C-15), 20.8 (C-15), 20.8 (C-14), 31.6 (C-9), 39.6 (C-8), 42.8 (C-12), 43.0 (C-4), 55.5 (C-10), 61.3 (C-11), 62.4 (ester-$CH_2$), 65.5 (C-16), 68.9 (C-6), 70.4 (C-7), 71.3 (C-13), 75.0 (C-5), 100.6 (isoxazole-4), 112.2 (C-2), 120.5 (oxazole-4), 1348.1 (oxazole-5), 150.5 (C-3), 156.7 (ester CO), 159.5 (isoxazole-5), 161.0 (C-1), and 162.9 (isoxazole-3); m/z 506 ($M^+$, 4%) and 262(100). (Found; $M^+$, 506.2266. $C_{25}H_{34}N_2O_9$ requires M, 506.2264).

## Example 10

### 2-(1-Normon-2-yl)-5-(3-formyl-5-isoxazolyl)oxazole

2-(1-Normon-2-yl)-5-(3-ethoxycarbonyl-5-isoxazolyl)oxazole (0.25g, 0.5mmol) (Example 9) in THF (10ml) at 20 °C was treated with triethylamine (0.28ml, 2.0mmol) and chlorotrimethylsilane (0.26ml, 2.0mmol) for 3h then filtered and evaporated. The residue in dichloromethane (5ml) at 0 °C was treated with diisobutylaluminium hydride (0.7ml x 1.5M in toluene, 1.0mmol) for 15min then quenched with methanol and evaporated. The residue was dissolved in THF/water (4:1, 25ml) and hydrochloric acid (5M, 20 drops) added. After 5min ethyl acetate was added and the solution washed with aqueous sodium hydrogen carbonate, dried and evaporated. The residue was chromatographed (silica, methanol/dichloromethane) to give the title compound
(0.15g, 56%); $\nu_{max}$ (film) 1710cm$^{-1}$; $\lambda_{max}$ (EtOH) 300nm ($\epsilon_m$ 26,600); $\delta_H$ (DMSO-$d_6$) 0.83 (3H, d, J 7.0Hz, 17-$H_3$), 1.15 (3H, d, J 7.0Hz, 14-$H_3$), 2.32 (3H, s, 15-$H_3$), 6.25 (1H, s, 2-H), 7.31 (1H, s, isoxazole), 8.02 (1H, s, oxazole), 10.13 (1H, s, CHO); $\delta_C$ ($CDCl_3$) 12.5 (C-17), 19.7 (C-15), 20.6 (C-17), 31.7 (C-9), 39.6 (C-8), 4.6 (C-12), 43.0 (C-4), 54.5 (C-10), 61.1 (C-11), 65.6 (C-16), 68.6 (C-6), 70.2 (C-7), 70.9 (C-13), 75.1 (C-5), 97.2 (isoxazole-4), 112.3 (C-2), 128.7 (oxazole-4), 138.9 (oxazole-5), 151.2 (C-3), 162.0, 162.2, 162.4 (C-1, isoxazole-3 and -5), and 184.2 (CHO); m/z 462 ($M^+$, 17%) and 218(100). (Found; $M^+$, 462.2011. $C_{23}H_{30}N_2O_4$ requires M, 462.2002).

Example 11

2-(1-Normon-2-yl)-5-(3-hydroxymethyl-5-isoxazolyl)oxazole

Sodium borohydride (5mg, 0.12mmol) was added to 2-(1-normon-2-yl)-5-(3-formyl-5-isoxazolyl)oxazole (Example 10) (23mg, 0.045mmol) in methanol (1ml) at 20 °C. After 30min acetic acid (3 drops) was added and the solution was evaporated and chromatographed to yield the title compound (16mg, 74%); $\nu_{max}$ (KBr) 1650cm$^{-1}$; $\lambda_{max}$ (EtOH) 300nm ($\epsilon_m$ 23,200); $\delta_H$ ($CDCl_3$) 0.95 (3H, d, J 7.0Hz, 17-$H_3$), 1.23 (3H, d, J 7.0Hz, 14-$H_3$), 2.29 (3H, s, 15-$H_3$), 4.73 (2H, s, $CH_2OH$), 6.31 (1H, s, 2-H), 6.61 (1H, s, isoxazole), 7.57 (1H, s, oxazole); $\delta_C$ ($CDCl_3$)
12.5 (C-17), 19.7 (C-15), 20.7 (C-14), 31.7 (C-9), 39.6 (C-8), 42.6 (C-12), 4.9 (C-4), 55.6 (C-10), 56.2 ($CH_2OH$), 65.6 (C-16), 68.6 (C-6), 70.2 (C-7), 71.1 (C-13), 75.1 (C-5), 99.5 (isoxazole-4), 112.3 (C-2), 127.4 (oxazole-4), 139.0 (oxazole-5), 150.2 (C-3), 159.4 (isoxazole-5), 162.6 (C-1), 164.4 (isoxazole-3); m/z 464 ($M^+$, 9%), and 220(100). (Found: $M^+$, 464.2128. $C_{23}H_{32}N_2O_8$ requires 464.2159).

Example 12

2-(1-Normon-2-yl)-5-(3-methylisoxazol-5-yl)oxazole

a) 3-Methylisoxazole-5-carboxaldehyde
A mixture of 3-methylisoxazole-5-methanol (2g), active manganese dioxide (3g), and benzene (20ml) was refluxed under a Dean and Stark trap for 2h then filtered and evaporated to give the aldehyde as white plates (1.0g, 49%); $\delta_H$ ($CDCl_3$) 2.5 (3H, s, Me), 7.0 (1H, s, isoxazole), and 10.1 (1H, s, CHO).

b) Tris(trimethylsilyl) N-dimethylaminomethylmonamide
(i) A mixture of monamide (1.7g, 5mmol), triethylamine (2.8ml, 20mmol), chlorotrimethylsilane (2.5ml, 20mmol), and THF (50ml) was stirred for 2h at 20 °C then filtered and evaporated and the residue purified by chromatography (silica, dichloromethane-methanol) to give tris(trimethylsilyl)monamide as a colourless oil.

This was dissolved in N,N,N′,N′-tetramethylmethylene diamine (20ml) containing acetic acid (20 drops). After 18h at 20 °C evaporation and chromatography (silica, methanol-dichloromethane) gave tris(trimethyl)silyl N-(dimethylaminomethyl)monamide as a white foam, (0.5g, 20%); $\nu_{max}$ (film) 3300, 1660, 1640 and 1250cm$^{-1}$; $\delta_H$ ($CDCl_3$) 0.92 (3H, d, J 7.0Hz, 17-$H_3$), 1.21 (3H, d, J 7.0Hz, 14-$H_3$), 2.22 (3H, s, 15-$H_3$), 2.29 (6H, s, $NMe_2$), 4.11 (2H, d, J 6.0Hz, 1′-$H_2$), 5.62 (1H, s, 2-H) and 6.0 (1H, bt, NH).

(ii) Eschenmoser's salt (N,N-dimethylmethyleneammonium iodide, 0.4g, 2.0mmol) was added to a solution of tris(trimethylsilyl)monamide (1.0g, 1.8mmol) in tetrahydrofuran (20ml) at 20 °C. After 1h at 20 °C the solution was partitioned between aqueous sodium hydrogen carbonate and ethyl acetate. The organic layer was dried ($MgSO_4$) and evaporated and the residue purified by chromatography (silica, methanol/dichloromethane) to give the title compound identical to that prepared above (0.8g, 72%).

c) Tris(trimethylsilyl) N-tosylmethylmonamide
The above tris(trimethylsilyl) N-dimethylaminomethyl monamide (1.0g, 1.6mmol) in tetrahydrfuran (10ml) at 0 °C was treated with iodomethane (0.6ml, 19mmol). After 60min the mixture was evaporated to dryness and DMF (20ml) and sodium p-toluenesulphonate (0.5g, 2.3mmol) and triethylamine (0.6ml, 4.3mmol) were added. After 18h sodium hydrogen carbonate/ethyl acetate partition and chromatography (silica, methanol/dichloromethane) gave tris(trimethylsilyl) N-(tosylmethyl)monamide as a colourless oil, (1.00g, 85%); $\delta_H$ ($CDCl_3$) 0.91 (3H, d, J 7.0Hz, 17-$H_3$), 1.23 (3H, d, J 7.0Hz, 14-$H_3$), 2.02 (3H, s, 15-$H_3$), 2.42 (3H, s, aryl-Me), 4.72 (2H, d, J 7.0Hz, 1′-$H_2$), 5.59 (1H, s, 2-H), 6.10 (1H, t, J 7.0Hz, NH), 7.28 (2H, d, J 8.0Hz, aryl), and 7.75 (2H, d, J 8.0Hz, aryl).

d) 2-(1-Normon-2-yl)-5-(3-methyl-5-isoxazolyl)oxazole
Carbon tetrachloride (0.15ml, 1.5mmol) was added to a solution containing the above tris(trimethylsilyl) N-tosylmethylmonamide (90mg, 0.12mmol), 3-methylisoxazole-5-carboxaldehyde (33mg, 0.30mmol), triphenylphosphine (120mg, 0.45mmol), triethylamine (0.09ml, 0.60mmol), and acetonitrile (1ml) at 20 °C. After 2h at 20 °C aqueous sodium hydrogen carbonate/ethyl acetate partition and chromatography (silica,

ether/hexane) gave tris(trimethylsilyl) 5-(3-methyl-5-isoxazolyl)-2-(1-normon-2-yl)-4-tosyl-2-isoxazoline as a white foam (0.055g, 56%); $\delta_H$ ($CDCl_3$) 0.91 (3H, d, J 7.0Hz, 17-$H_3$), 1.21 (3H, d, J 7.0Hz, 14-$H_3$), 2.13 (3H, s, 15-$H_3$), 2.30 (3H, s, het-Me), 2.43 (3H, s, aryl-Me), 5.36 (1H, d, J 5.0Hz, oxazoline-5H), 5.82 (1H, s, 2-H), 6.03 (1H, d, J 5.0Hz, oxazoline-4H), 6.21 (1H, s, isoxazole-H), 7.37 (2H, d, J 8.0Hz, aryl) and 7.82 (2H, d, J 8.0Hz, aryl).

A mixture of this oxazoline (55mg, 0.07mmol), DBU (2 drops), and aceonitrile (1ml) was stirred for 1h at 20 °C, then ethyl acetate/aqueous ammonium chloride partition and chromatography (silica, ether/hexane) gave tris(trimethylsilyl) protected title compound which was deprotected as in the previous preparation to give the title compound (20mg, 49%) identical to example 4.

Example 13

2-(1-Normon-2-yl)-5-(3-pyrrolidin-1-yl-5-isoxazolyl)oxazole

a) 3-(1-Pyrrolidinyl)isoxazole-5-carboxylic acid

3-Bromoisoxazole-5-carboxylic acid (5g, 26mmol) and pyrrolidine (50ml, 600mmol) were refluxed at 80 °C for 66h then added slowly to 5M hydrochloric acid (125ml) to give a resulting pH of 2. The product was extracted using diethyl ether, the organic layer separated, dried ($MgSO_4$), and evaporated to give 3-(1-pyrrolidinyl)isoxazole-5-carboxylic acid as white rhombs (2.31g, 49%); $\delta_H$ ($CDCl_3$) 2.0 (4H, m, $CH_2$), 3.3 (4H, M, N-$CH_2$) and 6.45 (1H, s, isoxazole).

b) 3-(1-Pyrrolidinyl)isoxazole-5-carboxaldehyde

To the above acid (2.28g, 12.5mmol) in tetrahydrofuran were added triethylamine (1.7ml, 12.5mmol) and isobutylchloroformate (4.7ml, 12.5mmol) under nitrogen at 0 °C. After 30min N,O dimethylhydroxylamine (3.66g, 37.5mmol) in dichloromethane (36ml) was added. After 1¼h at 0 °C water was added and the organic layer separated, dried ($MgSO_4$) and evaporated. To the product (0.9g, 4mmol) in tetrahydrofuran (7ml) was added diisobutylaluminium hydride in toluene (14ml, excess) at 0 °C under nitrogen. Methanol (30ml) and 1M hydrochloric acid were added and the product extracted with ethyl acetate, dried ($MgSO_4$), and evaporated and the residue chromatographed (silica, ethyl acetate) to give the title compound as a colourless oil (0.214g, 33%); $\delta_H$ ($CDCl_3$) 2.0 (4H, m, $CH_2$), 3.3 (4H, m, N-$CH_2$), 6.4 (1H, s, isoxazole) and 9.75 (1H, s, aldehyde).

c) 2-(1-Normon-2-yl)-5-(3-pyrrolidin-1-yl-5-isoxazolyl)oxazole

To a solution of the above aldehyde (0.275g, 1.66mmol) in acetonitrile (35ml) were added at 20 °C, tris-(trimethylsilyl) N-tosylmethylmonamide (1.513g, 2.08mmol), triphenylphosphine (1.749g, 6.66mmol), triethylamine (1.38ml, 10mmol) and hexachloroethane (3.95g, 16.66mmol). After 15min aqueous sodium hydrogen carbonate and ethyl acetate were added and the organic layer separated dried ($MgSO_4$), evaporated and the residue chromatographed (silica, ethyl acetate/hexane). To a solution of the product in acetonitrile (3ml) was added four drops of DBU at 20 °C. After 1¼h aqueous ammonium chloride solution and ethyl acetate were added, the organic layer separated dried ($MgSO_4$) and evaporated. To the residue were added tetrahydrofuran (4ml), water (1ml) and two drops of 5M hydrochloric acid. After 5min aqueous sodium hydrogen carbonate solution and ethyl acetate were added, the organic layer separated, dried ($MgSO_4$), evaporated and the residue chromatographed (silica, methanol/dichloromethane) to give the title compound as a colourless oil (0.039g, 13%); $\nu_{max}$ (KBr) 1655cm$^{-1}$; $\lambda_{max}$ (EtOH) 299nm ($\epsilon_m$ 22,200); $\delta_H$ ($CDCl_3$) 0.94 (3H, d, $CH_3$), 1.22 (3H, d, $CH_3$), 2.0 (4H, m, $CH_2$), 2,21 (3H, s, $CH_3$), 3.35 (4H, m, N-$CH_2$), 6.0 (1H, s, isoxazole), 6.25 (1H, s, olefin), 7.5 (1H, s, oxazole); m/z 503 ($M^+$, 30%) and 259 (100).

Example 14

5-(2-Furyl)-2-(1-normon-2-yl)oxazole

a) 2-Aminoacetylfuran hydrochloride

Bromine (2.1ml) was added to a solution of 2-acetylfuran (5.5g) in ether (100ml). When no bromine colour was present the solution was washed with sodium hydrogen carbonate solution, brine, then dried ($MgSO_4$) and quickly reduced to an oil. The oil was dissolved in methylene chloride (25ml) was stirred at room temperature for 1h with hexamethylene tetramine (7g). The product was filtered off and washed with methylene chloride then dried and redissolved in ethanol (40ml) - conc. hydrochloric acid (20ml). After 2h the reaction was cooled in a refrigerator and the product filtered off then recrystallised from water to yield (1.7g, 21%); $\delta_H$ (DMSO-$d_6$) 4.3 (2H, m, $CH_2$), 6.25 (1H, m, 4-H), 7.63 (1H, d, 3-H), 8.08

(1H, m, 5-H), and 8.52 (3H, br, $NH_3$).

b) N-[2-Oxo-2-(2-furyl)ethyl]monamide

2-Aminoacetylfuran hydrochloride (0.808g) in water (5ml) was added to a solution of isobutoxyformic mixed anhydride of monic acid (5mmol) at 0°C in THF (25ml). The reaction was stirred at room temperature for 2h then poured into water (100ml) and extracted with ethyl acetate. The organic extracts were washed with sodium hydrogen carbonate solution, brine, then dried ($MgSO_4$) and evaporated to an oil. Chromatography on silica (10g), eluting with 0 to 8% methanol in dichloromethane gave the product as an oil (0.71g, 31%); $\nu_{max}$ ($CHCl_3$) 3420, 1680, 1663, 1637, 1572 and 1506cm$^{-1}$; $\lambda_{max}$ (EtOH) 269 ($\epsilon_m$ 16,324), and 221nm (15,239); $\delta_H$ ($CDCl_3$) 0.90 (3H, d, 17-$H_3$), 1.17 (3H, d, 14-$H_3$), 2.15 (3H, s, 15-$H_3$), 4.58 (2H, d, 1′-$H_2$), 5.78 (1H, s, 2-H), 6.51 (1H, m, 4″-H), 6.78 (1H, t, NH), 7.27 (1H, d, 3″-H), and 7.59 (1H, m, 5″-H).

c) 5-(2-Furyl)-2-(1-normon-2-yl)oxazole

Trichloroacetyl chloride (0.22ml, 4.5 eq.) was added to a solution of the monamide derivative (200mg) [prepared in (b)] in methylene chloride (20ml), pyridine (0.22ml, 6 eq.) and DMAP (few crystals) at 0°C. After 1h the solution was evaporated to dryness and the residue dissolved in methanol (30ml) and treated with potassium carbonate (0.44g, 6 eq.) and a few drops of water. The reaction was stirred at room temperature overnight then evaporated to dryness and the residue dissolved in ethyl acetate which was washed with brine. After drying ($MgSO_4$) and evaporation of the solvent the crude product was chromatographed on silica (5g) eluting with 0 to 6% methanol in dichloromethane to afford pure product (30mg, 16%); $\nu_{max}$ ($CHCl_3$) 3400, 1652 and 1540cm$^{-1}$; $\lambda_{max}$ (EtOH) 306 ($\epsilon_m$ 20,238), and 232nm (7,006); $\delta_H$ ($CDCl_3$) 0.90 (3H, d, 17-$H_3$), 1.17 (3H, d, 14-$H_3$), 2.27 (3H, s, 15-$H_3$), 6.21 (1H, m, 2-H), 6.50 (2H, m, 5′-H and 4″-H), 7.20 (1H, m, 3″-H), and 7.41 (1H, m, 5″-H).

## Example 15

### 5-(5-Bromo-2-furyl)-2-(normon-2-yl)oxazole

a) 5-Bromo-2-bromoacetylfuran

5-Bromo-2-furoic acid (10g, 52mM) and thionyl chloride (10ml, 136mM) were heated under reflux for 3h. The thionyl chloride was then evaporated under reduced pressure and the residue dried under vacuum to give the acid chloride (10.2g, 93%); $\delta_H$ ($CDCl_3$) 7.33 (1H, d, 3-H) and 6.6 (1H, d, 4-H). This acid chloride (8g) in dichloromethane (50ml) was added dropwise to diazomethane (ca.80mM) in ether (250ml) at 0°C with stirring. The mixture was stirred at 0°C for 2h. After this time conc. hydrobromic acid (50ml) was added carefully and then the mixture was allowed to warm up to room temperature over 30min. The mixture was diluted with ethyl acetate and after separating the aqueous phase, washed with brine. The ethyl acetate solution was dried ($MgSO_4$) and evaporated to give the bromoketone (9.5g, 92%); $\delta_H$ ($CDCl_3$) 7.4 (1H, d, 3-H), 6.55 (1H, d, 4-H) and 4.27 (2H, s, $CH_2$).

b) 2-Aminoacetyl-5-bromofuran hydrochloride

Hexamine (4.8g, 34mM) in dichloromethane was added to 5-bromo-2-bromoacetylfuran (9g, 33mM) in dichloromethane with stirring. After stirring at room temperature for 1.5h the resulting solid was filtered off and dried. This solid was stirred in a mixture of methanol (50ml), water (50ml) and conc. hydrochloric acid (15ml) for 24h. The mixture was then evaporated to give the amine hydrochloride as a cream-coloured solid which was recrystallised from water to yield 4.5g, 55%; $\delta_H$ [$(CD_3)_2SO$] 8.75 (brs, NH), 7.93 (1H, d, 3-H), 7.12 (1H, d, 4-H) and 4.4 (2H, s, $CH_2$).

c) 5-(5-Bromo-2-furyl)-2-(normon-2-yl)oxazole

Monic acid (2.85g, 8.3mM) was converted to the isobutyloxy mixed anhydride and reacted with 2-aminoacetyl-5-bromofuran hydrochloride (2g, 8.3mM) to give the ketoamide (1.55g, 35%) after purification by chromatography on silica (methanol in dichloromethane gradient); $\delta_H$ ($CDCl_3$) 7.27 (1H, d, 4′-H), 6.93 (1H, t, NH), 6.53 (1H, d, 3-H), 5.83 (1H, s, 2-H), 4.6 (2H, d, $CH_2CO$), 2.15 (3H, s, 15-$H_3$), 1.2 (3H, d, 14-$H_3$) and 0.9 (3H, d, 17-$H_3$).

This ketoamide (1.4g) was cyclised to give the oxazole (550mg, 40.6%) after column chromatography on silica (methanol in dichloromethane gradient); $\nu_{max}$ (film) 3390 and 1660cm$^{-1}$; $\lambda_{max}$ (EtOH) 312nm ($\epsilon_m$ 18,465) ; $\delta_H$ ($CDCl_3$) 7.25 (1H, s, 4′-H), 6.53 (1H, d, 3″-H), 6.40 (1H, d, 4″-H), 6.25 (1H, s, 2-H), 2.27 (3H, s, 15-$H_3$), 1.24 (3H, d, 14-$H_3$) and 0.93 (3H, d, 15-$H_3$); m/z (rel. int.) 511 ($M^+$, 10%), 366 (4), 296 (8) and 269 (87) (Found: 511.1197. $C_{23}H_{30}NO_7Br$ requires 511.1206).

Example 16

5-(3-Furyl)-2-(1-normon-2-yl)oxazole

a) 3-Bromoacetylfuran

3-Furoyl chloride (5g, 38mM) in dichloromethane (40ml) was added slowly to diazomethane (ca.80mmol) in ether (250ml) at 0°C with stirring. The mixture was stirred at this temperature for 1.5h. After this time conc. hydrobromic acid (50ml) was added carefully at 0°C and then the mixture was allowed to warm up to room temperature over 0.5h. The mixture was diluted with water and ethyl acetate. The two layers were separated and the ethyl acetate solution was washed with aqueous sodium hydrogen carbonate, water, and dried ($MgSO_4$). The ethyl acetate was evaporated to give the bromide (6.25g, 86%); $\nu_{max}$ - (film) 1680, 1560 and 1410cm$^{-1}$; $\delta_H$ ($CDCl_3$) 8.2 (1H, d, 2-H), 17.48 (1H, t, 4-H), 6.8 (1H, d, 5-H) and 4.23 (2H, s, $CH_2$).

b) 3-Aminoacetylfuran hydrochloride

3-Bromoacetylfuran (5.5g) was dissolved in acetone (45ml) and water (15ml) and the treated with sodium azide (2.08g). The mixture was stirred for 1.5h and then diluted with water and extracted with ether. The ether extracts were washed with brine, dried ($MgSO_4$) and evaporated. The residue was taken up in ethanol (200ml) and 1M-hydrochloric acid (50ml), and then hydrogenated over 10% palladium on charcoal at atmospheric pressure. The mixture was then filtered and evaporated to give solid which was triturated with acetone to give a white solid (2.48g, 53%); $\delta_H$ [$(CD_3)_2SO$] 8.87 (1H, d, 2-H), 7.9 (1H, t, 4-H), 6.92 (1H, d, 5-H) and 4.33 (2H, s, $CH_2$).

c) N-[2-(3-Furyl)-2-oxoethyl]monamide

Monic acid (3.2g, 9.3mM) was converted to its isobutyloxy mixed anhydride which was then reacted with 3-aminoacetylfuran hydrochloride (1.5g, 9.3mM) to give the monamide (2.2g, 52%) after chromatography on silica (methanol in dichloromethane eluent); $\nu_{max}$ (film) 3390, 1690, 1660 and 1630cm$^{-1}$; $\lambda_{max}$ (EtOH) 218nm, ($\epsilon_m$ 21,988); $\delta_H$ ($CDCl_3$) 8.17 (1H, d, 2″-H), 7.49 (1H, t, 4″-H), 6.81 (1H, d, 5″-H), 6.54 (1H, t, NH), 5.80 (1H, s, 2-H), 4.57 (2H, d, $HNCH_2CO$), 2.21 (3H, s, 15-$H_3$), 1.23 (3H, d, 14-$H_3$) and 0.94 (3H, d, 17-$H_3$); $\delta_C$ ($CDCl_3$) 189.9 (C-2′), 167.3 (C-1), 152.3 (C-3), 147.9 (C-2″), 144.4 (C-5″), 124.9 (C-3″), 119.3 (C-2), 108.1 (C-4″), 74.7 (C-5), 70.7 (C-13), 70.2 (C-7), 68.7 (C-6), 65.1 (C-16), 60.8 (C-11), 55.5 (C-10), 46.8 (C-1′), 42.5 (C-4 and C-12), 39.5 (C-8), 31.5 (C-9), 20.5 (C-14), 18.7 (C-15) and 12.4 (C-17); m/z 451 ($M^+$, 2%), 349 (4), 306 (14), 227 (28) and 207 (86) (Found: 451.2201. $C_{23}H_{33}NO_8$ requires 451.2206).

d) 5-(Furyl)-2-(1-normon-2-yl)oxazole

N-[2-(3-Furyl)-2-oxoethyl]monamide (1.8g) was cyclised to give the required oxazole (0.625g, 36%) after chromatography on silica (methanol in dichloromethane eluent); $\nu_{max}$ (film) 3400 (br), 1705, 1500, 1380 and 1335cm$^{-1}$; $\lambda_{max}$ (EtOH) 290nm ($\epsilon_m$ 14,663); $\delta_H$ ($CDCl_3$) 7.74 (1H, s, 2″-H), 7.47 (1H, t, 4″-H), 7.11 (1H, s, 4′-H), 6.61 (1H, d, 5″-H), 6.24 (1H, s, 2-H), 2.29 (3H, s, 15-$H_3$), 1.23 (3H, d, 14-$H_3$) and 0.93 (3H, d, 1-$H_3$); $\delta_C$ ($CDCl_3$) 160.4 (C-1), 146.7 (C-3), 143.8 (C-2″), 143.6 (C-5′), 138.7 (C-5″), 122.0 (C-4′), 114.4 (C-3″), 112.6 (C-2), 107.4 (C-4″), 75.1 (C-5), 70.9 (C-13), 70.2 (C-7), 68.6 (C-6), 65.3 (C-16), 61.0 (C-11), 55.4 (C-10), 42.5 (C-4 and C-12), 39.3 (C-8), 31.5 (C-9), 20.6 (C-14), 19.4 (C-15) and 12.5 (C-17); m/z 433 ($M^+$, 14%), 361 (3), 218 (15) and 189 (100) (Found: 433.2100. $C_{23}H_{31}NO_7$ requires 433.2100).

Example 17

5-(5-Methyl-2-furyl)-2-(1-normon-2-yl)oxazole

a) 2-Aminoacetyl-5-methylfuran hydrochloride

Bromine (2.1ml) was added to a solution of 2-acetyl-5-methylfuran (6.2g) in ether (100ml). When no bromine colour remained the solution was washed with sodium hydrogen carbonate solution, brine, then dried ($MgSO_4$) and quickly reduced to an oil. The oil was dissolved in methylene chloride (25ml) and stirred at room temperature for 3h with hexamethylene tetramine (7g). The product was filtered off, washed with methylene chloride then dried and redissolved in methanol (40ml) - concentrated hydrochloric acid (20ml). After 1.5h the reaction was cooled in an ice-bath and the product filtered off then recrystallised from water to yield the title compound (1.45g, 16%).

b) N-[2-Oxo-2-(5-methyl-2-furyl)]ethyl monamide

2-Aminoacetyl-5-methylfuran hydrochloride (0.7g) was added to a solution of isobutoxy formic mixed anhydride of monic acid (4mmol) at 5°C in THF (40ml) containing triethylamine (0.56ml). Reaction was allowed to proceed according to the general method and the monamide isolated (1.3g, 63%); $\nu_{max}$ - ($CHCl_3$) 3400, 1660, 1630 and 1500cm$^{-1}$; $\lambda_{max}$ (EtOH) 282 ($\epsilon_m$ 16,900) and 219nm (16,400); $\delta_H$ ($CDCl_3$)

0.94 (3H, d, J 7Hz, 17-$H_3$), 1.24 (3H, d, J 6.5Hz, 14-$H_3$), 2.22 (3H, s, 15-$H_3$), 2.43 (3H, s, 5″-Me), 4.61 (2H, d, J 5Hz, collapses to a singlet in $CD_3OD$, 1′-$H_2$), 5.81 (1H, s, 2-H), 6.20 (1H, d, J 4Hz, 3″-H), 6.62-6.70 (1H, t, collapses to a singlet in $CD_3OD$) -NH-), 7.25 (1H, d, J 4Hz, 4″-H); $\delta_C$ ($CDCl_3$) 12.7 (C-17), 14.1 (furan -$CH_3$), 18.9 (C-15), 20.8 (C-14), 31.7 (C-9), 39.5 (C-8), 42.6 (C-4), 42.7 (C-12), 45.3 (C-1′), 55.6 (C-10), 61.1 (C-11), 65.4 (C-16), 68.8 (C-6), 70.4 (C-7), 71.1 (C-13), 74.9 (C-5), 109.5 (C-4″), 119.6 (C-3″), 120.4 (C-2), 149.5 (C-2″), 152.2 (C-3), 158.8 (C-5″), 167.4 (C-1), 183.2 (C-2′); m/z 465 ($M^+$, 20%) and 221 (100%).

c) 5-(5-Methyl-2-furyl)-2-(1-normon-2-yl)oxazole

The monamide (from (a) (0.63g) was cyclised according to the general procedure to give the title oxazole (0.43g, 71%); $\nu_{max}$ ($CHCl_3$) 3400, 1650, 1580cm$^{-1}$; $\lambda_{max}$ (EtOH) 313nm ($\epsilon_m$ 21,600); $\delta_H$ ($CD_3OD$) 0.95 (3H, d, J 7Hz, 17-$H_3$), 1.20 (3H, d, J 6.5Hz, 14-$H_3$), 2.28 (3H, s, 15-$H_3$), 2.35 (3H, s, 5″-Me), 6.15 (1H, singlet with fine coupling, 3″-H), 6.20 (1H, broadened singlet, 2-H), 6.57 (1H, d, J 3Hz, 4″-H) 7.20 (1H, s, 4′-H); $\delta_C$ ($CD_3OD$) 12.3 (C-17), 13.4 (5″-$CH_3$), 19.7 (C-15), 20.3 (C-14), 33.0 (C-9), 41.6 (C-8), 43.7 (C-12), 43.9 (C-4), 56.9 (C-10), 61.2 (C-11), 66.4 (C-16), 70.0 (C-6), 70.7 (C-7), 21.6 (C-13), 76.4 (C-5), 108.7 (C-4″), 109. 4 (C-3″), 122.0 (C-4′), 143.1 (C-2″), 144.1 (C-3), 149.2 (C-2′), 154.4 (C-5″), 161.8 (C-5′).

Example 18

5-(5-Methoxycarbonyl-2-furyl)-2-(1-normon-2-yl)oxazole

a) Methyl 2-acetylfuran-5-carboxylate

Stannic chloride (4.6ml, 39mmol) and acetic anhydride (15ml, 160mmol) at 5°C under argon were treated dropwise with a solution of methyl furoate (4.2ml, 39mmol) in dry benzene (20ml). On completion of the addition the cooling bath was removed and the mixture left 3 days at room temperature. The reaction was then neutralised carefully with 30% potassium carbonate solution and extracted with ethyl acetate. The organic extracts were washed with brine ($MgSO_4$) and evaporated. Silica chromatography gave a mixture of methyl 2-acetylfuran-5-carboxylate contaminated with methyl 2-acetoacetylfuran-5-carboxylate. This impurity was removed as the sodium chelate by repeated extraction with cold saturated sodium carbonate solution to give the title compound as a buff coloured solid in ca.15% yield; $\nu_{max}$ - ($CHCl_3$) 1715 and 1690cm$^{-1}$; $\delta_H$ ($CDCl_3$) 2.57 (3H, s, $CH_3CO$), 3.97 (3H, s, -$CO_2CH_3$), 7.28 (2H, s, aromatics).

b) Methyl 2-bromoacetylfuran-5-carboxylate

A solution of methyl 2-acetylfuran-5-carboxylate (1.4g, 8.3mmol) in dichloromethane (25ml) under argon was treated sequentially with triethylamine (1.74ml, 12.5mmol), trimethylsilyl triflate (2.35ml, 12.2mmol), allowed to react 0.5h at room temperature and then treated with N-bromosuccinimide (1.62g, 9.1mmol). After a further 0.75h at room temperature the reaction mixture was washed with water, with saturated brine, dried ($MgSO_4$), evaporated and the crude product subjected to silica chromatography eluting with dichloromethane to give the title compound (1.7g, 83%), m.p. 87-88°C; $\nu_{max}$ ($CHCl_3$) 1730 and 1685cm$^{-1}$; $\delta_H$ ($CDCl_3$) 3.96 (3H, s, -$CO_2CH_3$); 4.43 (2H, s, $BrCH_2CO$-), 7.20-7.55 (2H, m, aromatics); m/z 246 and 248 ($M^+$, 3% each) and 153 (100%).

c) Methyl 2-azidoacetylfuran-5-carboxylate

A solution of the above $\alpha$-bromoketone (1.5g, 6mmol) in acetone (12ml) and water (3.5ml) at 5°C was treated with sodium azide (0.39g, 6mmol) and allowed to react 0.5h at 5°C followed by 1h at room temperature. The reaction mixture was then diluted with ether, washed with brine, dried ($MgSO_4$), evaporated and subjected to silica chromatography eluting with dichloromethane resulting in the isolation of the title product (1.2g, 96%), m.p. 80-82°C; $\nu_{max}$ ($CHCl_3$) 2110, 1730 and 1700cm$^{-1}$; $\delta_H$ ($CDCl_3$) 3.96 (3H, s, -$CO_2CH_3$), 4.54 (2H, s, $N_3CH_2CO$-), 7.25-7.50 (2H, m, aromatics); m/z 209 ($M^+$, 2%) and 153 (100%).

d) 2-Aminoacetyl-5-methoxycarbonylfuran hydrochloride

The above $\alpha$-azidoketone (1.1g, 5.2mM) was subjected to hydrogenation in methanol (60ml) in the presence of 2N hydrochloric acid (30ml) using 5% palladium on charcoal (0.11g) as catalyst. After 15 minutes t.l.c. indicated reaction to be complete and work-up gave the title salt (1g, 87%); $\nu_{max}$ (KBr) 3700-2200 (b), 1728, 1683cm$^{-1}$; $\delta_H$ ($D_2O$) 3.93 (3H, s, -$CH_3$), 4.55 (2H, s, -$CH_2$), 7.39-7.43 (1H, d, J 4Hz, 4-H), 7.52-7.56 (2H, d, J 4Hz, 3-H).

e) N-[2-Oxo-2-(5-methoxycarbonyl-2-furyl)ethyl]monamide

The above amine hydrochloride (1.0g, 4.5mM) was reacted with the isobutoxyformic mixed anhydride of monic acid according to the established procedure and the title monamide isolated (1.3g, 57%); $\nu_{max}$ - ($CHCl_3$) 3,400 (br), 1720, 1690, 1660, 1630cm$^{-1}$; $\lambda_{max}$ (EtOH) 273 ($\epsilon_m$ 22,000) and 281 (sh.) nm (19,200);

$\delta_H$ ($CD_3OD$) 0.9-1.02 (3H, d, J 7Hz, 17-$H_3$), 1.16-1.26 (3H, d, J 6Hz, 14-$H_3$), 2.18 (3H, s, 15-$H_3$), 3.92 (3H, s, -$CO_2CH_3$), 4.60 (2H, s, 2′-$H_2$), 5.88 (1H, s, 2-H), 7.30-7.37 (1H, d, J 4Hz, 3″-H), 7.40-7.47 (1H, d, J 4Hz, 4″-H); $\delta_C$ $CD_3OD$) 12.3 (C-17), 19.0 (C-15), 20.3 (C-14), 33.0 (C-9), 41.7 (C-8), 43.7 (C-12 and C-14), 46.7 (C-1′), 52.9 (-$CO_2CH_3$), 56.7 (C-10), 61.2 (C-11), 66.3 (C-16), 70.0 (C-6), 70.7 (C-7), 71.6 (C-13), 76.2 (C-5), 118.9 (C-4″), 119.8 (C-3″), 120.5 (C-2), 147.9 (C-2″), 153.5 (C-3), 153.9 ($CO_2CH_3$), 159.8 (C-5″), 169.8 (C-1), 186.2 (C-2′), m/z 509 ($M^+$, 4%) and 82 (100%).

f) 5-(5-Methoxycarbonyl-2-furyl)-2-(1-normon-2-yl)oxazole

The above monamide (184mg, 0.36mM) was cyclised using established methodology to give the title oxazole (120mg, 68%); $\nu_{max}$ ($CHCl_3$) 3400, 1720, 1655$cm^{-1}$; $\lambda_{max}$ (EtOH) 326 ($\epsilon_m$ 26,900) and 243nm (5,800); $\delta_H$ ($CD_3OD$) 0.92-0.98 (3H, d, J 7Hz, 17-$H_3$), 1.17-1.23 (3H, d, J 6.5Hz, 14-$H_3$), 2.30 (3H, s, 15-$H_3$), 3.91 (3H, s, -$CO_2CH_3$), 6.27 (1H, s, 2-H), 6.83-6.86 (1H, d, J 3.5Hz, 3″-H), 7.31-7.35 (1H, d, J 3.5Hz, 4″-H), 7.54 (1H, s, 4′-H); $\delta_C$ ($CD_3OD$) 12.3 (C-17), 19.9 (C-15), 20.3 (C-14), 33.0 (C-9), 41.7 (C-8), 43.7 (C-12), 44.0 (C-4), 52.6 (-$CO_2CH_3$), 56.9 (C-10), 61.3 (C-11), 66.4 (C-16), 70.0 (C-6), 70.7 (C-7), 71.6 (C-13), 76.4 (C-5), 109.6 (C-4″), 113.1 (C-2), 120.8 (C-4′), 126.0 (C-3″), 142.5 (C-2″), 145.3 (C-3), 148.0 (C-2′), 151.2 (C-5″), 160.2 (-$CO_2CH_3$), 163.0 (C-5′); m/z 491 ($M^+$, 12%) and 247 (100%).

## Example 19

### 5-(5-Hydroxymethyl-2-furyl)-2-(1-normon-2-yl)oxazole

A suspension of the methoxycarbonylfuryl derivative (Example 18) (0.05g, 0.1mmol) in THF (2ml) at 5°C under an atmosphere of argon was treated sequentially with dimethylaminopyridine (catalytic amount), triethylamine (0.045ml, 0.33mmol), and trimethylsilyl chloride (0.038ml, 0.33mmol) and reaction stirred at 5°C for 2.5h, filtered and evaporated. The product was taken up in dry ether (5ml) refiltered and the solvent removed leaving a yellow oil. This oil was dissolved in dichloromethane (2ml), chilled to 5°C under argon and treated with diisobutylaluminium hydride (1.5M in toluene, 0.11ml). After an overnight reaction a further quantity of the hydride (0.10ml) was added in two portions over 1h. Methanol:dichloromethane (1:1, 1ml) was then added to decompose residual hydride and the solvents evaporated. Deprotection was effected by dissolving the residue in THF:water (4:1, 5ml) and treating with two drops 5M hydrochloric acid. After 5 minutes at room temperature sodium hydrogen carbonate solution was added to pH 8, the reaction partitioned between ethyl acetate and brine, the phases separated, and the organic phase dried ($MgSO_4$), filtered and evaporated. Chromatography (5-10% methanol-dichloromethane) gave the title oxazole as an off-white foam (0.025g, 54%); $\nu_{max}$ ($CHCl_3$) 3400 and 1655$cm^{-1}$; $\lambda_{max}$ (EtOH) 234 ($\epsilon_m$ 6,530) and 310nm (20,665); $\delta_H$ ($CD_3OD$) 0.95 (3H, d, J 7.0Hz, 17-$H_3$), 1.20 (3H, d, J 6.5Hz, 14-$H_3$), 2.28 (3H, s, 15-$H_3$), 4.56 (2H, s, -$CH_2OH$), 6.22 (1H, s, 2-H), 6.44 (1H, d, J 3.5Hz, 3″-H), 6.65 (1H, d, J 3.5Hz, 4″-H), 7.29 (1H, s, 4′-H); $\delta_C$ ($CD_3OD$) 12.3 (C-17), 19.8 (C-15), 20.3 (C-14), 33.0 (C-9), 41.7 (C-8), 43.7 (C-12), 43.9 (C-4), 56.9 (C-10), 57.3 (-$CH_2OH$), 61.2 (C-11), 66.4 (C-16), 70.0 (C-6), 70.7 (C-7), 71.6 (C-13), 76.4 (C-5), 109.1 (C-4″), 110.4 (C-3″), 113.3 (C-2), 122.9 (C-4′), 143.8 (C-2″), 144.4 (C-3), 149.7 (C-2″), 156.9 (C-5″), 162.2 (C-5′); m/z 463 ($M^+$, 24%) and 219 (100%).

## Example 20

### Sodium 5-(5-carboxylato-2-furyl)-2-(1-normon-2-yl)oxazole

The furanyloxazole (Example 18) (0.10g, 0.2mmol) was dissolved in trimethylorthoformate (1ml) and treated with a crystal of p-toluene-sulphonic acid at room temperature. After 5 minutes the reaction was diluted with ethyl acetate, washed with sodium hydrogen carbonate solution, brine, dried ($MgSO_4$) and evaporated leaving a yellow gum. This gum was dissolved in THF (1.8ml) and 1M sodium hydroxide solution (1.8ml) added. After refluxing for 2 hours, the mixture was cooled, adjusted to pH 2 and stirred for 0.5 hours. Brine was then added and the solution extracted with ethyl acetate. The extracts were washed with brine, dried ($MgSO_4$) and evaporated to give a cream coloured foam (0.11g). This product was dissolved in methanol (1.8ml) and potassium carbonate (0.153g) added at 5°C. The mixture was stirred for 0.5 hours, diluted with water adjusted to pH 2 and extracted with ethyl acetate. The extracts were washed with brine, dried ($MgSO_4$) and evaporated to give the free acid (0.07g). This product was treated with dilute sodium hydroxide solution to pH 7 and the resulting solution purified by HP20SS chromatography, eluting with 1-10% THF in water, to give the title compound as a white freeze-dried solid (0.045g, 45%); $\nu_{max}$ (KBr) 3400 (broad), 1650 (sh.), 1600 and 1555$cm^{-1}$; $\lambda_{max}$ ($H_2O$) 320 ($\epsilon_m$ 25,600), 238 (6,900) and 201nm (1700); $\delta_H$ ($D_2O$) 0.90 (3H, d, J 7.0Hz, 17-$H_3$), 1.15 (3H, d, J 6.5Hz, 14-$H_3$), 2.13 (3H, s, 15-$H_3$), 6.09 (1H, s, 2-H),

6.69 (1H, d, J 3.5Hz, 3″-H), 7.00 (1H, d, J 3.5Hz, 4″-H) and 7.35 (1H, s, 4′-H); $\delta_C$ ($D_2O$) 24.7 (C-17), 32.0 (C-15), 32.4 (C-14), 44.7 (C-9), 52.4 (C-8), 55.3 (C-4), 55,6 (C-12), 70.4 (C-10), 75.1 (C-11), 78.6 (C-16), 82.2 (C-6), 83.2 (C-13), 83.3 (C-7), 88.4 (C-5), 122.5 (C-4″), 125.7 (C-3″), 130.0 (C-2), 136.4 (C-4′), 155.5 (C-3), 157.7 (C-5″), 162.1 (C-2′), 162.7 (C-2″), 174.5 (C-5′), 179.4 ($-CO_2Na$); m/z (FAB thioglycerol) 500 ($MH^+$, 100%) and 522 ($MNa^+$, 20%).

Example 21

5-(5-Carboxyamido-2-furyl)-2-(1-normon-2-yl)oxazole

The furanyloxazole (Example 20) was converted to the free acid (0.11g), suspended in dichloromethane (15ml) and triethylamine (0.035ml). Cooled to 5°C and treated with isobutyl chloroformate (0.033ml). After 1.3 hours at 5°C ammonia gas was bubbled through the reaction mixture for 2 minutes then stirred for 15 minutes before diluting with ethyl acetate. This organic phase was washed with water, brine, dried ($MgSO_4$) and evaporated to give a crude product. Column chromatography eluting with 5-15% methanol in dichloromethane gave the title oxazole (0.05g, 45%); $\lambda_{max}$ (EtOH) 239 ($\epsilon_m$ 5,390) and 325nm (25,100); $\delta_H$ - ($CD_3OD$) 0.95 (3H, d, J 7.0Hz, 17-$H_3$), 1.20 (3H, d, J 6.5Hz, 14-$H_3$), 2.30 (3H, s, 15-$H_3$), 6.26 (1H, s, 2-H), 6.82 (1H, d, J 3.5Hz, 3″-H), 7.24 (1H, d, J 3.5Hz, 4′-H) and 7.40 (1H, s, 4′-H); $\delta_C$ ($CD_3OD$) 12.3 (C-17), 19.9 (C-15), 20.4 (C-14), 33.0 (C-9), 41.7 (C-8), 43.8 (C-12), 44.0 (C-4), 56.9 (C-10), 61.3 (C-11), 66.4 (C-16), 70.1 (C-6), 70.7 (C-7), 71.7 (C-13), 76.4 (C-5), 109.8 (C-4″), 113.1 (C-3″), 117.6 (C-2), 125.6 (C-4′), 142.7 (C-3), 146.7 (C-5″), 148.5 (C-2′), 150.9 (C-2″), 162.3 ($-CONH_2$) and 163.1 (C-5′); m/z 476 ($M^+$, 5%) and 43 (100%) (Found: $M^+$, 476.2166. $C_{24}H_{32}N_2O_8$ requires M, 476.2159).

Example 22

5-(5-Methoxyformimidoyl-2-furyl)-2-(1-normon-2-yl)oxazole

The furanyloxazole (Example 21) (0.045g, 0.094mmol) in dry THF (1.4ml) was treated with triethylamine (0.071ml, 0.52mmol) and trichloroacetyl chloride (0.047ml, 0.41mmol) at 5°C under an atmosphere of argon. After 0.75 hours the reaction mixture was partitioned between ethyl acetate and 1M hydrochloric acid, the phases separated and the organic phase washed with brine (x2), dried ($MgSO_4$) and evaporated. The residue was then dissolve in methanol (2ml), chilled to 5°C under argon and treated with potassium carbonate (0.064g, 0.47mmol). The mixture was stirred for 0.75 hours then partitioned between ethyl acetate and brine, the organic phase dried ($MgSO_4$), evaporated and the crude product subjected to silica chromatography (eluting with 10% methanol in dichloromethane) to give the title oxazole as an off-white foam (0.043g, 75%); $\nu_{max}$ ($CHCl_3$) 3350 and 1650cm$^{-1}$; $\lambda_{max}$ (EtOH) 242 ($\epsilon_m$ 5,300) and 327nm (23,00); $\delta_H$ - ($CD_3OD$) 0.93 (3H, d, J 7.0Hz, 17-$H_3$), 1.20 (3H, d, J 6.5Hz, 14-$H_3$), 2.30 (3H, s, 15-$H_3$), 3.89 (3H, s, $OCH_3$), 6.25 (1H, s, 2-H), 6.80 (1H, d, J 3.5Hz, 3″-H), 7.00 (1H, d, J 3.5Hz, 4″-H), and 7.54 (1H, s, 4′-H); $\delta_C$ ($CDCl_3$) 12.8 (C-17), 19.7 (C-15), 21.0 (C-14), 31.6 (C-9), 39.3 (C-8), 42.7 (C-4), 42.8 (C-12), 53.2 ($-OCH_3$), 55.7 (C-10), 61.6 (C-11), 656.4 (C-16), 68.9 (C-6), 70.6 (C-7), 71.4 (C-13), 75.3 (C-5), 108.6 (C-3″), 112.6 (C-2), 113.1 (C-4″), 124.3 (C-4′), 141.3 (C-3), 144.9 (C-5″), 145.6 (C-2′), 148.4 (C-2″), 160.0 (>C=NH), 161.5 (C-5′); m/z - (FAB thioglycerol) 491 ($MH^+$, 100%) and 513 ($MNa^+$, 7%).

Example 23

5-(2,5-Dimethyl-3-furyl)-2-(1-normon-2-yl)oxazole

a) 3-Bromoacetyl-2,5-dimethylfuran

3-Acetyl-2,5-dimethylfuran (1ml, 7.5mmol) was dissolved in dichloromethane (25ml) and treated sequentially with trimethylamine (1.62ml, 11.25mmol) and trimethylsilyltrifluoromethylsulphonate (2.25ml, 11.25mmol) at 5°C under an atmosphere of argon. After 0.5 hours the cooling bath was removed and the reaction treated with solid N-bromosuccinimide (1.54g, 8.6mmol). After a further 0.75 hours the reaction was washed with water, dried ($MgSO_4$), evaporated and subjected to silica chromatography to give a pale yellow solid (0.58g, 36%); $\nu_{max}$ ($CHCl_3$) 1670 and 1575cm$^{-1}$; $\delta_H$ ($CDCl_3$) 2.25 (3H, s), 2.50 (3H, s), 41.3 (2H, s) and 6.28 (1H, s).

b) 3-Azidoacetyl-2,5-dimethylfuran

The above $\alpha$-bromoketone (0.566g, 2.6mmol) was treated with sodium azide (0.17g, 2.6mmol) in aqueous acetone (1.5:5ml) at 5°C over 0.5 hours and the product isolated, after chromatography, as a cream

coloured solid (0.43g, 92%), m.p. 46-48°C; $\nu_{max}$ (CHCl$_3$) 2250, 1680 and 1570cm$^{-1}$; $\delta_H$ (CDCl$_3$) 2.28 (3H, s), 2.58 (3H, s), 4.24 (2H, s) and 6.22 (1H, s); m/z 179 (M$^+$, 13%) and 123 (100%).

c) 3-Aminoacetyl-2,5-dimethylfuran hydrochloride

3-Azidoacetyl-2,5-dimethylfuran (0.43g, 2.4mmol) was dissolved in methanol (30ml) and 2M hydrochloric acid (15ml) added. This mixture was hydrogenated in the presence of 5% palladium on charcoal (50mg) at room temperature and pressure for 12 minutes. The catalyst was then removed and the filtrate evaporated to give the title product as a buff coloured amorphous solid (0.36g, 79%); $\nu_{max}$ (KBr) 1677, 1572 and 1498cm$^{-1}$; $\delta_H$ (D$_2$O) 2.21 (3H, s), 2.49 (3H, s), 4.28 (2H, s) and 6.32 (1H, s); m/z 153 (M$^+$, 31%) and 123 (100) (Found: 153.0795. $C_8H_{11}NO_2$ requires 153.0790).

d) N-[2-Oxo-2-(2,5-dimethyl-3-furyl)ethyl]monamide

3-Aminoacetyl-2,5-dimethylfuran (0.19g, 1mmol) was reacted with the isobutoxyformic anhydride of monic acid in the usual way and the title monamide isolated as a colourless gum (0.23g, 47%); $\nu_{max}$ (EtOH) 216 ($\epsilon_m$ 20,230) and 270nm (4,380); $\delta_H$ (CD$_3$OD) 0.95 (3H, d, J 7.0Hz, 17-H$_3$), 1.20 (3H, d, J 6.5Hz, 14-H$_3$), 2.17 (3H, s, 15-H$_3$), 2.27 (3H, s, 5″-CH$_3$), 2.53 (3H, s, 2″-CH$_3$), 4.39 (2H, s, 1′-H), 5.88 (1H, s, 2-H) and 6.40 (1H, s, 4″-H); $\delta_C$ (CD$_3$OD) 10.5 (C-17), 11.2 (C-2″ methyl), 12.5 (C-5″methyl), 17.2 (C-15), 18.5 (C-14), 31.2 (C-9), 39.7 (C-8), 41.9 (C-12 and C-4), 47.2 (C-1′), 55.1 (C-10), 59.4 (C-11), 64.5 (C-16), 66.2 (C-6), 66.9 (C-7), 69.6 (C-13), 74.4 (C-5), 104.1 (C-4″), 119.0 (C-2), 119.1 (C-3″), 150.2 (C-3), 151.3 (C-5″), 157.1 (C-2″), 167.9 (C-1) and 190.7 (C-2′); m/z 479 (M$^+$, 32%) and 123 (100) (Found: 479.2506. $C_{25}H_{37}NO_8$ requires 479.2519).

e) 5-(2,5-Dimethyl-3-furyl)-2-(1-normon-2-yl)oxazole

The above monamide (0.18g, 0.3mmol) was cyclised using the standard procedure and the oxazole isolated as a foam (0.1g, 65%); $\nu_{max}$ (CHCl$_3$) 3400, 1660 and 1600cm$^{-1}$; $\lambda_{max}$ (EtOH) 298nm ($\epsilon_m$ 16,000); $\delta_H$ (CD$_3$OD) 0.95 (3H, d, J 7.0Hz, 17-H$_3$), 1.18 (3H, d, J 6.5Hz, 14-H$_3$), 2.25 (6H, s, 15-H$_3$ and 5″-CH$_3$), 2.46 (3H, s, 2″-CH$_3$), 6.19 (2H, s, 2-H and 4″-H) and 7.08 (1H, s, 4′-H); $\delta_C$ (CD$_3$OD) 12.3 (C-17), 13.2 (C-2″ methyl), 13.3 (C-5″ methyl), 33.0 (C-9), 41.7 (C-8), 43.8 (C-12), 43.9 (C-4), 56.9 (C-10), 61.3 (C-11), 66.4 (C-16), 70.0 (C-6), 70.7 (C-7), 71.7 (C-13), 76.5 (C-5), 105.5 (C-4″), 111.0 (C-3″), 113.7 (C-2), 122.4 (C-4′), 146.9 (C-3), 148.0 (C-2′), 148.2 (C-2″), 152.2 (C-5″) and 161.5 (C-5′); m/z 461 (M$^+$, 11%) and 217 (100).

## Example 24

### 5-(4-Cyano-2-furyl)-2-(1-normon-2-yl)oxazole

a) Ethyl 2-acetyl-4-furoate

Ethyl 3-furoate (12.4g, 88mmol) was reacted with acetic anhydride in the presence of boron trifluoride etherate according to the method of C Eugster et al, Helv. Chim. Acta., 40, 888 (1957). Silica chromatography (eluting with 2:1 hexane:ethyl acetate) gave unreacted starting material (3g, 25%) and the required furan derivative as a waxlike solid (8g, 50%); $\nu_{max}$ (CHCl$_3$) 1720, 1680 and 1580cm$^{-1}$; $\delta_H$ (CDCl$_3$) 1.38 (3H, t, J 7.0Hz), 2.50 (3H, s), 4.15-4.38 (2H, q, J 7.0Hz), 7.54 (1H, s) and 8.20 (1H, s); m/z 182 (M$^+$, 67%) and 137 (100%) (Found: M$^+$, 182.0583. $C_9H_{10}O_4$ requires M, 182.0579).

b) 2-Acetyl-4-furoic acid

Ethyl 2-acetyl-4-furoate (5g, 27.4mmol) was dissolved in ethanol (34ml) and a solution of potassium hydroxide (2.36g, 41.1mmol) in water (16ml) added. The resulting densely coloured solution was warmed for 15 minutes at 60°C then cooled, acidified and extracted with ethyl acetate (x4). The combined extracts were washed with a small volume of brine, dried (MgSO$_4$) and evaporated to give a brown amorphous solid (3.0g, 70%); $\delta_H$ [(CD$_3$)$_2$SO)] 2.48 (3H, s), 7.06 (1H, broad), 7.55 (1H, s) and 8.27 (1H, s).

c) 2-Acetyl-4-Carbamoylfuran

2-Acetyl-4-furoic acid (3.7g, 24mmol) was suspended in dichloromethane (80ml) and treated with oxalyl chloride (2.1ml, 24mmol) followed by dimethylformamide (catalytic amount). The reaction was stirred at room temperature for 0.5 hours. Argon was then bubbled through the reaction for 1 hour before evaporating to give a brown, waxlike solid; $\nu_{max}$ (CHCl$_3$) 1760 and 1680cm$^{-1}$. This product was dissolved in dry THF (150ml) cooled to 5°C and reacted with ammonia gas over 0.5 hours. Argon was then bubbled through the reaction for 0.5 hours prior to partitioning between ethyl acetate and water. The organic phase was then washed with brine, dried (MgSO$_4$) and evaporated to give the title compound as a brown powder (2.69g, 73%); $\nu_{max}$ (KBr) 1670, 1620 and 1580cm$^{-1}$; M$^+$, 153 (100%).

d) 2-Acetyl-4-cyanofuran

2-Acetyl-4-carbamoylfuran (1.5g, 9.8mmol) was suspended in dichloromethane (50ml), chilled to 5°C and treated with triethylamine (2.73ml, 19.6mmol) and trichloroacetyl chloride (1.21ml, 10.8mmol). The mixture was stirred for 0.5 hours, diluted with dichloromethane and washed sequentially with 1M sodium

hydroxide, water, 1M hydrochloric acid, brine then dried ($MgSO_4$), evaporated and subjected to silica chromatography (gradient elution 30%-100% ethyl acetate-hexane). The title furan was isolated as a cream coloured solid, m.p. 92°-93°C (0.9g, 68%); $\nu_{max}$ ($CHCl_3$) 2240 and 1685cm$^{-1}$; $\delta_H$ ($CDCl_3$) 2.52 (3H, s), 7.42 (1H, s) and 8.20 (1H, s); m/z 135 ($M^+$, 135.0323. $C_7H_5NO_2$ requires M, 135.0320).

e) 2-Bromoacetyl-4-cyanofuran

2-Acetyl-4-cyanofuran (0.7g, 5.2mmol) in dichloromethane (20ml) at 5°C was treated with triethylamine (1.09ml, 7.8mmol) followed by trimethylsilyltrifluoromethylsulphonate (1.5ml, 7.8mmol). The cooling bath was then removed and the reaction left for 0.5 hours. N-Bromosuccinimide (1.06g, 5.9mmol) was then added and the reaction continued. After 0.75 hours the reaction was washed with water, dried ($MgSO_4$), evaporated and chromatographed to give the title compound (0.25g, 23%); $\nu_{max}$ ($CHCl_3$) 2250, 1685 and 1575cm$^{-1}$; $\delta_H$ ($CDCl_3$) 4.41 (2H, s), 7.73 (1H, s) and 8.38 (1H, s); m/z 213 ($M^+$, 9%), 215 ($M^+$, 9%) and 120 (100).

f) N-[2-Oxo-2-(4-cyano-2-furyl)ethyl]monamide

Hexamethylenetetramine (2.45g, 17.5mM) was added to a solution of 2-bromoacetyl-4-cyanofuran (3.75g, 17.5mM) in dichloromethane (35ml) and the mixture stirred at room temperature for 3h. The precipitated product was filtered off, washed and dried (6.2g); $\nu_{max}$ (KBr) 2245, 1705, 1573 and 1499cm$^{-1}$. This product was then suspended in methanol (15ml) and concentrated hydrochloric acid (7.5ml) added and the mixture stirred 1.5h at ambient temperature. The solid was filtered off and dried in vacuo (3.15g, 96%); $\nu_{max}$ (KBr) 2250 and 1694cm$^{-1}$; $\delta_H$ ($D_2O$) 7.70 (1H, s) and 8.50 (1H, s). The $\alpha$-aminoketone (0.4g) was reacted with the isobutoxyformic anhydride of monic acid using the standard method to give the title compound (0.11g, 26%); $\nu_{max}$ ($CHCl_3$) 3400, 2240, 1720, 1660 and 1630cm$^{-1}$; $\nu_{max}$ (EtOH) 220nm ($\epsilon_m$ 15,200); $\delta_H$ ($CD_3OD$) 0.95 (3H, d, J 7.0Hz, 17-$H_3$), 1.20 (3H, d, J 6.5Hz, 14-$H_3$), 2.15 (3H, s, 15-$H_3$), 4.53 (2H, s, 1′-H), 5.87 (1H, s, 2-H), 7.71 (1H, s, 3″-H) and 8.54 (1H, s, 5″-H); $\delta_C$ ($CD_3OD$) 10.7 (C-17), 17.4 (C-15), 18.7 (C-14), 31.4 (C-9), 40.1 (C-8), 42.1 (C-12 and C-4), 45.0 (C-1′), 55.3 (C-10), 59.6 (C-11), 64.7 (C-16), 68.4 (C-6), 69.1 (C-7), 70.0 (C-13), 74.6 (C-5), 99.5 (C-4″), 111.1 (-CN), 117.0 (C-3′), 118.8 (C-2), 151.4 (C-3), 152.1 (C-2″), 153.4 (C-5″), 168.2 (C-1) and 183.7 (C-2′); m/z (FAB thioglycerol) 477 ($[M+H]^+$, 38%), 499 ($[M+Na]^+$, 14%) and 200 (100%).

g) 5-(4-Cyano-2-furyl)-2-(1-normon-2-yl)oxazole

The above monamide (0.09g, 0.19mmol) was cyclised according to the general procedure and the oxazole isolated as a crisp foam (0.038g, 44%); $\nu_{max}$ ($CHCl_3$) 3400, 2250 and 1650cm$^{-1}$; $\delta_H$ ($CD_3OD$) 0.95 (3H, d, J 7.0Hz, 17-$H_3$), 1.20 (3H, d, J 6.5Hz, 14-$H_3$), 2.29 (3H, s, 15-$H_3$), 6.25 (1H, s, 2-H), 7.03 (1H, s, 3″-H), 7.48 (1H, s, 4′-H) and 8.38 (1H, s, 5″-H); $\delta_C$ ($CD_3OD$) 12.3 (C-17), 19.7 (C-15), 20.3 (C-14), 33.0 (C-9), 41.7 (C-8), 43.7 (C-12), 44.0 (C-4), 56.7 (C-10), 61.2 (C-11), 66.4 (C-16), 70.0 (C-6), 70.7 (C-7), 71.6 (C-13), 76.3 (C-5), 100.7 (C-4″), 108.1 (C-3″), 113.0 (C-2), 113.4 (-CN), 125.7 (C-4′), 141.6 (C-3), 146.5 (C-2′), 151.2 (C-2″), 152.0 (C-5″) and 163.2 (C-5′); m/z 458 ($M^+$, 10%) and 214 (100) (Found: 458.2009. $C_{24}H_{30}N_2O_7$ requires 458.2053).

Example 25

5-(5-Cyano-2-furyl)-2-(1-normon-2-yl)oxazole

a) 2-Acetyl-5-furoic acid

Methyl 2-acetyl-5-furoate (1g, 6mmol) was dissolved in methanol (10ml) and a solution of potassium hydroxide (0.5g, 8.7mmol) in water (4ml) added. The resulting solution was warmed for 30min at 60°C then cooled, acidified and extracted with ethyl acetate (x2). The combined extracts were washed with a small volume of saturated brine, dried ($MgSO_4$) and evaporated to give a brown amorphous solid (0.92g, 99%); $\nu_{max}$ (KBr) 3500-2200, 1690, 1570 and 1510cm$^{-1}$.

b) 2-Acetyl-5-carbamoylfuran

2-Acetyl-5-furoic acid (0.92g, 6mmol) was suspended in dichloromethane (20ml) and treated with oxalyl chloride (0.53ml, 6mmol) followed by dimethylformamide (catalytic amount). The reaction was stirred at ambient for 0.5h. Argon was then bubbled through the reacton for 0.5h before evaporating to give a brown solid; $\nu_{max}$ ($CHCl_3$) 1750 and 1690cm$^{-1}$. This product was dissolved in dry THF (25ml), cooled to 5°C and reacted with ammonia gas over 15 minutes. Argon was then bubbled through the reaction for 0.5h prior to partitioning between ethyl acetate and water. The organic phase was then washed with brine, dried ($MgSO_4$) and evaporated to give the title compound as a beige coloured powder (0.75g, 75%); $\nu_{max}$ (KBr) 3400, 1690 and 1670cm$^{-1}$; m/z 153 ($M^+$, 87.5%) and 130 (100).

c) 2-Acetyl-5-cyanofuran

2-Acetyl-5-carbamoylfuran (0.75g, 4.9mmol) was suspended in dichloromethane (25ml), chilled to 5°C

and treated with triethylamine (1.36ml, 9.8mmol) and trichloroacetyl chloride (0.61ml, 5.4mmol). The mixture was stirred for 0.5h, diluted with dichloromethane and washed sequentially with 1M hydrochloric acid, water, aqueous sodium hydrogen carbonate, brine then dried ($MgSO_4$), evaporated and subjected to silica chromatography eluting with dichloromethane. The title furan was isolated as a low melting solid (0.5g, 75%); $\nu_{max}$ ($CHCl_3$) 2240, 1690, 1570 and 1495cm$^{-1}$; $\delta_H$ ($CDCl_3$) 2.57 (3H, s) and 7.30 (2H, s).

d) 2-Bromoacetyl-5-cyanofuran

2-Acetyl-5-cyanofuran (0.47g, 3.5mmol) in dichloromethane (15ml) at 5°C under argon was treated with triethylamine (0.73ml), 5.25mmol) followed by trimethylsilyltrifluoromethanesulphonate (1ml, 5.25mmol). The cooling bath was removed and the reaction left for 0.5h. N-Bromosuccinimide (0.7g, 3.85mmol) was then added and the reaction continued. After 0.5h the reaction was diluted with dichloromethane, washed with water, brine, dried ($MgSO_4$) and evaporated. The crude product was purified by chromatography on silica (eluting with n-hexane:ethyl acetate 3:1) to give the title furan as a yellow mobile liquid which crystallised slowly (0.59g, 80%); $\nu_{max}$ ($CHCl_3$) 2240, 1690, 1570 and 1495cm$^{-1}$; $\delta_H$ ($CDCl_3$) 4.40 (2H, s), 7.35 (1H, d, J 4.5Hz) and 7.52 (1H, d, J 4.5Hz).

e) N-[2-Oxo-2-(5-cyano-2-furyl)ethyl]monamide

Hexamethylenetetramine (0.385g, 2.75mM) was added to a solution of 2-bromoacetyl-5-cyanofuran (0.59g, 2.75mM) in dichloromethane (6ml) and the mixture stirred 3h at room temperature. The resulting crystalline solid was filtered off, washed and dried (0.93g); $\nu_{max}$ (KBr) 2230, 1690, 1565, 1500 and 1460cm$^{-1}$. This product was suspended in methanol (2ml) and concentrated hydrochloric acid (1ml) added and the mixture stirred 1.5h at ambient temperature. The solid was filtered off, washed with methanol and dried in vacuo (0.38g, 74%); $\nu_{max}$ (KBr) 2240, 1705, 1570, 1485 and 1420cm$^{-1}$. The $\alpha$-aminoketone (0.38g) thus formed was reacted with te isobutoxyformic anhydride of monic acid using the standard method to give the title monamide (0.57g, 60%); $\nu_{max}$ ($CHCl_3$) 3420, 2230, 1700, 1660 and 1630cm$^{-1}$; $\lambda_{max}$ (EtOH) 267 ($\epsilon_m$ 18,300) and 220nm (14,500); $\delta_H$ ($CD_3OD$) 0.95 (3H, d, J 7.0Hz, 17-$H_3$), 1.20 (3H, d, J 6.5Hz, 14-$H_3$), 2.16 (3H, s, 15-$H_3$), 4.56 (2H, s, 1′-H), 5.88 (1H, s, 2-H), 7.48 (2H, s, 3″-H and 4′-H); $\delta_C$ ($CD_3OD$) 12.3 (C-17), 19.1 (C-15), 20.4 (C-14), 33.0 (C-9), 41.7 (C-8), 43.8 (C-4 and C-12), 46.8 (C-1′), 56.9 (C-10), 61.3 (C-11), 66.3 (C-16), 70.1 (C-6), 70.8 (C-7), 71.6 (C-13), 79.4 (C-5), 111.5 (-CN), 118.3 (C-4″), 120.4 (C-2), 124.6 (C-3″), 129.5 (C-5″), 153.7 (C-3), 155.0 (C-2″), 169.9 (C-1) and 185.4 (C-2′); m/z (FAB thioglycerol) 477 ([M+H]$^+$, 38%), 499 ([M+Na]$^+$, 10%) and 111 (100%).

f) 5-(5-Cyano-2-furyl)-2-(1-normon-2-yl)oxazole

The above monamide (0.35g, 0.73mmol) was cyclised according to the general procedure and the oxazole isolated as a foam (0.14g, 41%); $\nu_{max}$ ($CHCl_3$) 3400, 2220, 1650, 1630, 1520 and 1505cm$^{-1}$; $\lambda_{max}$ (EtOH) 322 ($\epsilon_m$ 29,360) and 240nm (7,460); $\delta_H$ ($CD_3OD$) 0.94 (3H, d, J 7.0Hz, 17-$H_3$), 1.20 (3H, d, J 6.4Hz, 14-$H_3$), 2.30 (3H, s, 15-$H_3$), 6.27 (1H, s, 2-H), 6.89 (1H, d, J 3.8Hz, 3″-H), 7.44 (1H, d, J 3.8Hz, 4″-H) and 7.58 (1H, s, 4′-H); $\delta_C$ ($CD_3OD$) 12.3 (C-17), 19.9 (C-15), 20.4 (C-14), 33.0 (C-9), 41.7 (C-8), 43.7 (C-12), 44.0 (C-4), 56.9 (C-10), 61.3 (C-11), 66.4 (C-16), 70.0 (C-6), 70.7 (C-7), 71.6 (C-13), 76.4 (C-5), 109.0 (C-4″), 112.2 (-CN), 113.0 (C-2), 125.3 (C-3″), 126.8 (C-5″), 126.9 (C-4′), 141.8 (C-3), 149.2 (C-2′), 151.7 (C-2″) and 163.5 (C-5′); m/z 458 (M$^+$, 27%) and 214 (100) (Found: M$^+$, 458.2059. $C_{24}H_{30}NO_7$ requires 458.2053).

## Example 26

### 5-(4-Formyl-2-furyl)-2-(1-normon-2-yl)oxazole

Trimethylsilyl chloride (0.26ml, 2.1mM) was added to a solution of 5-(4-cyano-2-furyl)-2-(1-normon-2-yl)-oxazole (0.105g, 0.23mM) (Example 24) in dry THF (10ml) and triethylamine (0.29ml, 2.1mM), containing a catalytic amount of 4-dimethylaminopyridine. The mixture was stirred for 1h at 5°C then filtered and evaporated. The residue was dissolved in dichloromethane (8ml), cooled to -40°C and diisobutylaluminium hydride (1.5M in toluene, 0.30ml, 0.45mM) added slowly, under an atmosphere of argon. After 1.75h reaction at -30°C a further quantity of diisobutylaluminium hydride (0.3ml, 0.45mM) was added and the reacton continued for a further 0.5h. The solvents were evaporated and the residual gel treated with ethyl acetate (10ml) and water (5ml) filtered and separated. The organic phase was dried and evaporated to give an oil which was dissolved in THF (4ml) and water (1ml), 5N hydrochloric acid (2 drops) was added. The mixture was stirred at ambient temperature for 5 minutes, diluted with sodium hydrogen carbonate solution and extracted with ethyl acetate (2 x 10ml). The combined organic extracts were washed with brine, dried and evaporated to give a yellow gum. This crude product was subjected to silica chromatography eluting with 5-10% methanol in dichloromethane to give the title compound as a white foam (0.048g, 48%); $\nu_{max}$ - ($CHCl_3$) 1690cm$^{-1}$; $\lambda_{max}$ ($CH_3CN$) 303 ($\epsilon_m$ 26,900) and 207nm (14,000); $\delta_H$ ($CDCl_3$) 0.95 (3H, d, J 7.0Hz, 17-

$H_3$), 1.22 (3H, d, J 6.5Hz, 14-$H_3$), 2.31 (3H, s, 15-$H_3$), 6.27 (1H, s, 2-H), 6.93 (1H, s, 3″-H), 7.36 (1H, s, 4′-H), 8.08 (1H, s, 5″-H) and 9.96 (1H, s, -CHO); $\delta_C$ ($CDCl_3$) 12.7 (C-17), 19.6 (C-15), 20.8 (C-14), 31.7 (C-9), 39.6 (C-8), 42.8 (C-12), 42.9 (C-4), 55.6 (C-10), 61.3 (C-11), 65.5 (C-16), 68.9 (C-6), 70.4 (C-7), 71.3 (C-13), 75.2 (C-5), 103.0 (C-5″), 112.6 (C-2), 124.5 (C-4′), 130.1 (C-4″), 140.7 (C-2″), 146.2 (C-3), 148.6 (C-2′), 150.8 (C-3″), 161.6 (C-5′) and 184.0 (-CHO); m/z 461 ($M^+$, 11%) and 217 (100).

Example 27

5-(2-Cyanofuran-4-yl)-2-(1-normon-2-yl)oxazole

a) Ethyl 2-bromo-4-furoate

Ethyl 3-furoate (6.0g, 42mM) dissolved in alumina dried chloroform (30ml) was treated dropwise with a solution of bromine (1.9ml, 71mM) dissolved in dry chloroform (10ml). The resulting mixture was then warmed at gentle reflux for 15h. The cooled mixture was diluted with dichloromethane, washed with 10% sodium carbonate solution (1 x 50ml). Water (2 x 50ml), dried ($MgSO_4$) and evaporated. The crude product was subjected to silica chromatography eluting with 20-50% dichloromethane/hexane to give the title compound as a colourless oil (5.5g, 60%); $\delta_H$ ($CDCl_3$) 1.35 (3H, t, J 7.0Hz, $CH_3CH_2$), 4.36 (2H, q, J 7.0Hz, $CH_3CH_2$), 6.74 (1H, s, 3-H) and 8.05 (1H, s, 5-H).

b) 2-Bromo-4-furoic acid

Ethyl 2-bromo-4-furoate (4.0g, 18.2mM) dissolved in ethanol (50ml) was treated with potassium hydroxide (1.53g, 27.3mM) in water (50ml) and the mixture warmed at 60°C for 0.5h. The ethanol was then evaporated and the residue dissolved in water (20ml), covered with ethyl acetate (50ml) and acidified to pH 1.0. The organic phase was washed with water (x1), with brine (x1), dried ($MgSO_4$) and evaporated to give the title compound as a white amorphous solid (3.0g, 86%), m.p.55°C; $\nu_{max}$ ($CHCl_3$) 3600-2400 and 1700$cm^{-1}$; $\delta_H$ ($CDCl_3$) 6.78 (1H, s, 3-H) and 8.15 (1H, s, 5-H).

c) 2-Bromofuran-4-N-methoxy-N-methylamide

2-Bromo-4-furoic acid (1.35g, 7.0mM) was dissolved in dichloromethane (20ml) at 5°C and treated with triethylamine (1.07ml, 7.7mM) followed by ethyl chloroformate (0.57ml, 7.1mM). The reaction was stirred for 0.5h at 5°C then treated with triethylamine (1.39ml, 10mM) followed by N,O-dimethylhydroxylamine hydrochloride (0.70g, 7.2mM) and left for a further 2.0h. The reaction was diluted with dichloromethane, washed with water, dried ($MgSO_4$) and evaporated. Silica chromatography eluting with 2:1 hexane:ethyl acetate gave the title compound as a colourless oil (1.10g, 70%) contaminated with 5-10% (nmr) of an unidentified impurity; $\nu_{max}$ ($CHCl_3$) 1630$cm^{-1}$; $\delta_H$ ($CDCl_3$) inter alia 3.30 (3H, s, $NCH_3$), 3.70 (3H, s, $OCH_3$), 6.75 (1H, s, 3-H) and 7.95 (1H, s, 5-H).

d) 4-Acetyl-2-bromofuran

N-Methoxy-N-methyl-2-bromo-4-furamide (1.10g, 4.7mM) in dry THF (30ml) at 5°C under argon was treated with methylmagnesium iodide (3M solution in ether, 1.86ml, 5.5mM) and the reaction stirred 1.5h. Saturated ammonium chloride solution was then added carefully until effervescence ceased and the mixture extracted with ethyl acetate, washed with brine (x1), dried and evaporated. Silica chromatography eluting with 2:1 hexane:ethyl acetate gave the title compound (0.40g, 45%) as a cream coloured solid, m.p.68-70°C; $\nu_{max}$ ($CHCl_3$) 1680$cm^{-1}$; $\delta_H$ ($CDCl_3$) 2.40 (3H, s, $CH_3$), 6.80 (1H, s, 3-H) and 8.08 (1H, s, 5-H).

e) 4-Acetyl-2-cyanofuran

4-Acetyl-2-bromofuran (0.36g, 2.0mM) was dissolved in N-methylpyrrolidone (2.5ml) and cuprous cyanide (0.18g, 2.1mM) added and the mixture heated at 200°C for 2.5h. The reaction was cooled, treated with cold 2N hydrochloric acid with vigorous stirring and extracted with dichloromethane. The organic extracts were washed with water (x1), brine (x1), dried, and concentrated to a small volume. This crude product, coated on silica (0.5g), was applied to a column (12g silica) and the column eluted with 3:1 hexane:ethyl acetate. The required product was isolated (0.20g, 90%) as an oil which solidified on chilling; $\nu_{max}$ ($CHCl_3$) 2230, 1730 and 1695$cm^{-1}$; $\delta_H$ ($CDCl_3$) 2.48 (3H, s, $CH_3$), 7.53 (1H, s, 3-H) and 8.23 (1H, s, 5-H).

f) 4-Bromoacetyl-2-cyanofuran

4-Acetyl-2-cyanofuran (0.50g, 3.7mM) dissolved in dichloromethane (14ml) at 5°C was treated with triethylamine (0.77ml, 5.5mM) followed by trimethylsilyl triflate (1.07ml, 5.5mM) under an atmosphere of argon. The reaction was allowed to warm to room temperature over 0.5h and then treated with N-bromosuccinimide (0.74g, 4.1mM). After 0.5h the reaction was diluted with dichloromethane, washed with water (x2), dried ($MgSO_4$) and evaporated. silica chromatography (eluting with 2:1 dichloromethane:hexane) of the crude product afforded the title compound (0.60g, 76%) as a yellow solid

m.p.72-75 °C; $\nu_{max}$ ($CHCl_3$) 2240 and 1690$cm^{-1}$; $\delta_H$ ($CDCl_3$) 4.25 (2H, s, $CH_2$), 7.60 (1H, s, 3-H) and 8.40 (1H, s, 5-H).

g) 4-Azidoacetyl-2-cyanofuran

Sodium azide (0.17g, 2.6mM) was added to a solution of 4-bromoacetyl-2-cyanofuran (0.56g, 2.6mM) in a mixture of acetone (5ml) and water (1.5ml) at 5 °C. The mixture was stirred for 1.0h, diluted with ether, washed with brine (x1), dried ($MgSO_4$) and evaporated. The crude product was purified by silica chromatography (graded elution: 2:1 to 3.5:1 dichloromethane:hexane) to give the title compound (0.36g, 80%); $\nu_{max}$ ($CHCl_3$) 2240, 2120 and 1700$cm^{-1}$; $\delta_H$ ($CDCl_3$) 4.38 (2H, s, $CH_2$), 7.58 (1H, s, 2-H) and 8.38 (1H, s, 5-H).

h) 4-Aminoacetyl-2-cyanofuran

4-Azidoacetyl-2-cyanofuran (0.28g, 16.0mM) was dissolved in THF (10ml) and to this solution water (5ml) and 1N hydrochloric acid (3.4ml) were added. This solution was subjected to hydrogenation using 10% palladium on carbon (0.05g) as catalyst for 5 minutes. The catalyst was removed on a celite pad and the filtrate washed with ethyl acetate and evaporated to give the required product (0.26g), 90%); $\nu_{max}$ (KBr) 2250 and 1690$cm^{-1}$; $\delta_H$ ($D_2O$) 4.48 (2H, s, $CH_2$), 7.70 (1H, s, 3-H) and 8.60 (1H, s, 5-H).

i) N-[2-Oxo-2(2-cyanofuran-4-yl)ethyl]monamide

4-Aminoacetyl-2-cyanofuran (0.36g, 2.0mM) was reacted to give the title monamide (0.28g, 29%) using the general method; $\nu_{max}$ (KBr) 2240, 1775, 1700, 1660 and 1640$cm^{-1}$; $\lambda_{max}$ (EtOH) 227nm ($\epsilon_m$ 25,000); $\delta_H$ ($CD_3OD$) 0.45 (3H, d, J 7.0Hz, 17-$H_3$), 1.21 (3H, d, J 6.5Hz, 14-$H_3$), 2.15 (3H, s, 15-$H_3$), 4.48 (2H, s, 1′-$H_2$), 5.85 (1H, s, 2-H), 7.70 (1H, s, 3″-H) and 8.67 (1H, s, 5″-H); $\delta_C$ ($CD_3OD$) 12.3 (C-17), 19.0 (C-15), 20.3 (C-14), 33.0 (C-9), 41.7 (C-8), 43.7 (C-4 and C-12), 56.7 (C-10), 61.2 (C-11), 66.3 (C-16), 70.0 (C-6), 70.7 (C-7), 71.6 (C-13), 76.2 (C-5), 111.3 (C≡N), 120.4 (C-3″), 121.4 (C-2), 127.4 (C-4″), 128.8 (C-2″), 153.2 (C-5″), 153.6 (C-3), 169.8 (C-1) and 190.3 (C-2′); m/z (FAB 3-NOBA/Na) 499 ($MNa^+$, 11%), 176 (100) and 154 (100).

j) N-[2-Oxo-2-(2-cyanofuran-4-yl)ethyl](6,7,13-tris-trimethylsilyloxy)monamide

A solution of the monamide (0.26g, 0.55mM) in dry THF at 5 °C under argon was treated sequentially with a catalytic amount of dimethylaminopyridine, triethylamine (0.62ml, 4.4mM) and trimethylsilyl chloride (0.42ml, 3.3mM) and the reaction stirred for 2.0h. The precipitated solids were filtered off and the filtrate evaporated. The residue was chromatographed on a silica column (15g) eluting with 40% ethyl acetate/hexane to give the title monamide (0.34g, 88%); $\delta_H$ ($CDCl_3$) 0.07 to 0.23 (27H, m, silyl methyls), 0.90 (3H, d, J 7.0Hz, 17-$H_3$), 1.20 (3H, d, J 6.5Hz, 14-$H_3$), 2.20 (3H, s, 15-$H_3$), 4.56 (2H, d, J 4.7Hz, 1′-$H_2$), 5.75 (1H, s, 2-H), 6.25 (1H, t, J 4.7Hz, NH), 7.48 (1H, s, 3″-H) and 8.26 (1H, s, 5″-H).

k) 5-(2-Cyanofuran-4-yl)-2-(1-nor-6,7,13-tristrimethylsilyloxymon2-yl)oxazole

A solution of the tris(trimethylsilyl) protected monamide (0.34g, 0.48mM) in dichloromethane (15ml) at 5 °C under argon was treated sequentially with a catalytic amount of dimethylaminopyridine, pyridine (0.12ml, 1.44mM) and trichloroacetylchloride (0.11ml, 0.96mM). After 1.0h the reaction was diluted with dichloromethane, washed with sodium hydrogen carbonate solution, brine, dried ($MgSO_4$) and evaporated. The residue was subjected to a silica coumn (12g) eluting with 3:1 hexane:ethyl acetate to give the title oxazole (0.12g, 37%); $\delta_H$ ($CDCl_3$) 0.09 to 0.20 (27H, m, silyl methyl's), 0.90 (3H, d, J 7.0Hz, 17-$H_3$), 1.20 (3H, d, J 6.5Hz, 14-$H_3$), 2.28 (3H, s, 15-$H_3$), 6.23 (1H, s, 2-H), 7.21 (1H, s, 4′-H), 17.30 (1H, s, 3″-H) and 7.83 (1H, s, 5″-H).

l) 5-(2-Cyanofuran-4-yl)-2-(1-normon-2-yl)oxazole

A solution of the oxazole (0.12g, 0.18mM) in THF (4ml) and water (1ml) was treated with 5N hydrochloric acid (2 drops) at room temperature with stirring under argon for 4 minutes. The reaction was then quenched with sodium hydrogen carbonate solution, partitioned between ethyl acetate and water and the organic phase washed with brine, dried ($MgSO_4$) and evaporated. The residue was subjected to a silica column (2.5g) eluting with 7.5% methanol/dichloromethane to give the title oxazole (0.073g, 90%) as a white foam; $\nu_{max}$ ($CHCl_3$) 2230 and 1655$cm^{-1}$; $\lambda_{max}$ (EtOH) 284nm ($\epsilon_m$ 24,300); $\delta_H$ ($CD_3OD$) 0.94 (3H, d, J 7.0Hz, 17-$H_3$), 1.20 (3H, d, J 6.5Hz, 14-$H_3$), 2.28 (3H, s, 15-$H_3$), 6.22 (1H, s, 2-H), 7.33 (1H, s, 4′-H), 7.68 (1H, s, 3″-H) and 8.23 (1H, s, 5″-H); $\delta_C$ ($CD_3OD$) 11.3 (C-17), 18.8 (C-15), 19.3 (C-14), 32.0 (C-9), 40.7 (C-8), 42.8 (C-12), 42.9 (C-4), 55.9 (C-10), 60.3 (C-11), 65.4 (C-16), 69.0 (C-6), 69.7 (C-7), 70.7 (C-13), 75.4 (C-5), 110.7 (C≡N), 112.2 (C-2), 116.8 (C-2″), 119.5 (C-3″), 124.1 (C-4′), 127.6 (C-4″), 141.8 (C-3), 144.0 (C-5″), 149.2 (C-2′) and 161.8 (C-5′); m/z 458 ($M^+$, 22%) and 214 (100%). (Found: $M^+$, 458.2041. $C_{24}H_{30}N_2O_7$ requires 458.2053).

Example 28

5-(2-Methoxyformimidoylfuran-4-yl)-2-(1-normon-2-yl)oxazole

The monamide [Example 27(i)] (0.094g, 0.2mM) was cyclised according to the general method. The title oxazole was the major product (0.045g, 46%) [the cyanofuranyloxazole (Example 27) was also isolated (0.01g, 10%)]; $\nu_{max}$ ($CHCl_3$) 1660cm$^{-1}$; $\lambda_{max}$ (EtOH) 240 ($\epsilon_m$ 15,900) and 286nm (20,900); $\delta_H$ ($CD_3OD$) 0.95 (3H, d, J 7.0Hz, 17-$H_3$), 1.20 (3H, d, J 6.5Hz, 14-$H_3$), 2.28 (3H, s, 15-$H_3$), 3.89 (3H, s, OC$H_3$), 6.22 (1H, s, 2-H), 7.20 (1H, s, 3″-H), 7.34 (1H, s, 4′-H) and 8.08 (1H, s, 5″-H); $\delta_C$ ($CD_3OD$) 12.3 (C-17), 19.8 (C-15), 20.4 (C-14), 33.0 (C-9), 41.7 (C-8), 43.8 (C-12), 43.9 (C-4), 53.9 (O$CH_3$), 56.9 (C-10), 61.3 (C-11), 66.4 (C-16), 70.1 (C-6), 70-1 (C-7), 71.7 (C-13), 76.4 (C-5), 110.3 (C-3″), 113.4 (C-2), 118.0 (C-2″), 124.2 (C-4′), 142.4 (C-5″), 144.1 (C-3), 147.6 (C-2′), 149.6 (C-4″), 161.3 (C=N) and 162.5 (C-5′); m/z 490 ($M^+$, 17.5%) and (24, 100). [Found: $M^+$, 490.2317. $C_{25}H_{34}N_2O_8$ requires $M^+$, 490,2315].

Example 29

5-(4-Methylfuran-2-yl)-2-(1-normon-2-yl)oxazole

a) 4-Methylfuran-2-carbaldehyde diethyl acetal
4-Bromofurfural diethyl acetal (7.4g, 30mM) in dry THF (25ml) was added dropwise to n-butyllithium (1.26M in hexane, 25ml, 31mM) at -70°C with stirring under argon. After 0.5h at -70°C methyl iodide (2.5ml, 40mM) was added and the reaction allowed to warm to 0°C over 2.5h. The reaction was then partitioned between ethyl acetate (100ml) and brine (50ml) and the organic phase separated and washed x1 with brine, dried ($MgSO_4$) and evaporated. Silica chromatography (1:1 dichloromethane:hexane) gave the required product as a straw-coloured liquid (4g, 69%) contaminated with approx. 5-10% (nmr) of an unidentified impurity; $\delta_H$ ($CDCl_3$) inter alia 1.23 (6H, t, J 7.5Hz), 2.02 (3H, s), 3.64 (4H, q, J 7.5Hz), 5.54 (1H, s), 6.34 [1H, s (broadened), 3-H] and 7.23 (1H, s (broadened), 5-H]; m/z 184 ($M^+$, 7%) and 111 (100).

b) 4-Methylfuroic acid
4-Methylfurfural diethyl acetal (4g, 22mM) dissolved in diethyl ether (30ml) was treated with 2N hydrochloric acid (16ml) and stirred at ambient 2.0h. The reaction was diluted with ether and washed consecutively with water, sodium hydrogen carbonate solution, brine and then concentrated. $\delta_H$ ($Et_2O$) inter alia 7.12 (1H, s, 3-H), 7.60 (1H, s, 5-H) and 9.66 (1H, s, CHO). This aldehyde in a small volume of diethyl ether (10ml) was added to a Tollens solution containing silver nitrate (7.6g, 44mM) in water (75ml) + sodium hydroxide (7.6g) in water (75g) with concentrated ammonia (0.88g/ml) added until clear solution obtained, and the resulting mixture stirred in a stoppered flask 3.0h at ambient. The reaction was then filtered on a celite pad, the filtrate diluted with ether and 30% sulphuric acid added to pH 2.0. The organic phase was separated and the aqueous phase extracted x2 with ether. The combined organic extracts were washed x2 with brine, dried ($MgSO_4$) and evaporated to give the title compound (2.0g, 74%) as a solid; $\nu_{max}$ ($CHCl_3$) 3600-2400, 1690 and 1600cm$^{-1}$; $\delta_H$ ($CDCl_3$) inter alia 2.08 (3H, s, $CH_3$), 7.25 (1H, s, 3-H), 7.48 (1H, s, 5-H) and 9.69 (1H, s, exchanges with $D_2O$, $CO_2H$); m/z 126 ($M^+$, 100%).

c) 2-Bromoacetyl-4-methylfuran
4-Methylfuroic acid (2.09g, 16.6mM) dissolved in dry dichloromethane under argon was treated dropwise with oxalyl chloride (1.59ml, 18.2 mM) followed by dimethylformamide (2 drops) and the reaction left 1.5h at ambient. The resulting solution was evaporated and azeotroped x2 with dry benzene. This acid chloride dissolved in dichloromethane (ca.20ml) was added dropwise to a solution of diazomethane (ca.70mM) in ether at 5°C and the reaction left for 0.5h. $\nu_{max}$ (film) 2100 (v.s.), 1680 and 1600cm$^{-1}$. Concentrated hydrobromic acid was then added until no diazoketone remained. The mixture was diluted with ether and water (10ml), the phases separated and the organic phase washed with water, sodium hydrogen carbonate solution, brine, dried ($MgSO_4$) and evaporated. The crude product was subjected to a silica column (50g, dichloromethane) and the title compound isolated as a mobile oil (2.12g, (64%) contaminated with ca.15% of chloroacetyl compound; $\nu_{max}$ ($CHCl_3$) 1670 and 1660cm$^{-1}$; $\delta_H$ ($CDCl_3$) 2.08 (3H, s, $CH_3$), 4.28 and 4.54 (2H, 2s, $CH_2Br$ and $CH_2Cl$), 7.24 (1H, s, 3-H) and 7.49 [1H, s (perturbed), 5-H].

d) 2-Azidoacetyl-4-methylfuran
Tetramethylguanidinium azide (0.58g, 3.6mM) was added to an ice-cooled solution of 2-bromoacetyl-4-methylfuran (0.67g, 3.3mM) in dichloromethane (25ml) under argon. The mixture was stirred for 1.5h, reduced in volume and applied to a silica column (20g) eluting with 2:1 hexane:ethyl acetate. The title

compound was isolated as a low melting pale yellow solid (0.53g, 96%); $\nu_{max}$ ($CHCl_3$) 2100, 1690 and 1600cm$^{-1}$; $\delta_H$ ($CDCl_3$) 2.10 (3H, s, $CH_3$), 4.42 (2H, s, $CH_2N_3$), 7.25 (1H, s, 3-H) and 7.52 [1H, s (perturbed), 5-H].

e) 2-Aminoacetyl-4-methylfuran hydrochloride

2-Azidoacetyl-4-methylfuran (0.49g, 3.0mM) was dissolved in THF (20ml) and to this solution water (6ml) and 1N hydrochloric acid (6ml, 6.0mM) were added. The mixture was hydrogenated over 10% palladium on carbon (0.1g) for 10 minutes, filtered through a celite pad and the filtrate washed with ethyl acetate and evaporated to give the required product (0.5g, 95%); $\nu_{max}$ (KBr) 1680 and 1480cm$^{-1}$; $\delta_H$ ($D_2O$) 2.05 (3H, s, $CH_3$), 4.43 (2H, s, $CH_2$), 7.40 (1H, s, 3-H) and 7.42 (1H, s (br.), 5-H); m/z 139 ($M^+$, 95%) and 109 (100).

f) N-[2-Oxo-2-(4-methylfuran-2-yl)ethyl]monamide

2-Aminoacetyl-4-methylfuran hydrochloride (0.48g, 2.3mM) was reacted to give the title monamide (0.44g, 41%) using the general method; $\nu_{max}$ ($CHCl_3$) 1665, 1635 and 1595cm$^{-1}$; $\lambda_{max}$ (EtOH) 222 ($\epsilon_m$ 17,000) and 278nm ($\epsilon_m$ 14,800); $\delta_H$ ($CD_3OD$) 0.95 (3H, d, J 7.0Hz, 17-$H_3$), 1.20 (3H, d, J 6.5Hz, 14-$H_3$), 2.10 (3H, s, furan $CH_3$, 2.16 (3H, s, 15-$H_3$), 4.50 (2H, s, 1′-$H_2$), 5.88 (1H, s, 2-H), 7.28 (1H, s, 3″-H), and 7.58 (1H, s, 5″-H); $\delta_C$ ($CD_3OD$) 9.3 (furan $CH_3$), 12.1 (C-17), 19.0 (C-15), 20.3 (C-14), 33.0 (C-9), 41.6 (C-8), 43.7 (C-4 and C-12), 46.1 (C-1′), 56.9 (C-10), 61.2 (C-11), 66.3 (C-16), 70.0 (C-6), 70.7 (C-7), 71.6 (C-13), 76.2 (C-5), 120.6 (C-2), 121.3 (C-3″), 124.4 (C-4), 145.8 (C-5″), 152.3 (C-3), 153.2 (C-2), 169.8 (C-1) and 185.7 (C-2′); m/z 465 ($M^+$, 22%) and 221 (100). (Found: $M^+$ 465.2362 ($C_{24}H_{35}NO_8$ requires M 465.2363).

g) 5-(4-Methylfuran-2-yl)-2-(1-normon-2-yl)oxazole

The monamide (0.43g, 0.92mM) was cyclised according to the general method to give the title oxazole (0.25g, 60%); $\nu_{max}$ ($CHCl_3$) 1710, 1655 and 1580cm$^{-1}$; $\lambda_{max}$ (EtOH) 233 ($\epsilon_m$ 7,150) and 310nm ($\epsilon_m$ 22,900); $\delta_H$ ($CD_3OD$) 0.95 (3H, d, J 7.0Hz, 17-$H_3$), 1.20 (3H, d, J 6.5H, 14-$H_3$), 2.08 (3H, s, furan $CH_3$), 2.27 (3H, s, 15-$H_3$), 6.22 (1H, s, 2-H), 6.57 (1H, s, 3″-H), 7.24 (1H, s, 4′-H) and 7.36 (1H, s, 5″-H); $\delta_C$ - ($CD_3OD$) 9.5 (furan $CH_3$), 12.2 (C-17), 19.7 (C-15), 20.3 (C-14), 33.0 (C-9), 4.6 (C-8), 43.7 (C-12), 43.9 (C-4), 56.9 (C-10), 61.3 (C-11), 66.4 (C-16), 70.0 (C-6), 70.7 (C-7), 71.6 (C-13), 76.4 (C-5), 110.8 (C-3″), 113.3 (C-2), 122.7 (C-4′), 123.3 (C-4″), 141.1 (C-5″), 144.0 (C-2″), 144.7 (C-3), 149.5 (C-2′) and 162.0 (C-5′); m/z 447 ($M^+$, 24%) and 203 (100). (Found: $M^+$, 447.2254. $C_{24}H_{33}NO_7$ requires M 447.2257).

## Example 30

### 5-(4-Hydroxymethylfur-2-yl)-2-(1-normon-2-yl)oxazole

A solution of 5-(4-formylfuran-2-yl)-2-(1-normon-2-yl)oxazole (example 13) (0.065g, 0.14mM) in methanol (5ml) was treated with sodium borohydride (0.031g, 0.33mM) at room temperature under argon. After 15mins. glacial acetic acid (2 drops) was added to quench the reaction and the mixture coated onto silica (0.2g) and applied to a silica column (2g) eluting with 2-10% methanol in dichloromethane to give the title oxazole (0.06g, 92%); $\nu_{max}$ ($CHCl_3$) 3400 and 1655cm$^{-1}$; $\lambda_{max}$ (EtOH) 308nm ($\epsilon_m$ 23,310); $\delta_H$ ($CD_3OD$) 0.95 (3h, d, J 7.0Hz, 17-$H_3$), 1.20 (3H, d, J 6.5Hz, 14-$H_3$), 2.27 (3H, s, 15-$H_3$), 4.51 (2H, s, -$CH_2OH$), 6.23 (1H, s, 2-H), 6.73 (1H, s, 3″-H), 7.29 (1H, s, 4′-H) and 7.55 (1H, s, 5″-H); $\delta_C$ ($CD_3OD$) 12.3 (C-17), 19.7 (C-15), 20.3 (C-14), 33.0 (C-9), 41.7 (C-8), 43.7 (C-12), 43.9 (C-4), 56.4 (-$CH_2OH$), 56.9 (C-10), 61.3 (C-11), 66.4 (C-16), 70.0 (C-6), 70.7 (C-7), 71.6 (C-13), 76.4 (C-5), 108.5 (C-3″), 113.3 (C-2), 123.1 (C-4′), 129.2 (C-4″), 141.5 (C-5″), 143.6 (C-2″), 145.2 (C-3), 149.8 (C-2′) and 162.2 (C-5′); m/z 463 ($M^+$, 21%) and 219 (100). (Found: $M^+$, 463.2211. $C_{24}H_{33}NO_8$ requires M 463.2206).

## Biological Data

The activity of the normonyl derivatives of the Examples against various bacteria which are important in the diseases of humans was assayed in vitro using serial dilutions in nutrient agar with 5% chocolated horse blood. The MIC's were determined after incubation for 18h at 37°C and are shown in the Table.

| MICs ($\mu$g/ml) against Human Bacteria | | | | | | | | | | |
|---|---|---|---|---|---|---|---|---|---|---|
| Organism | Example No. | | | | | | | | | |
| | 1 | 2 | 3 | 4 | 5 | 6 | 7 | 8 | 9 | 10 |
| H.influenzae Q1 | 4 | 8 | 2 | 0.25 | - | 0.5 | 1 | 2 | 4 | 1 |
| B.catarrhalis 1502 | 4 | 8 | 2 | 0.25 | 64 | 1 | 1 | 0.5 | 1 | - |
| Strep.pyogenes CN10 | 0.5 | 8 | 1 | 1 | 32 | 0.5 | 0.5 | 0.25 | 0.25 | - |
| Strep.pneumoniae PU7 | 0.25 | 4 | 1 | - | 32 | 1 | 0.25 | 0.5 | 2 | 0.5 |
| Staph.aureus Oxford | 1 | 8 | 2 | 1 | 32 | 1 | 0.5 | 0.5 | 1 | 1 |

MICs (μg/ml) against Human Bacteria

| Organism | Example No. | | | | | | | | |
|---|---|---|---|---|---|---|---|---|---|
| | 11 | 13 | 14 | 15 | 16 | 17 | 18 | 19 | 20 |
| H.influenzae Q1 | 0.25 | - | 2 | 3 | 0.5 | 1 | 1 | 0.5 | 16 |
| B.catarrhalis 1502 | - | 4 | 4 | 8 | 4 | 4 | 2 | 2 | 64 |
| Strep.pyogenes CN10 | - | 1 | 1 | 4 | 1 | 1 | 2 | 1 | 64 |
| Strep.pneumoniae PU7 | 1 | 1 | 1 | - | - | 0.25 | 2 | 2 | 128 |
| Staph.aureus Oxford | 2 | 1 | 2 | 2 | 1 | 2 | 2 | 2 | >128 |

| Organism | Example No. | | | | | | | | | |
|---|---|---|---|---|---|---|---|---|---|---|
| | 21 | 22 | 23 | 24 | 25 | 26 | 27 | 28 | 29 | 30 |
| H.influenzae Q1 | 0.5 | 16 | 4 | 0.5 | 2 | 2 | - | 4 | 1 | 0.5 |
| B.catarrhalis 1502 | 8 | 32 | 8 | 0.03 | 2 | 2 | 2 | 8 | 4 | 2 |
| Strep.pyogenes CN10 | 0.5 | 8 | 4 | 1 | 1 | 1 | - | - | 2 | 1 |
| Strep.pneumoniae PU7 | 1 | 32 | 16 | 1 | 0.13 | 2 | 0.5 | 16 | 2 | 1 |
| Staph.aureus Oxford | 8 | 64 | 2 | 0.5 | 2 | 2 | - | 16 | 2 | 2 |

## Claims

### Claims for the following Contracting States : AT, BE, CH, DE, DK, FR, GB, GR, IT, LI, LU, NL, SE

1. A compound of formula (I):

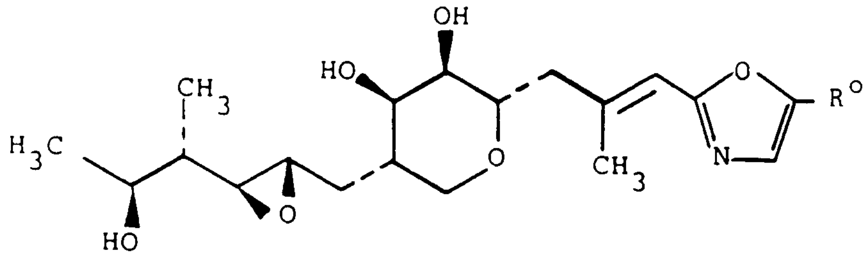

(I)

in which $R^o$ denotes a group of the formula (a), (b) or (c):

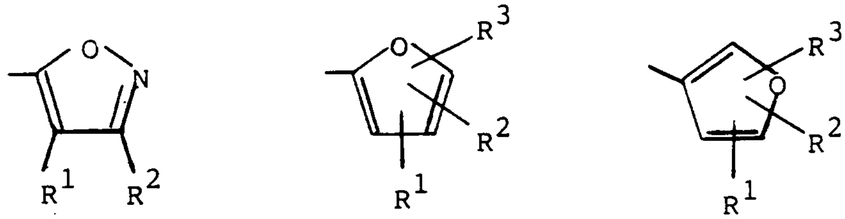

(a) (b) (c)

in which formulae $R^1$, $R^2$, and $R^3$ are the same or different and each is selected from hydrogen, halogen, optionally substituted $(C_{1-6})$alkyl, aryl, aralkyl, heterocyclyl, $(C_{1-6})$alkoxy, hydroxy, carboxy and salts thereof, $(C_{1-6})$alkoxycarbonyl, carbamoyl, mono- or di-$(C_{1-6})$alkylcarbamoyl, sulphamoyl, mono- and di-$(C_{1-6})$alkylsulphamoyl, cyano, nitro, amino, mono-and di-$(C_{1-6})$alkylamino, acylamino, ureido, $(C_{1-6})$-alkoxycarbonylamino, $(C_{1-6})$alkoxyimino, 2,2,2-trichloroethoxycarbonylamino, acyl, $(C_{1-6})$alkylthio, arylthio, $(C_{1-6})$alkanesulphinyl, arylsulphinyl, $(C_{1-6})$alkanesulphonyl and arylsulphonyl.

2. A compound as claimed in claim 1 in which $R^1$ and $R^3$ is each hydrogen.

3. A compound as claimed in claim 1 in which $R^o$ denotes a group of the formula (a) and $R^1$ is hydrogen.

4. A compound as claimed in claim 1 in which $R^o$ denotes a group of the formula (b) and $R^1$ and $R^3$ is each hydrogen.

5. A compound of formula (I) as defined in claim 1 selected from:
2-(1-normon-2-yl)-5-(3-phenylisoxazol-5-yl)oxazole;
2(1-normon-2-yl)-5-(3-p-methylthiophenylisoxazol-5-yl)oxazole;
2-(1-normon-2-yl)-5-(3-p-methylsulphonylphenylisoxazol-5-yl)oxazole;
2-(1-normon-2-yl)-5-(3-methylisoxazol-5-yl)oxazole;
5-(3-bromo-5-isoxazolyl)-2-(1-normon-2-yl)oxazole;
2-(1-normon-2-yl)-5-(3-methoxy-5-isoxazolyl)oxazole;
2-(1-normon-2-yl)-5-(3-isopropyl-5-isoxazolyl)oxazole;
5-(3-t-butyl-5-isoxazolyl)-2-(1-normon-2-yl)oxazole;
2-(1-normon-2-yl)-5-(3-ethoxycarbonyl-5-isoxazolyl)oxazole;
2-(1-normon-2-yl)-5-(3-formyl-5-isoxazolyl)oxazole;
2-(1-normon-2-yl)-5-(3-hydroxymethyl-5-isoxazolyl)oxazole;
2-(1-normon-2-yl)-5-(3-pyrrolidin-1-yl-5-isoxazolyl)oxazole;
5-(2-furyl)-2-(1-normon-2-yl)oxazole;
5-(5-bromo-2-furyl)-2-(normon-2-yl)oxazole;
5-(3-furyl)-2-(1-normon-2-yl)oxazole;

5-(5-methyl-2-furyl)-2-(1-normon-2-yl)oxazole;
5-(5-methoxycarbonyl-2-furyl)-2-(1-normon-2-yl)oxazole;
5-(5-hydroxymethyl-2-furyl)-2-(1-normon-2-yl)oxazole;
sodium 5-(5-carboxylato-2-furyl)-2(1-normon-2-yl)oxazole;
5-(5-carboxyamido-2-furyl)-2-(1-normon-2-yl)oxazole;
5-(5-methoxyformimidoyl-2-furyl)-2-(1-normon-2-yl)oxazole;
5-(2,5-dimethyl-3-furyl)-2-(1-normon-2-yl)oxazole;
5-(4-cyano-2-furyl)-2-(1-normon-2-yl)oxazole;
5-(5-cyano-2-furyl)-2-(1-normon-2-yl)oxazole;
5-(4-formyl-2-furyl)-2-(1-normon-2-yl)oxazole;
5-(2-cyanofuran-4-yl)-2-(1-normon-2-yl)oxazole;
5-(2-methoxyformimidoylfuran-4-yl)-2-(1-normon-2-yl)oxazole;
5-(4-methylfuran-2-yl)-2-(1-normon-2-yl)oxazole; and
5-(4-hydroxymethylfur-2-yl)-2-(1-normon-2-yl)oxazole.

**6.** A process for the preparation of a compound of formula (I) as defined in claim 1 which process comprises reacting a compound of formula (III):

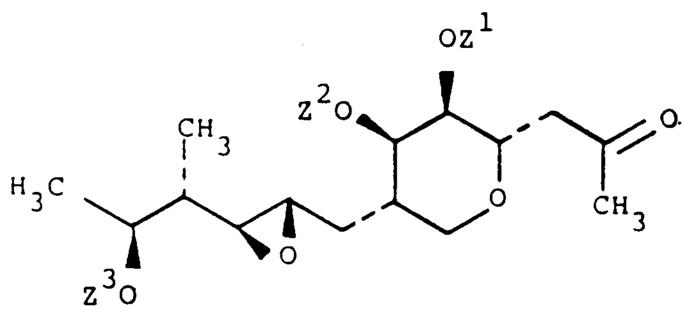

(III)

in which $Z^1$, $Z^2$ and $Z^3$ are the same or different and each is hydrogen or a hydroxyl-protecting group, with a compound of formula (IV):

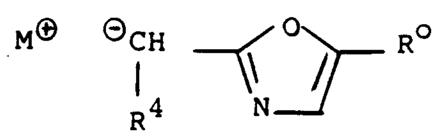

(IV)

in which:
$R^o$ is as defined in relation to formula (I),
$M^+$ is a metal cation, and $R^4$ is an anion-stabilising group which will spontaneously eliminate with a $\beta$-hydroxyl group to produce an olefin;
and, where necessary, removing any hydroxyl-protecting groups.

**7.** A process for the preparation of a compound of formula (I) as defined in claim 1 which process comprises cyclising a compound of formula (VII):

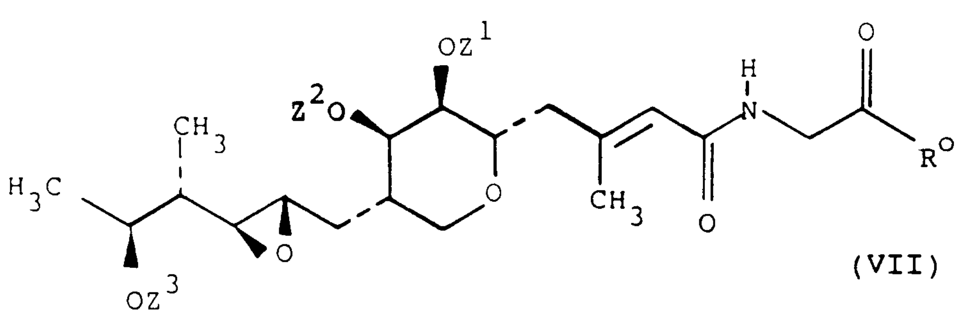

(VII)

in which R° is as defined in relation to formula (I), and $Z^1$, $Z^2$ and $Z^3$ are the same or different and each is hydrogen or a hydroxyl-protecting group; and, where necessary, removing any hydroxyl-protecting groups.

8. A process for the preparation of a compound of formula (I) as defined in claim 1 which process comprises treating a compound of formula (VIII):

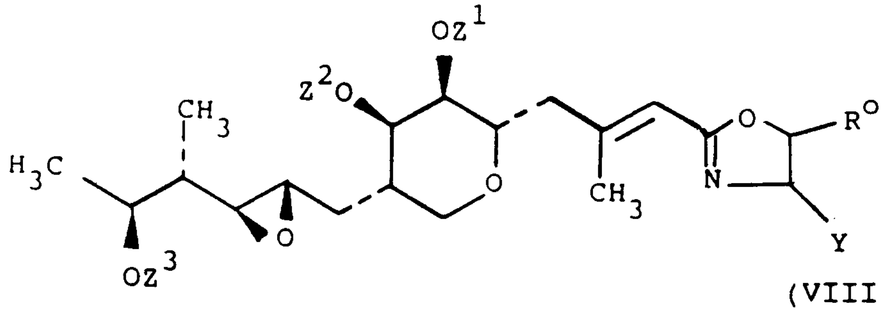

(VIII)

in which $Z^1$, $Z^2$, and $Z^3$ are the same or different and each is hydrogen or a hydroxyl-protecting group, R° isas defined in claim 1, and Y is a leaving group; with a strong base, and, where necessary removing any hydroxyl-protecting group.

9. A pharmaceutical or veterinary composition which comprises a compound as claimed in any one of claims 1 to 5 together with a pharmaceutically or veterinarily acceptable carrier or excipient.

10. A compound as claimed in any one of claims 1 to 5 for use in therapy.

**Claims for the following Contracting State : ES**

1. A process for the preparation of a compound of formula (I):

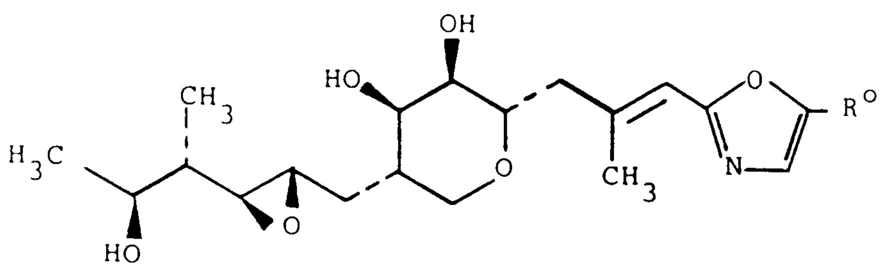

(I)

in which R° denotes a group of the formula (a), (b) or (c):

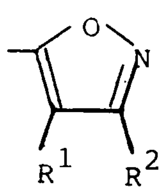

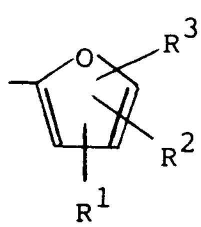

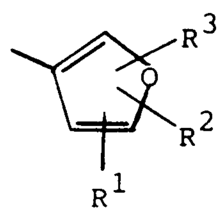

(a) (b) (c)

in which formulae $R^1$, $R^2$, and $R^3$ are the same or different and each is selected from hydrogen, halogen, optionally substituted $(C_{1-6})$alkyl, aryl, aralkyl, heterocyclyl, $(C_{1-6})$alkoxy, hydroxy, carboxy and salts thereof, $(C_{1-6})$alkoxycarbonyl, carbamoyl, mono- or di-$(C_{1-6})$alkylcarbamoyl, sulphamoyl, mono- and di$(C_{1-6})$alkylsulphamoyl, cyano, nitro, amino, mono-and di-$(C_{1-6})$alkylamino, acylamino, ureido, $(C_{1-6})$alkoxycarbonylamino, $(C_{1-6})$alkoxyimino, 2,2,2-trichloroethoxycarbonylamino, acyl, $(C_{1-6})$alkylthio, arylthio, $(C_{1-6})$alkanesulphinyl, arylsulphinyl, $(C_{1-6})$alkanesulphonyl and arylsulphonyl; which process comprises reacting a compound of formula (III):

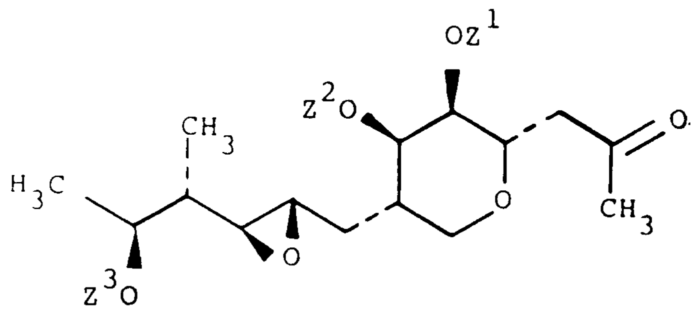

(III)

in which $Z^1$, $Z^2$ and $Z^3$ are the same or different and each is hydrogen or a hydroxyl-protecting group, with a compound of formula (IV):

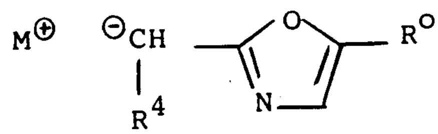

(IV)

in which:
$R^o$ is as defined in relation to formula (I),
$M^+$ is a metal cation, and $R^4$ is an anion-stabilising group which will spontaneously eliminate with a $\beta$-hydroxyl group to produce an olefin;
and, where necessary, removing any hydroxyl-protecting groups.

**2.** A process for the preparation of a compound of formula (I) as defined in claim 1 which process comprises cyclising a compound of formula (VII):

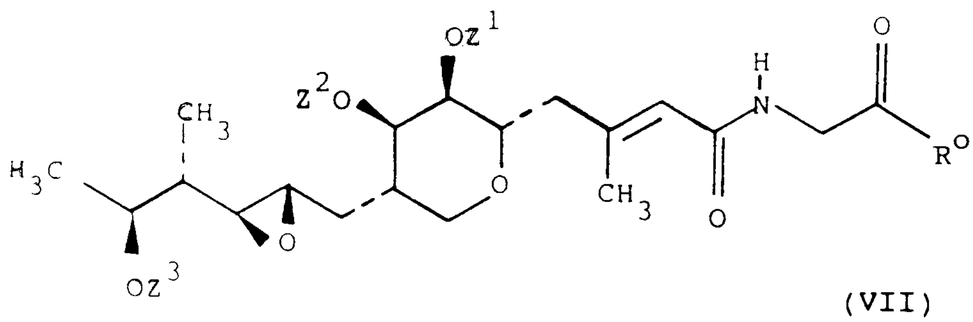

(VII)

in which $R^o$ is as defined in relation to formula (I), and $Z^1$, $Z^2$ and $Z^3$ are the same or different and each is hydrogen or a hydroxyl-protecting group; and where necessary, removing any hydroxyl-protecting groups.

**3.** A process as claimed in claim 2 in which cyclisation is effected using a chlorinating agent, a carboxylic anhydride, a mixed anhydride or an acid chloride.

4. A process for the preparation of a compound of formula (I) as defined in claim 1 which process comprises treating a compound of formula (VIII):

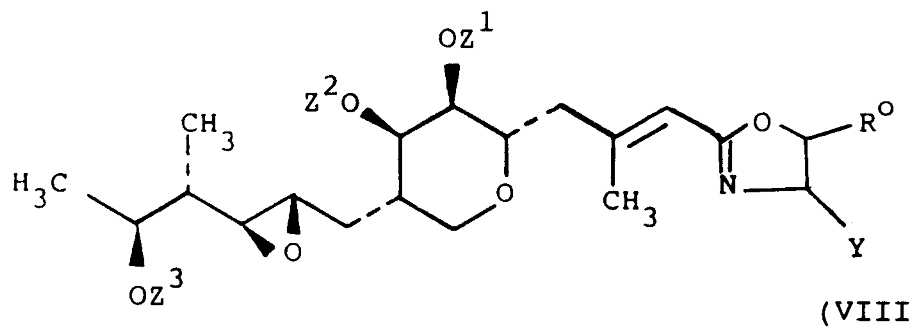

(VIII)

in which $Z^1$, $Z^2$, and $Z^3$ are the same or different and each is hydrogen or a hydroxyl-protecting group, $R^o$ isas defined in claim 1, and Y is a leaving group; with a strong base, and where necessary removing any hydroxyl-protecting group.

5. A process as claimed in claim 4 in which Y is arylsulphonyl.

6. A process as claimed in claim 4 or claim 5 in which the strong base is 1,8-diazobicyclo[5.4.0]undec-7-ene or 1,5-diazobicyclo[4.3.0]non-5-ene.

7. A process for the preparation of a compound of formula (VIII):

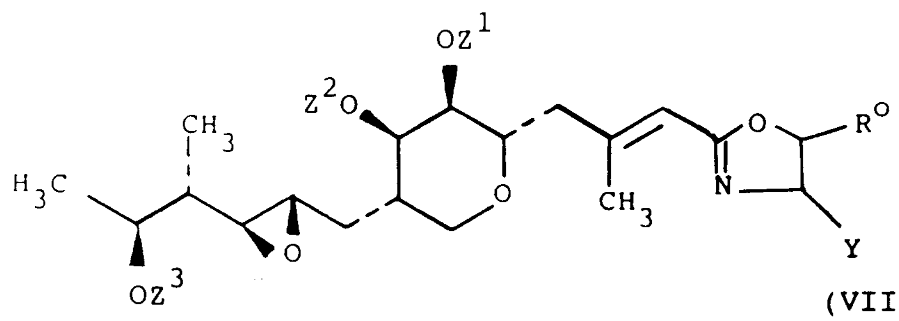

(VIII)

in which $Z^1$, $Z^2$, and $Z^3$ are the same or different and each is a hydroxyl-protecting group, $R^o$ is as defined in claim 1, and Y is a leaving group;
which process comprises treating a compound of formula (IX):

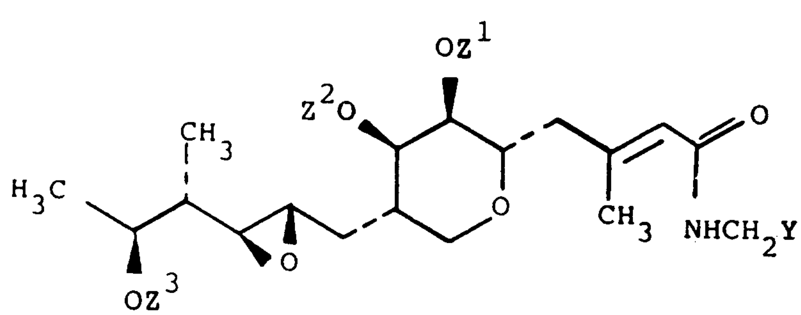

(IX)

in which $Z^1$, $Z^2$, and $Z^3$ are the same or different and each is hydrogen or a hydroxyl-protecting group, and Y is as defined in claim 10,
with a compound formula (X):

$R^oCHO$ (X)

in which $R^o$ is as defined in claim 1, or a corresponding analogue thereof in which the aldehyde functionality of the compound of formula (X) is masked, under dehydrating conditions.

8. A process as claimed in claim 7 in which the dehydrating conditions comprise using triphenylphosphine in combination with carbon tetrachloride or hexachloroethane, in the presence of triethylamine.

9. A process for the preparation of a compound of formula (I) as defined in claim 1 which process comprises treating a compound of formula (XII):

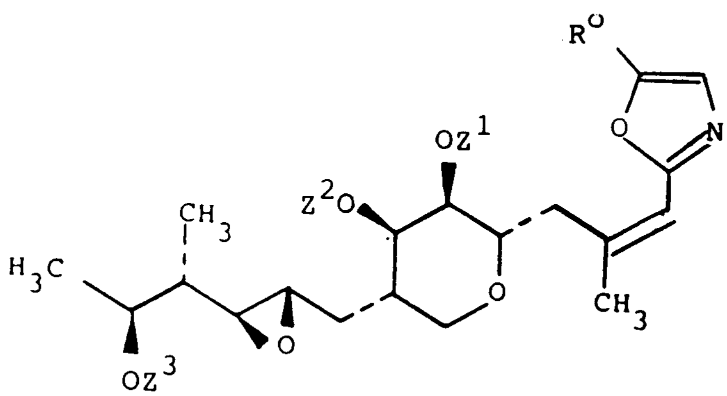

(XII)

in which $R^o$ is as defined in claim 1, and $Z^1$, $Z^2$ and $Z^3$ are the same or different and each is hydrogen or a hydroxyl-protecting group, under conditions effective for isomerising the carbon-carbon double bond of the compound of formula (XII).

10. A process as claimed in any one of claims 1 to 9 for the preparation of a compound of formula (I) in which $R^o$ is a group of formula (a) and $R^1$ is hydrogen.

11. A process as claimed in any one of claims 1 to 9 for the preparation of a compound of formula (I) in which $R^o$ is a group of formula (b) and $R^1$ and $R^3$ is each hydrogen.

12. A process as claimed in any one of claims 1 to 9 for the preparation of a compound of formula (I) in which $R^1$ and $R^3$ is each hydrogen.

13. A process as claimed in any one of claims 1 to 9 for the preparation of a compound of formula (I):
2-(1-normon-2-yl)-5-(3-phenylisoxazol-5-yl)oxazole;
2(1-normon-2-yl)-5-(3-p-methylthiophenylisoxazol-5-yl)oxazole;
2-(1-normon-2-yl)-5-(3-p-methylsulphonylphenylisoxazol-5-yl)oxazole;
2-(1-normon-2-yl)-5-(3-methylisoxazol-5-yl)oxazole;
5-(3-bromo-5-isoxazolyl)-2-(1-normon-2-yl)oxazole;
2-(1-normon-2-yl)-5-(3-methoxy-5-isoxazolyl)oxazole;
2-(1-normon-2-yl)-5-(3-isopropyl-5-isoxazolyl)oxazole;
5-(3-t-butyl-5-isoxazolyl)-2-(1-normon-2-yl)oxazole;
2-(1-normon-2-yl)-5-(3-ethoxycarbonyl-5-isoxazolyl)oxazole;
2-(1-normon-2-yl)-5-(3-formyl-5-isoxazolyl)oxazole;
2-(1-normon-2-yl)-5-(3-hydroxymethyl-5-isoxazolyl)oxazole;
2-(1-normon-2-yl)-5-(3-pyrrolidin-1-yl-5-isoxazolyl)oxazole;
5-(2-furyl)-2-(1-normon-2-yl)oxazole;

5-(5-bromo-2-furyl)-2-(normon-2-yl)oxazole;
5-(3-furyl)-2-(1-normon-2-yl)oxazole;
5-(5-methyl-2-furyl)-2-(1-normon-2-yl)oxazole;
5-(5-methoxycarbonyl-2-furyl)-2-(1-normon-2-yl)oxazole;
5-(5-hydroxymethyl-2-furyl)-2-(1-normon-2-yl)oxazole;
sodium 5-(5-carboxylato-2-furyl)-2-(1-normon-2-yl)oxazole;
5-(5-carboxyamido-2-furyl)-2-(1-normon-2-yl)oxazole;
5-(5-methoxyformimidoyl-2-furyl)-2-(1-normon-2-yl)oxazole;
5-(2,5-dimethyl-3-furyl)-2-(1-normon-2-yl)oxazole;
5-(4-cyano-2-furyl)-2-(1-normon-2-yl)oxazole;
5-(5-cyano-2-furyl)-2-(1-normon-2-yl)oxazole;
5-(4-formyl-2-furyl)-2-(1-normon-2-yl)oxazole;
5-(2-cyanofuran-4-yl)-2-(1-normon-2-yl)oxazole;
5-(2-methoxyformimidoylfuran-4-yl)-2-(1-normon-2-yl)oxa zole;
5-(4-methylfuran-2-yl)-2-(1-normon-2-yl)oxazole; and
5-(4-hydroxymethylfur-2-yl)-2-(1-normon-2-yl)oxazole.

**14.** A process for the preparation of a pharmaceutical or veterinary composition which comprises admixing a compound of formula I as defined in claim 1 together with a pharmaceutically or veterinarily acceptable carrier or excipient.

**Patentansprüche**
**Patentansprüche für folgende Vertragsstaaten : AT, BE, CH, DE, DK, FR, GB, GR, IT, LI, LU, NL, SE**

**1.** Verbindung der Formel (I):

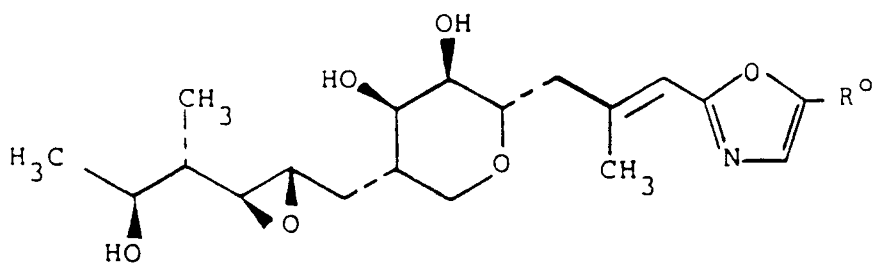

(I)

bei der $R^0$ einen Rest der Formel (a), (b) oder (c):

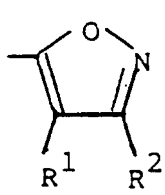

(a)

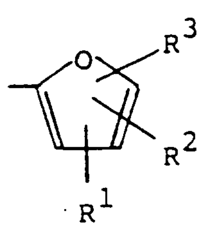

(b)

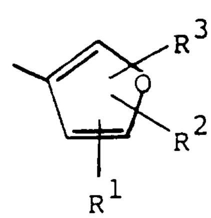

(c)

bedeutet, in denen $R^1$, $R^2$ und $R^3$ gleich oder verschieden sind und jeweils ausgewählt sind aus Wasserstoffatomen, Halogenatomen, gegebenenfalls substituierten $(C_{1-6})$-Alkyl-, Aryl-, Aralkyl-, Heterocyclyl-, $(C_{1-6})$-Alkoxy-, Hydroxy-, Carboxyresten und deren Salzen, $(C_{1-6})$-Alkoxycarbonyl-, Carbamoyl-, Mono- und Di-$(C_{1-6})$-Alkylcarbamoyl-, Sulphamoyl-, Mono- und Di-$(C_{1-6})$-Alkylsulphamoyl-, Cyano-, Nitro-, Amino-, Mono- und Di-$(C_{1-6})$-Alkylamino-, Acylamino, Ureido-, $(C_{1-6})$-Alkoxycarbonylamino-, $(C_{1-6})$-Alkoxyimino-, 2,2,2-Trichlorethoxycarbonylamino-, Acyl-, $(C_{1-6})$-Alkylthio-, Arylthio-, $(C_{1-6})$-Alkansulphinyl-, Arylsulphinyl-, $(C_{1-6})$-Alkansulphonyl- und Arylsulphonylresten.

**2.** Verbindung gemäß Anspruch 1, bei der $R^1$ und $R^3$ jeweils Wasserstoffatome sind.

**3.** Verbindung gemäß Anspruch 1, bei der $R^0$ einen Rest mit der Formel (a) darstellt und $R^1$ ein Wasserstoffatom ist.

**4.** Verbindung gemäß Anspruch 1, bei der $R^0$ einen Rest mit der Formel (b) darstellt und $R^1$ und $R^3$ jeweils Wasserstoffatome sind.

**5.** Verbindung mit der Formel (I) gemäß Anspruch 1, ausgewählt aus:
2-(1-Normon-2-yl)-5-(3-phenylisoxazol-5-yl)oxazol;
2-(1-Normon-2-yl)-5-(3-p-methylthiophenylisoxazol-5-yl)oxazol;
2-(1-Normon-2-yl)-5-(3-p-methylsulphonylphenylisoxazol-5-yl)oxazol;
2-(1-Normon-2-yl)-5-(3-methylisoxazol-5-yl)oxazol;
5-(3-Bromo-5-isoxazolyl)-2-(1-normon-2-yl)oxazol;
2-(1-Normon-2-yl)-5-(3-methoxy-5-isoxazolyl)oxazol;
2-(1-Normon-2-yl)-5-(3-isopropyl-5-isoxasolyl)oxazol;
5-(3-t-Butyl-5-isoxazolyl)-2-(1-normon-2-yl)oxazol;
2-(1-Normon-2-yl)-5-(3-ethoxycarbonyl-5-isoxazolyl)oxazol;
2-(1-Normon-2-yl)-5-(3-formyl-5-isoxazolyl)oxazol;
2-(1-Normon-2-yl)-5-(3-hydroxymethyl-5-isoxazolyl)oxazol;
2-(1-Normon-2-yl)-5-(3-pyrrolidin-1-yl-5-isoxazolyl)oxazol;
5-(2-Furyl)-2-(1-normon-2-yl)oxazol;
5-(5-Bromo-2-furyl)-2-(normon-2-yl)oxazol;
5-(3-Furyl)-2-(1-normon-2-yl)oxazol;
5-(5-Methyl-2-furyl)-2-(1-normon-2-yl)oxazol;
5-(5-Methoxycarbonyl-2-furyl)-2-(1-normon-2-yl)oxazol;
5-(5-Hydroxymethyl-2-furyl)-2-(1-normon-2-yl)oxazol;
Natrium-5-(5-Carboxylato-2-furyl)-2-(1-normon-2-yl)oxazol;
5-(5-Carboxyamido-2-furyl)-2-(1-normon-2-yl)oxazol;
5-(5-Methoxyformimidoyl-2-furyl)-2-(1-normon-2-yl)oxazol;
5-(2,5-Dimethyl-3-furyl)-2-(1-normon-2-yl)oxazol;
5-(4-Cyano-2-furyl)-2-(1-normon-2-yl)oxazol;
5-(5-Cyano-2-furyl)-2-(1-normon-2-yl)oxazol;
5-(4-Formyl-2-furyl)-2-(1-normon-2-yl)oxazol;
5-(2-Cyanofuran-4-yl)-2-(1-normon-2-yl)oxazol;
5-(2-Methoxyformimidoylfuran-4-yl)-2-(1-normon-2-yl)oxazol;
5-(4-Methylfuran-2-yl)-2-(1-normon-2-yl)oxazol und
5-(4-Hydroxymethylfur-2-yl)-2-(1-normon-2-yl)oxazol.

**6.** Verfahren zur Herstellung einer Verbindung mit der Formel (I) gemäß Anspruch 1, das die Umsetzung einer Verbindung mit der Formel (III):

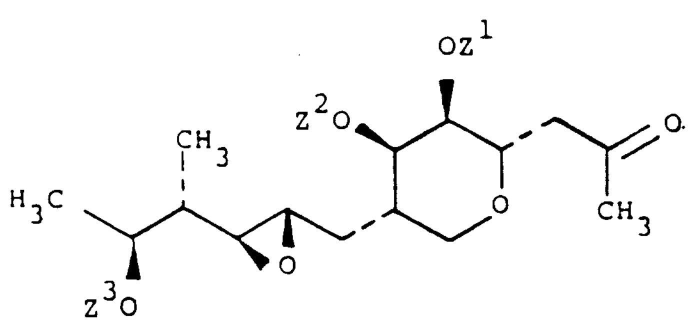

(III)

in der $Z^1$, $Z^2$ und $Z^3$ gleich oder verschieden sind und jeweils Wasserstoffatome oder eine Schutzgruppe für Hydroxyl bedeuten
mit einer Verbindung mit der Formel (IV):

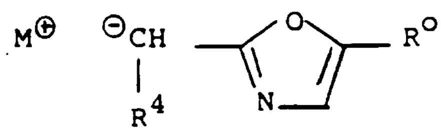

(IV)

umfaßt, bei der:
$R^0$ wie für Formel (I) definiert ist,
$M^+$ ein Metallkation ist und $R^4$ eine anionenstabilisierende Gruppe ist, die mit einer $\beta$-Hydroxylgruppe spontan eliminiert wird, wobei ein Olefin erhalten wird;
und, falls notwendig, Entfernen der Schutzgruppen für Hydroxyl.

**7.** Verfahren zur Herstellung einer Verbindung der Formel (I) gemäß Anspruch 1, das die Zyklisierung einer Verbindung mit der Formel (VII):

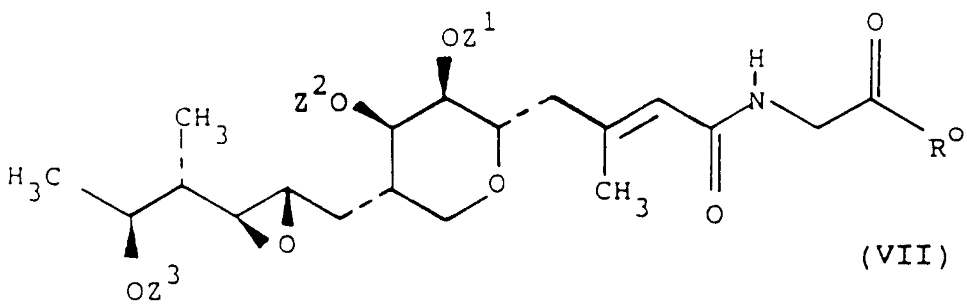

(VII)

umfaßt, bei der $R^0$ wie für Formel (I) definiert ist, und $Z^1$, $Z^2$ und $Z^3$ gleich oder verschieden sein können und jeweils Wasserstoff oder eine Schutzgruppe für Hydroxylbedeuten; und, falls notwendig, Entfernen der Schutzgruppen für Hydroxyl.

**8.** Verfahren zur Herstellung einer Verbindung der Formel (I) gemäß Anspruch 1, das das Behandeln einer Verbindung mit der Formel (VIII):

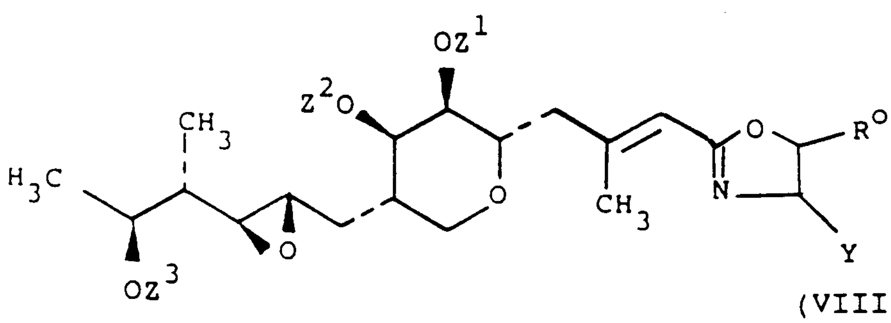

(VIII)

umfaßt bei der $Z^1$, $Z^2$ und $Z^3$ gleich oder verschieden sind und jeweils Wasserstoffatome oder eine Schutzgruppe für Hydroxyl sind, $R^0$ wie in Anspruch 1 definiert ist, und Y eine Abgangsgruppe ist; mit einer starken Base und falls notwendig, das Entfernen der Schutzgruppen für Hydroxyl.

**9.** Arzneimittel oder Tierarzneimittel, das eine Verbindung gemäß eines der Ansprüche 1 bis 5 in Verbindung mit einem pharmazeutisch oder veterinärmedizinisch verträglichen Träger oder Bindemittel umfaßt.

**10.** Verbindung gemäß eines der Ansprüche 1 bis 5 zur therapeutischen Anwendung.

**Patentansprüche für folgenden Vertragsstaat : ES**

**1.** Verfahren zur Herstellung einer Verbindung mit der Formel (I):

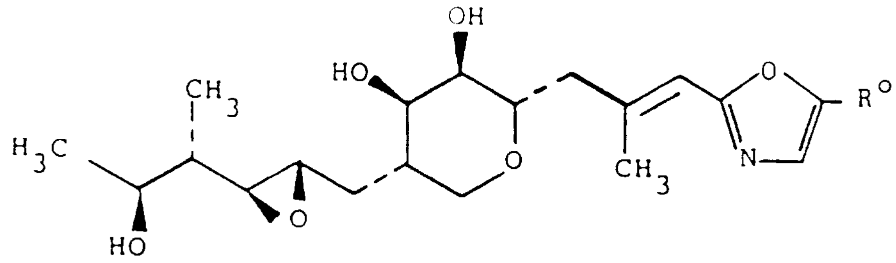

(I)

bei der $R^0$ einen Rest der Formel (a), (b) oder (c):

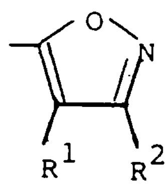

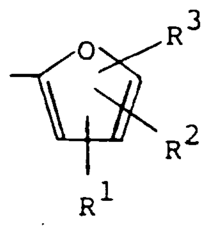

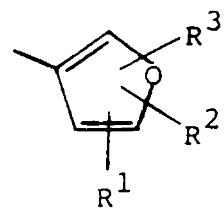

(a) (b) (c)

bedeutet, in denen $R^1$, $R^2$ und $R^3$ gleich oder verschieden sind und jeweils ausgewählt sind aus Wasserstoffatomen, Halogenatomen, gegebenenfalls substituierten ($C_{1-6}$)-Alkyl-, Aryl-, Aralkyl-, Heterocyclyl-, ($C_{1-6}$)-Alkoxy-, Hydroxy-, Carboxyresten und deren Salzen, ($C_{1-6}$)-Alkoxycarbonyl-, Carbamoyl-, Mono- und Di-($C_{1-6}$)-Alkylcarbamoyl-, Sulphamoyl-, Mono- und Di-($C_{1-6}$)-Alkylsulphamoyl-, Cyano-, Nitro-, Amino-, Mono- und Di-($C_{1-6}$)-Alkylamino-, Acylamino, Ureido-, ($C_{1-6}$)-Alkoxycarbonylamino-, ($C_{1-6}$)-Alkoxyimino-, 2,2,2-Trichlorethoxycarbonylamino-, Acyl-, ($C_{1-6}$)-Alkylthio-, Arylthio-, ($C_{1-6}$)-Alkansulphinyl-, Arylsulphinyl-, ($C_{1-6}$)-Alkansulphonyl- und Arylsulphonylresten;
das die Umsetzung einer Verbindung der Formel (III)

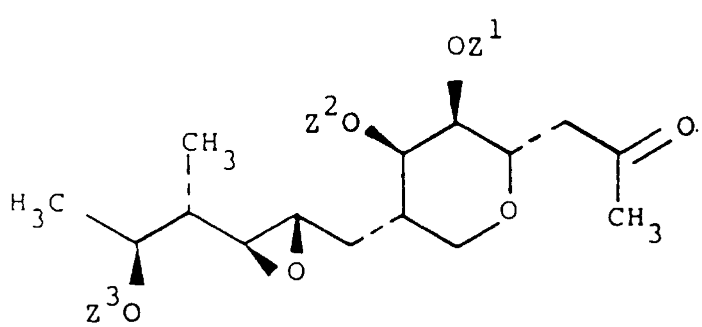

(III)

in der $Z^1$, $Z^2$ und $Z^3$ gleich oder verschieden sind und jeweils Wasserstoffatome oder eine Schutzgruppe für Hydroxyl bedeuten,
mit einer Verbindung mit der Formel (IV):

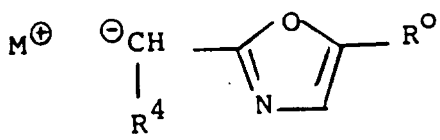

(IV)

umfaßt, bei der:
$R^0$ wie für Formel (I) definiert ist,
$M^+$ ein Metallkation ist und $R^4$ eine anionenstabilisierende Gruppe ist, die mit einer $\beta$-Hydroxylgruppe spontan eliminiert wird, wobei ein Olefin erhalten wird;
und, falls notwendig, Entfernen der Schutzgruppen für Hydroxyl.

**2.** Verfahren zur Herstellung einer Verbindung der Formel (I) gemäß Anspruch 1, das die Zyklisierung einer Verbindung der Formel (VII):

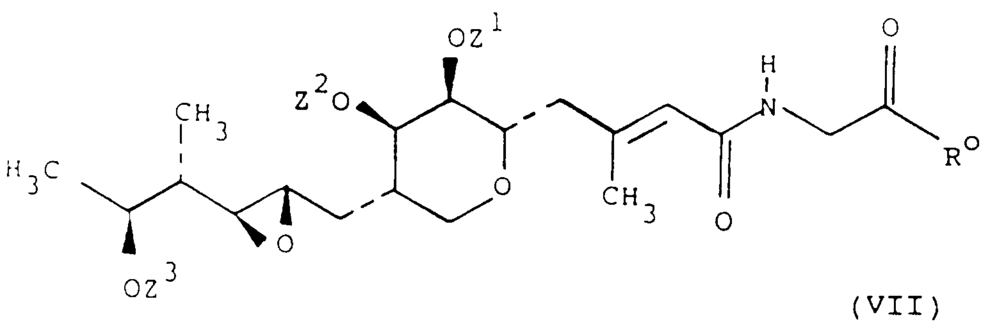

(VII)

umfaßt, bei der $R^0$ wie für Formel (I) definiert ist, und $Z^1$, $Z^2$ und $Z^3$ gleich oder verschieden sein können und jeweils Wasserstoff oder eine Schutzgruppe für Hydroxyl bedeuten; und, falls notwendig, Entfernen aller Schutzgruppen für Hydroxyl.

**3.** Verfahren gemäß Anspruch 2, bei dem die Zyklisierung mit Hilfe eines Chlorierungsmittels, eines Carbonsäureanhydrids, eines gemischten Anhydrids oder eines Säurechlorids durchgeführt wird.

**4.** Verfahren zur Herstellung einer Verbindung der Formel (I) gemäß Anspruch 1, das das Behandeln einer Verbindung der Formel (VIII):

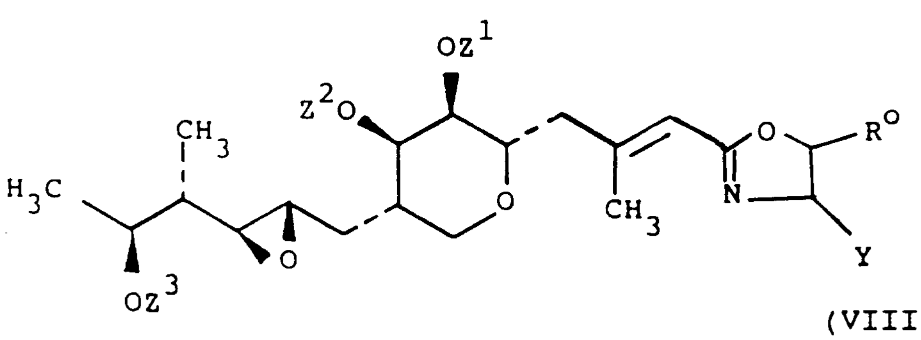

(VIII)

bei der $Z^1$, $Z^2$ und $Z^3$ gleich oder verschieden sind und jeweils Wasserstoffatome oder eine Schutzgruppe für Hydroxyl bedeuten, $R^0$ wie in Anspruch 1 definiert ist, und Y eine Abgangsgruppe ist; mit einer starken Base umfaßt und, falls notwendig, Entfernen der Schutzgruppen für Hydroxyl.

**5.** Verfahren gemäß Anspruch 4, bei dem Y ein Arylsulphonylrest ist.

6. Verfahren gemäß Anspruch 4 oder Anspruch 5, bei dem die starke Base 1,8-Diazobicyclo[5.4.0]undec-7-en oder 1,5-Diazobicyclo[4.3.0]non-5-en ist.

7. Verfahren zur Herstellung einer Verbindung der Formel (VIII):

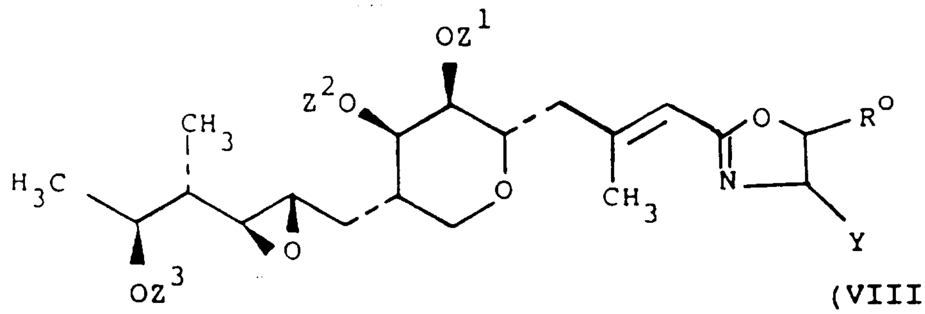

(VIII)

bei der $Z^1$, $Z^2$ und $Z^3$ gleich oder verschieden sind und jeweils eine Schutzgruppe für Hydroxyl bedeuten, $R^0$ wie in Anspruch 1 definiert ist und Y eine Abgangsgruppe ist;
das Verfahren das Behandeln einer Verbindung der Formel (IX):

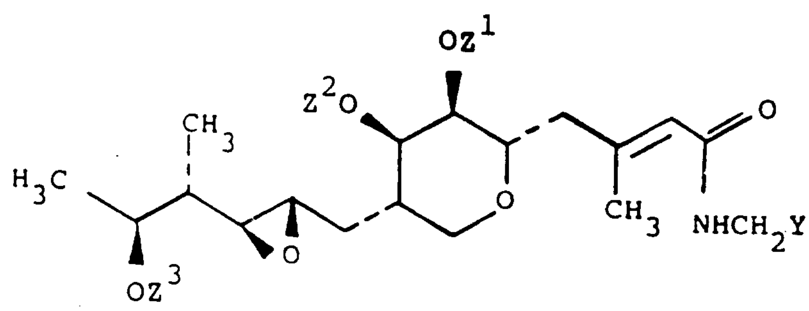

(IX)

bei der $Z^1$, $Z^2$ und $Z^3$ gleich oder verschieden sind und jeweils Wasserstoffatome oder eine Schutzgruppe für Hydroxyl bedeuten und Y wie in Anspruch 10 definiert ist, unter dehydratisierenden Bedingungen
mit einer Verbindung der Formel (X):

$R^0CHO$ (X)

umfaßt, bei der $R^0$ wie in Anspruch 1 definiert ist,
oder einem dementsprechenden Analogon davon, bei dem die Aldehydfunktionalität der Verbindung der Formel (X) maskiert ist.

8. Verfahren gemäß Anspruch 7, bei dem die dehydratisierenden Bedingungen die Verwendung von Triphenylphosphin in Verbindung mit Tetrachlorkohlenstoff oder Hexachlorethan in Gegenwart von Triethylamin umfassen.

9. Verfahren zur Herstellung einer Verbindung der Formel (I) gemäß Anspruch 1, das die Behandlung einer Verbindung der Formel (XII):

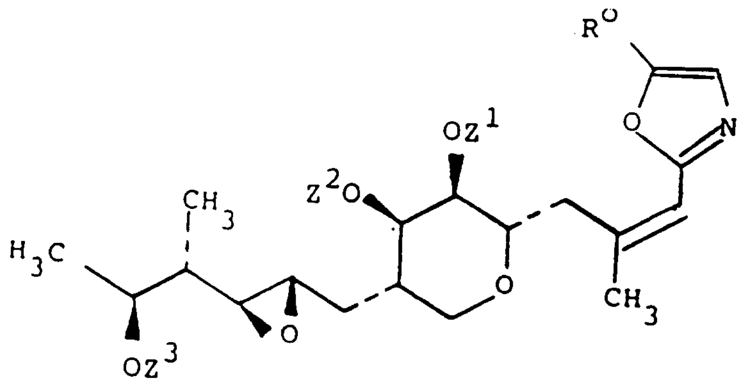

(XII)

bei der $R^0$ gemäß Anspruch 1 definiert ist und $Z^1$, $Z^2$ und $Z^3$ gleich oder verschieden sind und jeweils Wasserstoffatome oder eine Schutzgruppe für Hydroxyl bedeuten, unter Bedingungen umfaßt, die wirksam sind zur Isomerisierung der Kohlenstoff-Kohlenstoff-Doppelbindung der Verbindung der Formel (XII).

**10.** Verfahren gemäß einem der Ansprüche 1 bis 9 zur Herstellung einer Verbindung der Formel (I), bei der $R^0$ ein Rest der Formel (a) ist und $R^1$ ein Wasserstoffatom ist.

**11.** Verfahren gemäß einem der Ansprüche 1 bis 9 zur Herstellung einer Verbindung der Formel (I), bei der $R^0$ ein Rest der Formel (b) ist und $R^1$ und $R^3$ jeweils Wasserstoffatome sind.

**12.** Verfahren gemäß einem der Ansprüche 1 bis 9 zur Herstellung einer Verbindung der Formel (I), bei der $R^1$ und $R^3$ jeweils Wasserstoffatome sind.

**13.** Verfahren gemäß einem der Ansprüche 1 bis 9 zur Herstellung einer Verbindung der Formel (I), nämlich
2-(1-Normon-2-yl)-5-(3-phenylisoxazol-5-yl)oxazol;
2-(1-Normon-2-yl)-5-(3-p-methylthiophenylisoxazol-5-yl)oxazol;
2-(1-Normon-2-yl)-5-(3-p-methylsulphonylphenylisoxazol-5-yl)oxazol;
2-(1-Normon-2-yl)-5-(3-methylisoxazol-5-yl)oxazol;
5-(3-Bromo-5-isoxazolyl)-2-(1-normon-2-yl)oxazol;
2-(1-Normon-2-yl)-5-(3-methoxy-5-isoxazolyl)oxazol;
2-(1-Normon-2-yl)-5-(3-isopropyl-5-isoxazolyl)oxazol;
5-(3-t-Butyl-5-isoxazolyl)-2-(1-normon-2-yl)oxazol;
2-(1-Normon-2-yl)-5-(3-ethoxycarbonyl-5-isoxazolyl)oxazol;
2-(1-Normon-2-yl)-5-(3-formyl-5-isoxazolyl)oxazol;
2-(1-Normon-2-yl)-5-(3-hydroxymethyl-5-isoxazolyl)oxazol;
2-(1-Normon-2-yl)-5-(3-pyrrolidin-1-yl-5-isoxazolyl)oxazol;
5-(2-Furyl)-2-(1-normon-2-yl)oxazol;
5-(5-Bromo-2-furyl)-2-(normon-2-yl)oxazol;
5-(3-Furyl)-2-(1-normon-2-yl)oxazol;
5-(5-Methyl-2-furyl)-2-(1-normon-2-yl)oxazol;
5-(5-Methoxycarbonyl-2-furyl)-2-(1-normon-2-yl)oxazol;
5-(5-Hydroxymethyl-2-furyl)-2-(1-normon-2-yl)oxazol;
Natrium-5-(5-Carboxylato-2-furyl)-2-(1-normon-2-yl)oxazol;
5-(5-Carboxyamido-2-furyl)-2-(1-normon-2-yl)oxazol;
5-(5-Methoxyformimidoyl-2-furyl)-2-(1-normon-2-yl)oxazol;
5-(2,5-Dimethyl-3-furyl)-2-(1-normon-2-yl)oxazol;
5-(4-Cyano-2-furyl)-2-(1-normon-2-yl)oxazol;
5-(5-Cyano-2-furyl)-2-(1-normon-2-yl)oxazol;
5-(4-Formyl-2-furyl)-2-(1-normon-2-yl)oxazol;

5-(2-Cyanofuran-4-yl)-2-(1-normon-2-yl)oxazol;
5-(2-Methoxyformimidoylfuran-4-yl)-2-(1-normon-2-yl)oxazol;
5-(4-Methylfuran-2-yl)-2-(1-normon-2-yl)oxazol und
5-(4-Hydroxymethylfur-2-yl)-2-(1-normon-2-yl)oxazol.

**14.** Verfahren zur Herstellung eines Arzneimittels oder Tierarzneimittels, die das Mischen einer Verbindung der Formel (I) gemäß Anspruch 1 mit einem pharmazeutisch oder veterinärmedizinisch verträglichen Träger oder Bindemittel umfaßt.

**Revendications**

**Revendications pour les Etats contractants suivants : AT, BE, CH, DE, DK, FR, GB, GR, IT, LI, LU, NL, SE**

**1.** Composé de formule (I):

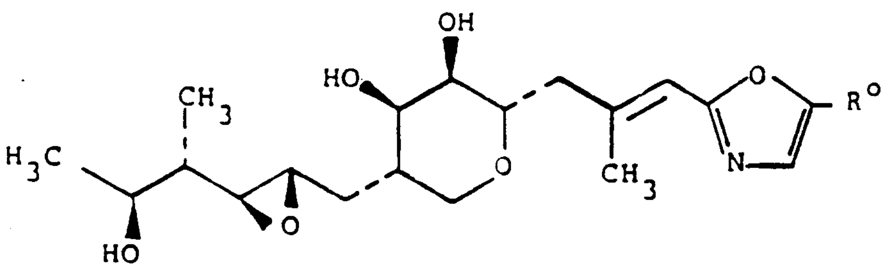

(I)

dans lequel R° représente un groupe de formule (a), (b), ou (c):

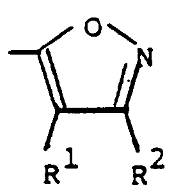

(a)

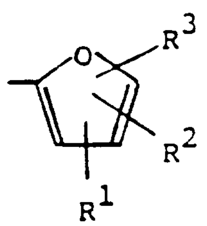

(b)

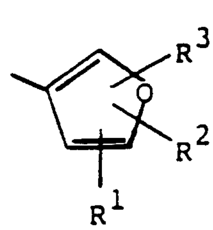

(c)

formules dans lesquelles $R^1$, $R^2$, et $R^3$ sont identiques ou différents, et chacun est choisi parmi l'hydrogène, les halogènes, les groupes, éventuellement substitués, alkyle $C_{1-6}$, aryle, aralkyle, hétérocyclyle, alcoxy $C_{1-6}$, hydroxy, carboxy, et les sels de celui-ci, les groupes alcoxy$C_{1-6}$carbonyle, carbamoyle, mono- et di-alklyC$_{1-6}$carbamoyle, sulfamoyle, mono- et di-alklC$_{1-6}$sulfamoyle, cyano, nitro, amino, mono-et di-alkylC$_{1-6}$amino, acylamino, ureido, alcoxyC$_{1-6}$carbonylamino, alcoxyC$_{1-6}$imino, 2,2,2-trichloroéthoxycarbonylamino, acyle, alkyleC$_{1-6}$thio, arylthio, alcaneC$_{1-6}$sulfinyle arylsulfinyle, alcaneC$_{1-6}$sulfonyle, et arylsulfonyle.

**2.** Composé suivant la revendication 1 dans lequel $R^1$et $R^3$ sont chacun, un atome d'hydrogène.

**3.** Composé suivant la revendication 1, dans lequel R° représente un groupe de formule (a) et $R^1$ est un atome d'hydrogène.

**4.** Composé suivant la revendication 1, dans lequel R° représente un groupe de formule (b) et $R^1$ et $R^3$ sont chacun, des atomes d'hydrogène.

**5.** Composé de formule (I) suivant la revendication 1, choisi parmi:
- le 2-(1-normon-2-yl)-5-(3-phénylisoxasol-5-yl)oxazole;
- le 2-(1-normon-2-yl)-5-(3-p-méthylthiophénylisoxasol-5-yl)oxazole;
- le 2-(1-normon-2-yl)-5-(3-p-méthylsulfonylphéylisoxasol-5-yl)oxazole;

- le 2-(1-normon-2-yl)-5-(3-méthylisoxasol-5-yl)oxazole;
- le 5-(3-bromo-5-isoxalolyl)-2-(1-normon-2-yl)oxazole;
- le 2-(1-normon-2-yl)-5-(3-méthoxy-5-isoxasolyl)oxazole;
- le 2-(1-normon-2-yl)-5-(3-isopropyl-5-isoxasolyl)oxazole;
- le 5-(3-ter-butyl-5-isoxazolyl)-2-(1-normon-2-yl)oxazole;
- le 2-(1-normon-2-yl)-5-(3-éthoxycarbonyl-5-isoxasolyl)oxazole;
- le 2-(1-normon-2-yl)-5-(3-formyl-5-isoxasolyl)oxazole;
- le 2-(1-normon-2-yl)-5-(3-hydroxyméthyl-5-isoxasolyl)oxazole;
- le 2-(1-normon-2-yl)-5-(3-pyrrolidin-1-yl-5-isoxasolyl)oxazole;
- le 5-(2-furyl)-2-(1-normon-2-yl)oxazole;
- le 5-(5-bromo-2-furyl)-2-(1-normon-2-yl)oxazole;
- le 5-(3-furyl)-2-(1-normon-2-yl)oxazole;
- le 5-(5-méthyl-2-furyl)-2-(1-normon-2-yl)oxazole;
- le 5-(5-méthoxycarbonyl-2-furyl)-2-(1-normon-2-yl)oxazole;
- le 5-(5-hydroxyméthyl-2-furyl)-2-(1-normon-2-yl)oxazole;
- le 5-(5-carboxylato-2-furyl)-2-(1-normon-2-yl)oxazole sodique;
- le 5-(5-carboxyamido-2-furyl)-2-(1-normon-2-yl)oxazole;
- le 5-(5-méthoxyformimidoyl-2-furyl)-2-(1-normon-2-yl)oxazole;
- le 5-(2,5-diméthyl-3-furyl)-2-(1-normon-2-yl)oxazole;
- le 5-(4-cyano-2-furyl)-2-(1-normon-2-yl)oxazole;
- le 5-(5-cyano-2-furyl)-2-(1-normon-2-yl)oxazole;
- le 5-(4-formyl-2-furyl)-2-(1-normon-2-yl)oxazole;
- le 5-(2-cyanofuran-2-yl)-2-(1-normon-2-yl)oxazole;
- le 5-(2-méthoxyformimidoylfuran-4-yl)-2-(1-normon-2-yl)oxazole;
- le 5-(4-méthylfuran-2-yl)-2-(1-normon-2-yl)oxazole; et
- le 5-(4-hydroxyméthylfur-2-yl)-2-(1-normon-2-yl)oxazole.

**6.** Procédé de préparation d'un composé de formule (I) suivant la revendication 1, lequel procédé comprend la réaction d'un composé de formule (III):

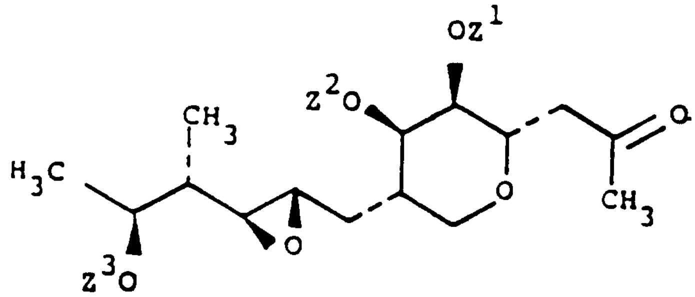

(III)

dans lequel $Z^1$, $Z^2$, et $Z^3$ sont identiques ou différents, et chacun d'eux est un atome d'hydrogène ou un groupe hydroxyle protecteur,
avec un composé de formule (IV):

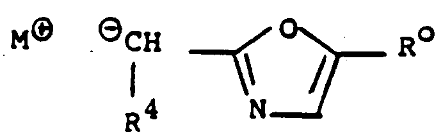

(IV)

dans lequel:
- $R^o$ est tel que défini pour la formule (I),
- $M^+$ est un cation métallique, et $R^4$ est un groupe stabilisateur d'anions qui sera éliminé spontanément avec un groupe $\beta$-hydroxyle, pour donner une oléfine;
et, si nécessaire, l'élimination de tout groupe hydroxyle protecteur.

7. Procédé de préparation d'un composé de formule (I) suivant la revendication 1, lequel procédé comprend la cyclisation d'un composé de formule (VII):

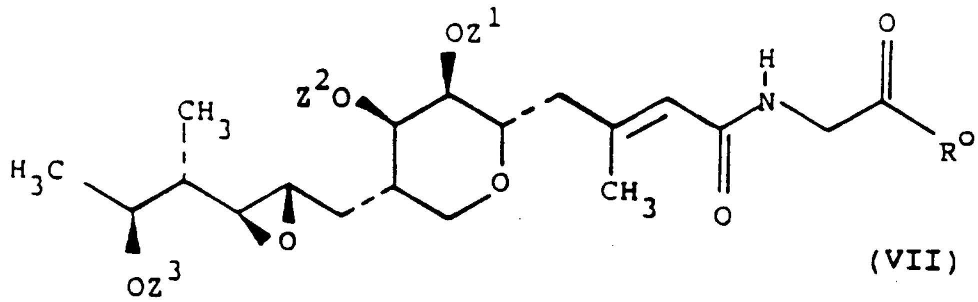

(VII)

dans lequel $R^o$ est tel que défini pour la formule (I), et $Z^1$, $Z^2$, et $Z^3$ sont identiques ou différents et chacun d'eux est un atome d'hydrogène ou un groupe hydroxyle protecteur;
et, si nécessaire, l'élimination de tout groupe hydroxyle protecteur.

8. Procédé de préparation d'un composé de formule (I) suivant la revendication 1, lequel procédé comprend le traitement d'un composé de formule (VIII):

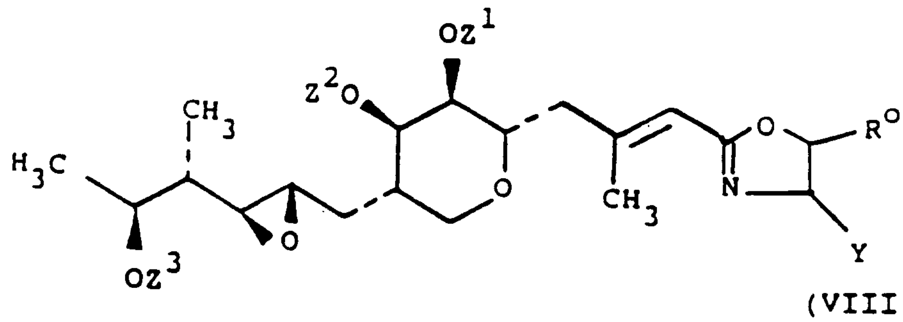

(VIII)

dans lequel $Z^1$, $Z^2$, et $Z^3$ sont identiques ou différents et chacun d'eux est un atome d'hydrogène ou un groupe hydroxyle protecteur, $R^o$ est tel que défini dans la revendication 1, et Y est un groupe partant;
avec une base forte, et, si nécessaire, l'élimination de tout groupe hydroxyle protecteur.

9. Composition pharmaceutique ou vétérinaire qui comprend un composé suivant l'une quelconque des revendications 1 à 5, associé à un véhicule ou à un excipient acceptable du point de vue pharmaceutique ou vétérinaire.

10. Composé suivant l'une quelconque des revendications 1 à 5, pour l'utilisation en thérapie.

**Revendications pour l'Etat contractant suivant : ES**

1. Procédé de préparation d'un composé de formule (I):

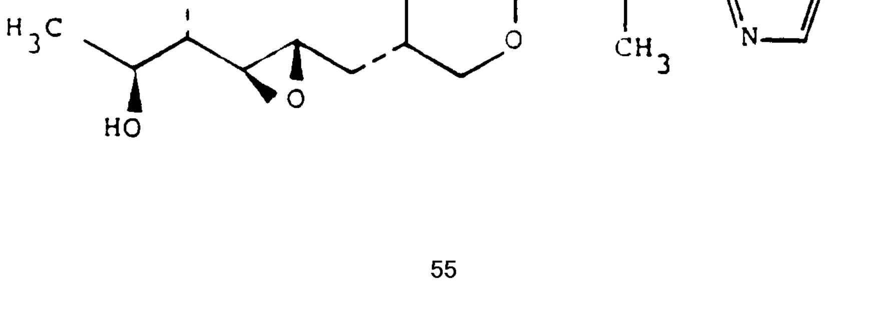

(I)

dans lequel R° représente un groupe de formule (a), (b), ou (c):

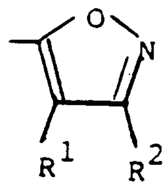

(a)

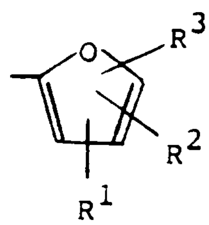

(b)

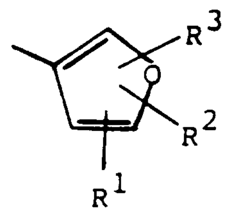

(c)

formules dans lesquelles $R^1$, $R^2$, et $R^3$ sont identiques ou différents, et chacun est choisi parmi l'hydrogène, les halogènes les groupes, éventuellement substitués alkyle $C_{1-6}$, aryle, aralkyle, hétérocyclyle, alcoxy $C_{1-6}$, hydroxy carboxy, et les sels de celui-ci, les groupes alcoxy$C_{1-6}$carbonyle, carbamoyle, mono- et di-alkyl$C_{1-6}$carbamoyle, sulfamoyle, mono- et di-alkyl$C_{1-6}$sulfamoyle cyano nitro amino, mono-et di-alkyl$C_{1-6}$amino, acylamino, ureido, alcoxy$C_{1-6}$carbonylamino, alcoxy$C_{1-6}$imino, 2,2,2-trichloroéthoxycarbonylamino, acyle, alkyle$C_{1-6}$thio, arylthio, alcane$C_{1-6}$sulfinyle, arylsulfinyle alcane$C_{1-6}$sulfonyle, et arylsulfonyle;
lequel procédé comprend la réaction d'un composé de formule (III):

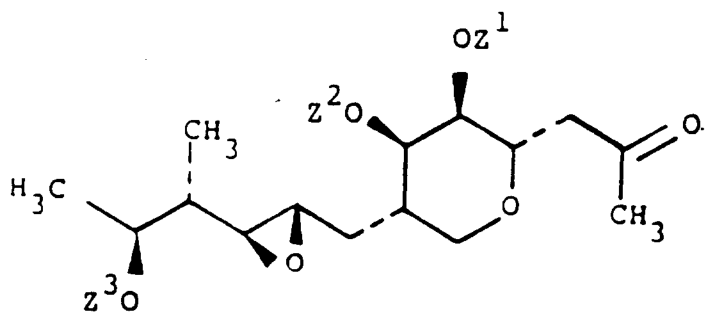

(III)

dans lequel $Z^1$, $Z^2$, et $Z^3$ sont identiques ou différents; et chacun d'eux est un atome d'hydrogène ou un groupe hydroxyle protecteur,
avec un composé de formule (IV):

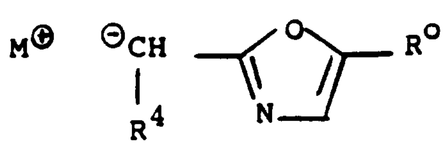

(IV)

dans lequel:
- R° est tel que défini pour la formule (I),
- $M^+$ est un cation métallique, et $R^4$ est un groupe stabilisateur d'anions qui sera éliminé spontanément avec un groupe $\beta$-hydroxyle, pour donner une oléfine;
et, si nécessaire, l'élimination de tout groupe hydroxyle protecteur.

**2.** Procédé de préparation d'un composé de formule (I) suivant la revendication 1, lequel procédé comprend la cyclisation d'un composé de formule (VII):

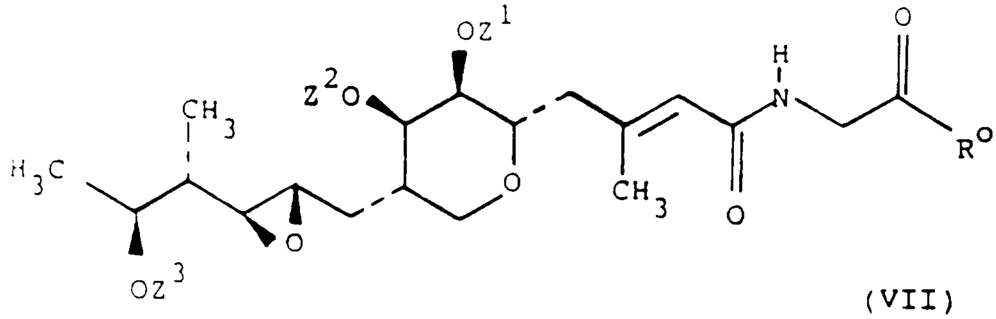

(VII)

dans lequel R° est tel que défini pour la formule (I), et $Z^1$, $Z^2$, et $Z^3$ sont identiques ou différents et chacun d'eux est un atome d'hydrogène ou un groupe hydroxyle protecteur; et, si nécessaire, l'élimination de tout groupe hydroxyle protecteur.

**3.** Procédé de préparation d'un composé de formule (I) suivant la revendication 1, lequel procédé comprend le traitement d'un composé de formule (VIII):

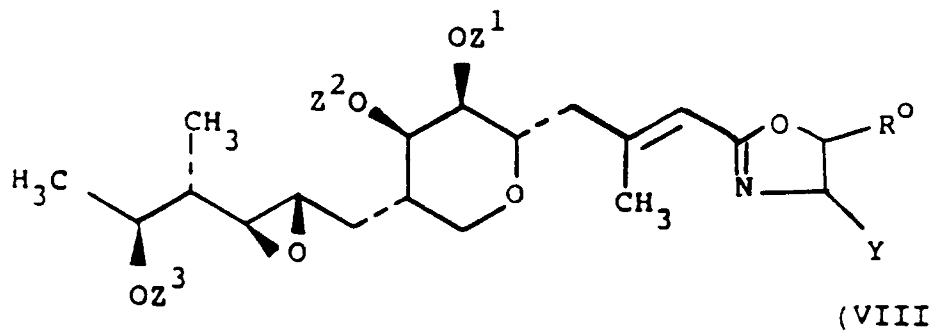

(VIII)

dans lequel $Z^1$, $Z^2$, et $Z^3$ sont identiques ou différents et chacun d'eux est un atome d'hydrogène ou un groupe hydroxyle protecteur, R° est tel que défini dans la revendication 1, et Y est un groupe partant; avec une base forte, et, si nécessaire, l'élimination de tout groupe hydroxyle protecteur.

**5.** Procédé suivant la revendication 4, dans lequel Y est un groupe arylsulfonyle.

**6.** Procédé suivant la revendication 4, ou suivant la revendication 5, dans lequel la base forte est le 1,8-diazobicyclo[5.4.0]-undéc-7-ène ou le 1,5-diazobicyclo[4.3.0]non-5-ène.

**7.** Procédé de préparation d'un composé de formule (VIII):

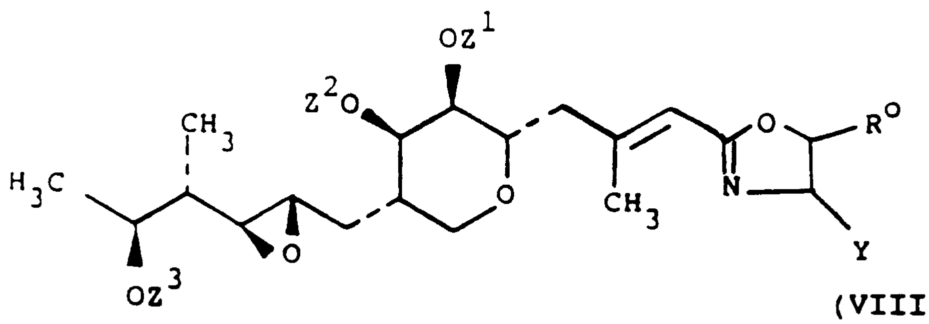

(VIII)

dans lequel $Z^1$, $Z^2$, et $Z^3$ sont identiques ou différents et chacun d'eux est un atome d'hydrogène ou un groupe hydroxyle protecteur, R° est tel que défini dans la revendication 1, et Y est un groupe partant; lequel procédé comprend le traitement d'un composé de formule (IX):

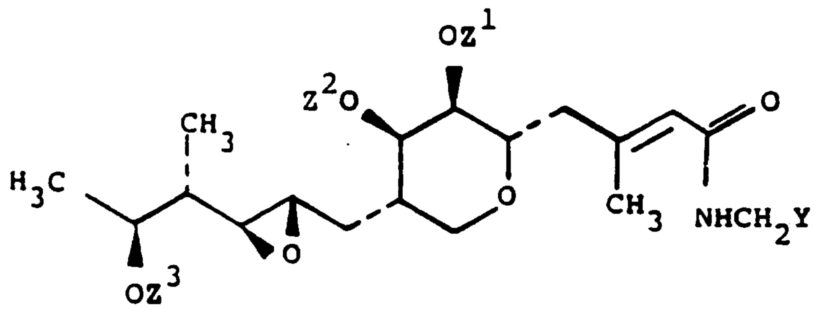

(IX)

dans lequel $Z^1$, $Z^2$, et $Z^3$ sont identiques ou différents et chacun d'eux est un atome d'hydrogène ou un groupe hydroxyle protecteur, et Y est tel que défini dans la revendication 10,
avec un composé de formule (X):

$R^oCHO$ (X)

dans lequel $R^o$ est tel que défini dans la revendication 1,
ou un analogue correspondant de celui-ci, dans lequel la fonction aldéhyde du composé de formule (X) est masquée,
dans des conditions de déshydratation.

**8.** Procédé suivant la revendication 7, dans lequel les conditions de déshydratation comprennent l'utilisation de la triphénylphosphine en combinaison avec le tétrachlorure de carbone ou l'hexachloroéthane, en présence de triéthylamine.

**9.** Procédé de préparation d'un composé de formule (I) suivant la revendication 1, lequel procédé comprend le traitement d'un composé de formule (XII):

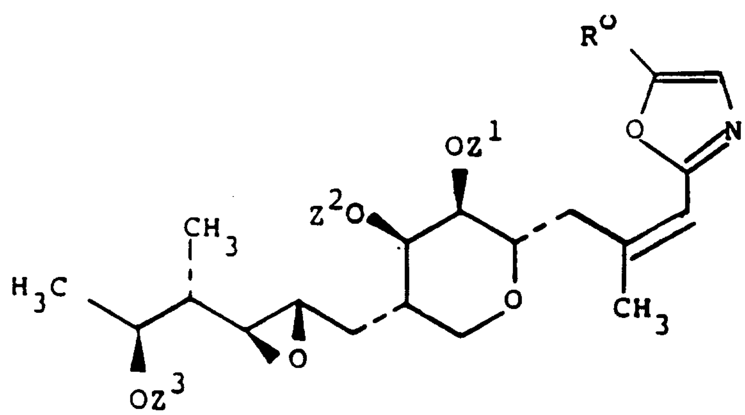

(XII)

dans lequel $R^o$ est tel que défini dans la revendication 1, et $Z^1$, $Z^2$, et $Z^3$ sont identiques ou différents et chacun d'eux est un atome d'hydrogène ou un groupe hydroxyle protecteur,
dans des conditions efficaces pour l'isomérisation des doubles liaisons carbone-carbone du composé de formule (XII).

**10.** Procédé suivant l'une quelconque des revendications 1 à 9, pour la préparation d'un composé de formule (I) dans lequel $R^o$ est un groupe de formule (a), et $R^1$ est un atome d'hydrogène.

**11.** Procédé suivant l'une quelconque des revendications 1 à 9, pour la préparation d'un composé de formule (I) dans lequel $R^o$ est un groupe de formule (b), et $R^1$ et $R^3$ sont chacun, un atome d'hydrogène.

**12.** Procédé suivant l'une quelconque des revendications 1 à 9, pour la préparation d'un composé de formule (I) dans lequel $R^1$ et $R^3$ sont chacun, un atome d'hydrogène.

**13.** Procédé suivant l'une quelconque des revendications 1 à 9, pour la préparation d'un composé de formule (I):
- le 2-(1-normon-2-yl)-5-(3-phénylisoxasol-5-yl)oxazole;

- le 2-(1-normon-2-yl)-5-(3-p-méthylthiophénylisoxasol-5-yl)oxazole;
- le 2-(1-normon-2-yl)-5-(3-p-méthylsulfonylphénylisoxasol-5-yl)oxazole;
- le 2-(1-normon-2-yl)-5-(3-méthylisoxasol-5-yl)oxazole;
- le 5-(3-bromo-5-isoxalolyl)-2-(1-normon-2-yl)oxazole;
- le 2-(1-normon-2-yl)-5-(3-méthoxy-5-isoxasolyl)oxazole;
- le 2-(1-normon-2-yl)-5-(3-isopropyl-5-isoxasolyl)oxazole;
- le 5-(3-ter-butyl-5-isoxazolyl)-2-(1-normon-2-yl)oxazole;
- le 2-(1-normon-2-yl)-5-(3-éthoxycarbonyl-5-isoxasolyl)oxazole;
- le 2-(1-normon-2-yl)-5-(3-formyl-5-isoxasolyl)oxazole;
- le 2-(1-normon-2-yl)-5-(3-hydroxyméthyl-5-isoxasolyl)oxazole;
- le 2-(1-normon-2-yl)-5-(3-pyrrolidin-1-yl-5-isoxasolyl)oxazole;
- le 5-(2-furyl)-2-(1-normon-2-yl)oxazole;
- le 5-(5-bromo-2-furyl)-2-(1-normon-2-yl)oxazole;
- le 5-(3-furyl)-2-(1-normon-2-yl)oxazole;
- le 5-(5-méthyl-2-furyl)-2-(1-normon-2-yl)oxazole;
- le 5-(5-méthoxycarbonyl-2-furyl)-2-(1-normon-2-yl)oxazole;
- le 5-(5-hydroxyméthyl-2-furyl)-2-(1-normon-2-yl)oxazole;
- le 5-(5-carboxylato-2-furyl)-2-(1-normon-2-yl)oxazole sodique;
- le 5-(5-carboxyamido-2-furyl)-2-(1-normon-2-yl)oxazole;
- le 5-(5-méthoxyformimidoyl-2-furyl)-2-(1-normon-2-yl)oxazole;
- le 5-(2,5-diméthyl-3-furyl)-2-(1-normon-2-yl)oxazole;
- le 5-(4-cyano-2-furyl)-2-(1-normon-2-yl)oxazole;
- le 5-(5-cyano-2-furyl)-2-(1-normon-2-yl)oxazole;
- le 5-(4-formyl-2-furyl)-2-(1-normon-2-yl)oxazole;
- le 5-(2-cyanofuran-2-yl)-2-(1-normon-2-yl)oxazole;
- le 5-(2-méthoxyformimidoylfuran-4-yl)-2-(1-normon-2-yl)oxazole;
- le 5-(4-méthylfuran-2-yl)-2-(1-normon-2-yl)oxazole; et
- le 5-(4-hydroxyméthylfur-2-yl)-2-(1-normon-2-yl)oxazole.

**14.** Procédé de préparation d'une composition pharmaceutique ou vétérinaire qui comprend le mélange d'un composé de formule (I) suivant la revendication 1, et d'un véhicule ou d'un excipient acceptable du point de vue pharmaceutique ou vétérinaire.